(19)

Europäisches
Patentamt

European
Patent Office

Office européen
des brevets

(11)  **EP 3 723 096 A1**

(12)  **EUROPEAN PATENT APPLICATION**

(43) Date of publication:
**14.10.2020   Bulletin 2020/42**

(21) Application number: **19169090.8**

(22) Date of filing: **12.04.2019**

(51) Int Cl.:
**G16B 20/20** (2019.01)          *G16B 30/10* (2019.01)
**G16B 50/10** (2019.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **European Molecular Biology
Laboratory
69117 Heidelberg (DE)**

(72) Inventor: **The designation of the inventor has not
yet been filed**

(74) Representative: **Kuttenkeuler, David
Boehmert & Boehmert
Anwaltspartnerschaft mbB
Pettenkoferstrasse 22
80336 München (DE)**

(54)  **COMPREHENSIVE DETECTION OF SINGLE CELL GENETIC STRUCTURAL VARIATIONS**

(57)     The present invention provides a method for detecting structural variations (SV) within genomes of single cells or population of single cells by integrating a three-layered information of sequencing read depth, read strand orientation and haplotype phase. The method of the invention can detect deletions, duplications, polyploidies, translocations, inversions, and copy number neutral loss of heterozygosity (CNN-LOH), and more. The method of the invention can fully karyotype a genome comprehensively, and may be applied in research and clinical approaches. For example, the methods of the invention are useful for analysing cellular samples of patients for diagnosing or aiding a diagnosis, in reproductive medicine to detect embryonic abnormalities, or during therapeutic approaches based on cellular therapies to quality control genetically engineered cells, such as in adoptive T cell therapy and others. The method of the invention may further be applied in research to decipher the karyotypes of cellular models (cell lines), patient samples, or to further unravel genetic and mechanistic pathways leading to the generation of any SV within genomes.

EP 3 723 096 A1

**Description**

FIELD OF THE INVENTION

The present invention provides a method for detecting structural variations (SV) within genomes of single cells or population of single cells by integrating a three-layered information of sequencing read depth, read strand orientation and haplotype phase. The method of the invention can detect deletions, duplications, polyploidies, translocations, inversions, and copy number neutral loss of heterozygosity (CNN-LOH), and more. The method of the invention can fully karyotype a genome comprehensively, and may be applied in research and clinical approaches. For example, the methods of the invention are useful for analysing cellular samples of patients for diagnosing or aiding a diagnosis, in reproductive medicine to detect embryonic abnormalities, or during therapeutic approaches based on cellular therapies to quality control genetically engineered cells, such as in adoptive T cell therapy and others. The method of the invention may further be applied in research to decipher the karyotypes of cellular models (cell lines), patient samples, or to further unravel genetic and mechanistic pathways leading to the generation of any SV within genomes.

DESCRIPTION

Structural variation (SV), in which rearrangements delete, duplicate, invert or translocate DNA segments up to megabases in size, is a major source of genetic variation implicated in numerous diseases. Recent methodological and technological advances enabled the cataloguing of SVs in diverse human populations. Beyond these germline variants, it is becoming increasingly clear that human tissues display ample somatic variation, particularly SVs, a variant class that arises dynamically and at high rates causing extensive genetic heterogeneity. Somatic SV analyses in cellular populations can facilitate studying genetic mosaicism and aberrant clonal expansions, allow lineage tracing, and in the context of cancer may enable improved disease classification and management. SV discovery however remains challenging, with translocations, inversions, complex SV classes, cellular ploidy alterations and SVs arising in repetitive regions frequently escaping detection in genetic heterogeneity contexts.

Somatic structural variation plays key roles in health and disease[10,2]. Cancers, for instance, exhibit vast differences in chromosome number and cytogenetic structure across individual tumor cells[79]. SVs in cancer show dynamic patterns of formation, and can arise as punctuated bursts in periods of genomic instability[4,5] leading to intra-tumor heterogeneity. They represent the leading class of genomic driver alteration in several cancer types[2,1], and comprise copy-number aberrations (CNAs) and copy-balanced SVs which can have dramatic consequences by resulting in gene disruption, gene loss or amplification, gene fusion, enhancer hijacking and reorganized topologically-associating domains (TADs)[2,5]. Recent studies have detected somatic/post-zygotic SVs also in normal tissues including brain, skin and blood[1], where these variants may affect health through decline of tissue functions and/or promotion of disease processes including cancer and leukemia development. Indeed, post-zygotic CNAs in the blood of ageing donors have been associated with leukemia, solid tumors, and common illnesses including type-2-diabetes and coronary heart disease. Post-zygotic SVs also arise during early development where the resultant mosaicism can cause genetic disorders, with repercussions for genetic counseling and testing[56]. Due to their dynamic nature, somatic SVs can profoundly affect disease course. In prostate cancer patients, diverse SV classes affecting the androgen receptor locus can gradually lead to therapy resistance. Moreover, a punctuated burst resulting in complex SVs (i.e. chromothripsis) has been implicated in the spontaneous cure of WHIM syndrome, a congenital immune disorder. The wide diversity of diseases in which somatic SVs are implicated, their prevalence and dynamic occurrence necessitate efficient detection approaches. Single cell analysis should in principal be ideal for this purpose, as it may enable SV detection at low variant allele frequency (VAF) down to the individual cell[15]. Current single cell methods scaling to hundreds or thousands of cells, however, are geared towards CNAs[16-18]. Other SV classes, including translocations, inversions and complex SV classes typically escape detection, despite their relevance to a wide variety of disease processes.

Whether arising in the germline or somatically, SVs represent a particularly difficult-to-identify class of variation. Due to their size which often exceeds DNA sequence read lengths by far, current detection methods depend partly on indirect inference including the interpretation of paired-ends, read-depth, and clipped or split-reads. These methods require extensive sequence coverage for confident SV calling (~20-fold or higher when bulk sequencing is used)[17], which limits their utility for SV detection in heterogeneous contexts - with the exception of read-depth analysis, which can be pursued for variants with relatively low VAF (typically ≥10% VAF), but which is limited to CNAs[10]. Single cell analyses, by comparison, can enable detecting SVs down to the individual cell, and facilitate dissecting patterns of SV co-occurrence and cell-type specific SVs[17]. However, while CNAs are already routinely analyzed in single cells, and scalable[16] as well as commercial applications (e.g., the 10X Genomics "The Chromium Single Cell CNV Solution") are becoming available, the detection of additional SV classes such as balanced and complex SVs in single cells faces important challenges: Currently available SV detection methodology requires the identification of reads (or read pairs) traversing the SV's breakpoints[55]; this remains challenging due to high coverage requirements of such approach, and low as well as uneven coverage levels including localised allelic drop outs in single cells[17]. Due to the requirement for breakpoint-spanning reads, these detection methods break down once SV breakpoints reside in repetitive regions, which are abundant in the genome and in which SVs display enrichment. Moreover, whole genome amplification (WGA), used to increase the amount of DNA accessible, can result in read chimera[19] that may resemble SVs and can thus lead to calling artefacts. And while recent studies have shown that chimera filtering is feasible in conjunction with sufficient sequence coverage[19,20], SV discovery in hundreds (or thousands) of single cells would necessitate vast sequencing costs, and accordingly has not been pursued yet. Additionally, most current methods do not indicate which haplotype a given variant resides on, which may lead to reduced calling power compared to haplotype-aware single cell analyses[57].

Known in art is single-cell/single-strand genome sequencing (Strand-seq)[67,21], a technique based on labelling nascent (i.e. non-template) DNA strands during replication with a nucleoside analogue (BrdU), followed by removal of the non-template strand, and subsequent short read sequencing of the remaining strand[67,21]. Strand-seq was previously shown to successfully map sister chromatid exchanges[21,71], misoriented genomic contigs[21], and heritable (germline) inversions[37]. It was further recently demonstrated that Strand-seq enables whole chromosome-length haplotyping[322,72] and guiding *de novo* genome assembly.

The aim of the present invention was therefore to provide a means and methods to facilitate the comprehensive detection of complex genetic variation, complex structural variations within genomes and chromosomes, and to quantify cellular chromosome stability.

BRIEF DESCRIPTION OF THE INVENTION

Generally, and by way of brief description, the main aspects of the present invention can be described as follows:

In **a first aspect,** the invention pertains to a method for analyzing sequencing data of at least one target chromosomal region by single cell tri-channel processing (scTRIP), comprising providing strand specific sequence data of at least one target chromosomal region of at least one single cell, wherein the strand-specific sequencing data comprise a multitude of strand specific sequence reads obtained by sequencing of the target chromosomal region of at least one single cell, aligning the sequence reads, or if the sequence reads are equally fragmented, each fragmented portion of such sequence read, to a reference assembly, and then assign in any given selected window the at least two of three layers of sequence information: (i) number of total sequence reads, or portions thereof (also known as "read depth"); (ii) number of forward (or Watson) sequence reads, or portions thereof, and number of reverse (or Crick) sequence reads, or portions thereof; (iii) number of sequence reads, or portion thereof, assigned with a specific haplotype identity (such as H1 and/or H2).

In **a second aspect,** the invention pertains to a method of detecting a structural variation (SV) in a target chromosomal region, the method comprising performing the method according to the first aspect and further comprising the step: Identifying a structural variation (SV) by performing step (d) for a multiplicity (at least two) of windows within the sequence data of the positional ordered and aligned sequence reads, and identifying within the multiplicity of windows a sub-region comprising one or more windows having an unusual/altered/changed distribution of the information of any one, or all of, or any combination of, channels (i) to (iii).

In **a third aspect,** the invention pertains to a method of karyotyping a single cell, or a population of multiple single cells, the method comprising,

(a) Providing strand specific sequence data of the at least one target chromosomal region, preferably the complete genome, of at least one single cell, or each of the population of single cells,

(b) Performing a method of the first or second aspect,

(c) Detecting SV within the target chromosomal region of said single cell, or the population of single cells, and

(d) Obtaining an in-silico karyotype based on all detected SVs.

In **a fourth aspect,** the invention pertains to a method of diagnosing a disease in subject, the method comprising, providing strand specific sequence data of one or more cells of the subject, performing a method according to the first or second aspect, detecting within the one or more cells any SV, and comparing the detected SV with a reference state, wherein an altered number, type or location of one or more SV in the sample of the subject indicated the presence of a condition, such as a disease, for example cancer.

In **a fifth aspect,** the invention pertains to a method for assessing the chromosomal stability of a single cell, or within a population of single cells, the method comprising performing a method according to any one of the preceding aspects, and wherein an increased total number, or increased number of any one type or multiple types, of SV in the said single cell or population of single cells, indicates chromosomal instability.

In **a sixth aspect,** the invention pertains to a **computer readable medium** comprising computer readable instructions stored thereon that when run on a computer instruct the computer to perform a method according to any of the aspects or embodiments of the invention.

DETAILED DESCRIPTION OF THE INVENTION

In the following, the elements of the invention will be described. These elements are listed with specific embodiments; however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **a first aspect,** the invention pertains to a method for analyzing sequencing data of at least one target chromosomal region by single cell tri-channel processing (scTRIP), comprising providing strand specific sequence date of at least one target chromosomal region of at least one single cell wherein the strand-specific sequencing data comprise a multitude of strand specific sequence reads obtained by sequencing of the target chromosomal region of at least one single cell, aligning the sequence reads, or if the sequence reads are equally fragmented, each portion of a sequence read, to a reference, and then assign in any given selected window the at least two of three layers of information: (i) number of total sequence reads, or portions thereof (also known as "read depth"); (ii) number of forward (or Watson) sequence reads, or portions thereof, and number of reverse (or Crick) sequence reads, or portions thereof; (iii) number of sequence reads, or portion thereof, assigned with a specific haplotype identity (for example, H1 or H2).

More specifically the first aspect of the invention pertains to the following methods steps, which may be carried out in any sequence technical possible or sensible:

(a) providing strand-specific sequence data of the at least one target chromosomal region of at least one single cell, wherein the strand-specific sequencing data comprise a multitude of strand specific sequence reads obtained by sequencing of the target chromosomal region of at least one single cell;

(b) aligning each sequence read, or portion thereof, to a reference sequence of the at least one target chromosomal region to bring the sequence reads, or portions thereof, into a positional order along the reference sequence of the at least one target chromosomal region;

(c) assigning to each aligned sequence read, or portion thereof, from (b) a chromosomal haplotype identity (H1/H2) along the at least one target chromosomal region; and

(d) assigning to at least one predetermined sequence window of the positional ordered and aligned sequence reads, or portions thereof, any two of the following channels of sequence information:

(i) number of total sequence reads, or portions thereof, aligned in the at least one predetermined sequence window;

(ii) number of forward sequence reads, or portions thereof, and number of reverse sequence reads, or portions thereof, aligned in the at least one predetermined sequence window;

(iii) number of sequence reads, or portions thereof, assigned to a first (H1) haplotype identity; and/or number of sequence reads, or portions thereof, assigned to a second (H2) haplotype identity, aligned in the at least one predetermined sequence window.

The present invention preferably applies the herein described methods in order to karyotype a candidate cell, tissue, or subject, as an example for diagnostic or quality control purposes. For example in one implementation of the first aspect the invention, alternatively or additionally, pertains to a method of karyotyping a genome of at least one single cell of interest, comprising: a) obtaining a plurality of (preferably non-overlapping) strand specific sequences from random locations of the genome of the at least one single cell; b) mapping said test strand specific sequences to a genomic reference scaffold to obtain a test distribution of mapped strand specific sequences; c) assigning to a predetermined sequence window within the reference scaffold (i) number of mapped sequence reads, (ii) number of mapped forward strand reads and number of reverse strand reads, preferably a ratio thereof, and (iii) haplotype identity (H1/H2), preferably the number of H1 and the number of H2 haploidentical reads, or portions thereof, to obtain a three layered test distribution of mapped sequences; d) identifying a statistically significant alteration between an expected distribution, wherein such an alteration indicates a karyotypic abnormality in the genome of the at least one single cell; or e) comparing the three layered test distribution to a reference distribution obtained from a reference cell (such as a healthy cell), wherein if a significant difference is present said difference indicates a karyotypic difference between the at least one single cell and the reference cell.

The inventors developed a technique to integrate three types of valuable information to a sequenced target chromosomal region, such as complete chromosomes or genomes, which consist of read depth, template strand identity (the forward or reverse strand derived from the mother cell after replication), and the haplo-phase or -type, which indicates the identity of a sequence to be derived from the paternal or maternal chromosome present in all diploid organisms. The inventors surprisingly discovered that when analysing the data of sequenced single cells and comparing the data with the genetically expected distribution of the three layers of information, many previously hardly-detectable structural variations within chromosomal regions could be easily identified either by just analysing the sequence data of a single cell, or by looking at a segregation pattern of multiple cells of the same genetic origin. For the latter approach an unusual segregation or distribution of complete or portions of chromosomes within the population can be used to identify polyploidies or translocations within the sequenced genomes.

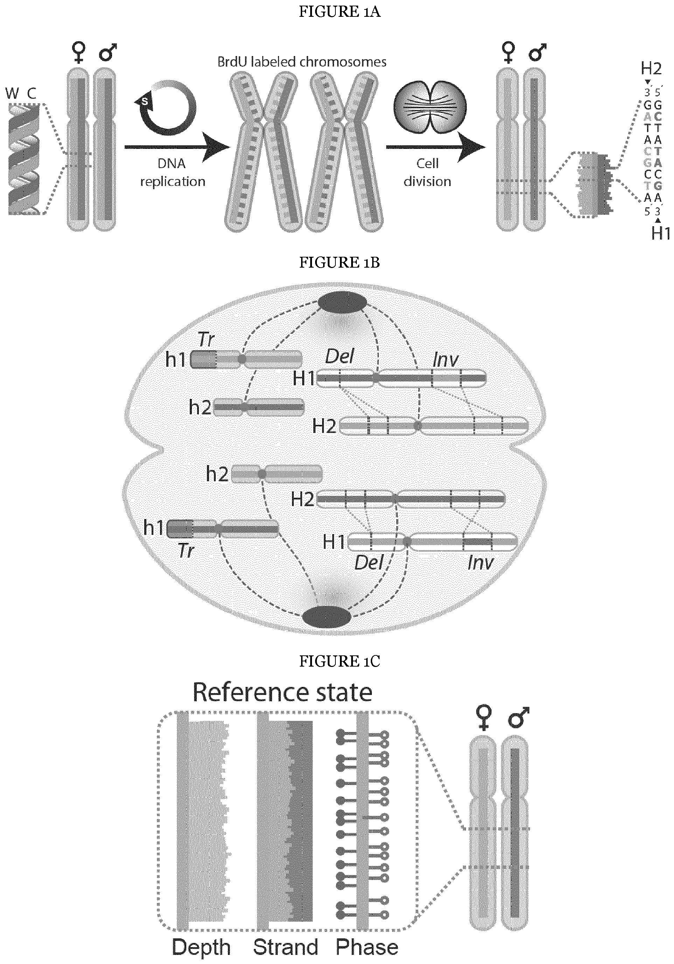

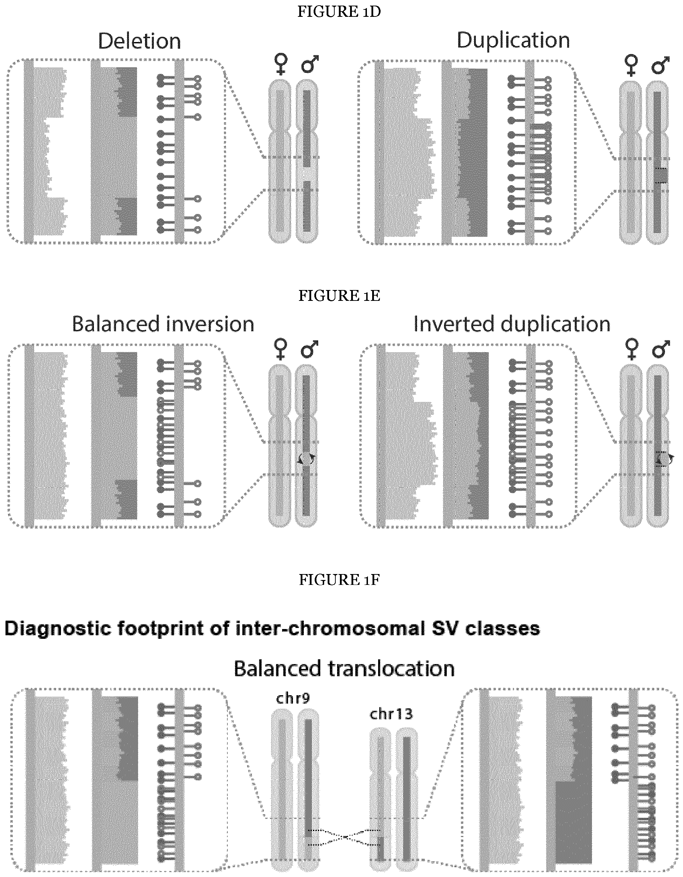

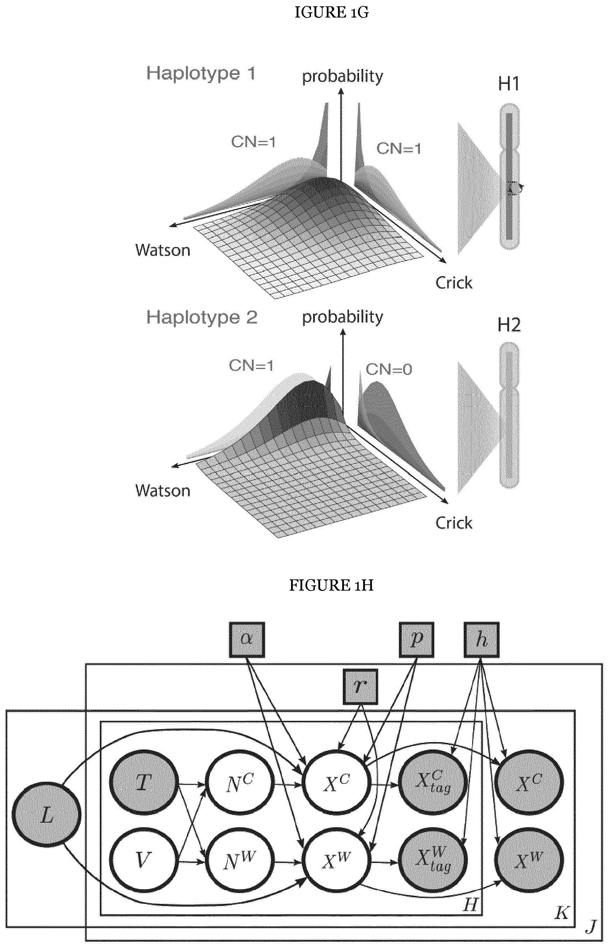

The inventive approach exploits Strand-seq to perform haplotype-aware detection of somatic variation in single cells. Detected classes of variation include deletions, duplications, inversions, translocations, complex SV classes, copy-number neutral losses in heterozygosity (CNN-LOH) and cellular ploidy alterations. The inventive approach leverages patterns of mitotic segregation of template strands (*i.e.* chromatid segregation patterns), which reflect a 'genetic signal' not previously considered for detecting SVs in cellular populations. The invention leverages this information by analyzing in each single cell three orthogonal data layers (or 'channels') - read depth, strand orientation and haplotype phase - the integration of which yields a set of discriminative SV diagnostic footprints via a novel approach according to the invention that is herein termed 'three-channel processing' (**Figure 1**). The inventive approach surprisingly does not require read pairs traversing the SV breakpoints, which renders the approach amenable to scalable low pass sequencing strategies with low sequencing coverage as is the case in single cell sequencing, and enables the detection of SVs flanked by repeat sequence. Herein the examples showcase utility through analysis of cell lines and primary leukemias, revealing previously unresolved or incompletely resolved variant classes in conjunction with repeat-associated and punctuated-equilibrium like SV formation, and resolving subclones defined through single cell SV profiles. The invention will open up a range of research opportunities by enabling scalable, cost-efficient analyses of a wide variety of SV classes in single cells.

In the following terms used in context of the invention shall be defined in detail, which definitions often will comprise specifically preferred embodiments of the herein described invention with respect to such terms. For such embodiments or preferred definitions of certain terms, the above said with respect to combination of embodiments and aspects equally apply.

The term "sequencing data" shall refer to data obtained by sequencing a polynucleotide and wherein such sequencing data comprises a multiplicity of sequences reads, and each sequence read is derived from sequencing a template polynucleotide strand. In preferred embodiments of the invention the template polynucleotide strand is a forward or reverse (W or C) strand.

The term "sequence read" as used herein refers to a nucleotide sequence obtained from or read from a nucleic acid molecule obtained from a biological cell or virus. Sequence reads can be obtained through various methods known in the art. Generally, sequence reads are obtained post- amplification (e.g., polymerase chain reaction, such as bridge amplification) of a nucleic acid fragment that is obtained or enriched from a test sample. The length of sequence reads may vary depending on the sequencing method used. Preferred lengths of a sequence read usable in context of the invention are 50 to 500 nucleotides long, preferably around 100 to 200 nucleotides.

Sequencing methods usable in context of the invention are selected from any methods known to the skilled person. However, currently so called "next generation sequencing" approaches are preferred and include so-called parallelized sequencing-by-synthesis or sequencing-by-ligation platforms currently employed by for example Illumina, Life Technologies, and Roche, or electronic-detection based methods such as Ion Torrent technology commercialized by ThermoFisher, etc. Sequencing methods may also include so called "third generation sequencing (TGS)" technologies such as nanopore sequencing methods. Other approaches include "single molecule real-time (SMRT)" sequencing (for example by Pacific Biosciences), and so called "long-read sequencing" that is capable of obtaining sequence reads longer than 1kb. These both provide what's conventionally termed long-read sequence data (i.e. sequence reads >1000 base pairs)

In context of the present invention it is specifically preferred that a sequence of a target chromosomal region (for example of a test cell) is provided as a strand-specific sequence read, or a portion thereof. Such sequence read, or portion thereof, retains the strand-specific information of for example the template strand of the chromosomal region from which the read was sequenced, and which was inherited by the sequenced single cell following mitosis of the mother cell. Such template strands, as will be further explained herein, can either be a forward or reverse, or often also referred to as Watson or Crick. Any method that will allow for a retaining of the information of strand identity shall be comprised by, and suitable for, the methods of the present invention, as essential is only the strand specific information and not the method of how the information of strand identity is obtained. One way of retaining strand identity during sequencing is by strand-specific sequencing or "Strand-seq". The method is described in detail in Falconer et al. 2012 Nature Methods. 9 (11): 1107-1112, which shall be incorporated herein by reference in its entirety. Specifically incorporated herein by reference is the methods section of the publication. In brief, Strand-seq involves the use of BrdU nucleotides for one synthesis phase (S-phase) of a cell so that before mitosis the newly-generated sister chromatids of each chromosomes are in one strand marked by the incorporated BrdU nucleotides and in the other strand (template strand) devoid of BrdU. After mitosis, the daughter cells are treated such that the BrdU strand is nicked and thus only the non BrdU-labelled strand can be amplified during PCR. Using specific adapters, the original template strand information is retained in the amplified fragments such that only the strand identity of the template strand can be ascertained following sequencing. Aligning the so obtained sequence reads to a reference genome scaffold then indicates the direction of the read and from which strand - Watson or Crick - the read was obtained.

The term "karyotype" refers to the genomic characteristics of an individual cell or cell line of a given species or test sample; e.g., as defined by both the number and morphology of the chromosomes. Typically, the karyotype is presented as a systematized array of prophase or metaphase (or otherwise condensed) chromosomes from a photomicrograph or computer-generated image. Alternatively, interphase chromosomes may be examined as histone- depleted DNA fibers released from interphase cell nuclei. In one embodiment, the karyotyping methods of this invention are specifically suitable for the detection of copy-number neutral SVs. The methods of the invention may also be used to determine Copy-Number Polymorphisms (or also referred to "copy number variations") in a test cell or a test genome. Since the Sequence-Based Karyotyping methods may be performed on prokaryotic cells, the presence of chromosomes is not essential for the methods of the invention.

As used herein the terms "structural variation", "SV", "chromosomal aberration" or "chromosome abnormality", are used interchangeably, and refer to a deviation between the structure of the subject chromosome or karyotype and a normal (i. e., "non-aberrant") homologous chromosome or karyotype. The terms "normal" or "non- aberrant", when referring to chromosomes or karyotypes, refer to the predominate karyotype or banding pattern found in healthy individuals of a particular species and gender. SVs detectable by the methods of the present invention are preferably large or medium sized SVs (200kb or larger).

SVs can be numerical or structural in nature, and include aneuploidy, polyploidy, inversion, balanced or unbalanced translocation, deletion, duplication, inversion-duplication, and the like. SVs may be correlated with the presence of a pathological condition (e.g., trisomy 21 in Down syndrome, chromosome 5p deletion in the cri-du-chat syndrome, and a wide variety of unbalanced chromosomal rearrangements leading to dysmorphology and mental impairment, as well as proliferative disorders and in particular cancer) or with a predisposition to developing a pathological condition. Chromosome abnormality also refers to genomic abnormality for the purposes of this disclosure where the test organism (e.g., prokaryotic cell) may not have a classically defined chromosome.

Furthermore, chromosome abnormality includes any sort of genetic abnormality including those that are not normally visible on a traditional karyotype using optical microscopes, traditional staining, of FISH. One advantage of the present invention is that chromosomal abnormality previously undetectable by optical methods or even sequencing methods (e.g., abnormalities involving 4 Mb, 600 kb, 200 kb, 40 kb or smaller) can be detected due to the integration of the three layers of information.

For purposes of the present invention, the term "copy-number variations (CNVs)" refers to a form of structural variation of the DNA of a genome that results in the cell having an abnormal or, for certain genes, a normal variation in the number of copies of one or more sections of the DNA. CNVs correspond to relatively large regions of the genome that have been deleted (fewer than the normal number) or duplicated (more than the normal number) on certain chromosomes. Correspondingly, the term "copy number neutral" shall denote a variation that does not result in the cell having unusual copy numbers of sequence elements such as genes.

The term "diagnostic footprint" in context of the present invention shall mean a pattern of the three layered information of the invention that is specific or at least indicative for a SV. A diagnostic footprint is therefore characterized by an alteration of the data distribution expected for a specific experiment. The specific pattern that indicates a SV will vary depending on the analysed data. For example a diploid cell may be sequenced to contain for each chromosome a WW, CC or WC strand distribution. Depending on the strand distribution, the same SV may have a different diagnostic footprint. Such footprints or patterns are for example provided herein in table 1.

In context of the herein disclosed invention the term "target chromosomal region" shall refer to a DNA sequence of one or more, full or partial, chromosomes of any organism or virus, which is the object of an inquiry in context of the invention. A target chromosomal region may refer to just one sequence of a part of a single chromosome, or to both the paternal and maternal region of any chromosome. In some embodiments the target chromosomal region which is the object of an inquiry according to the invention is a whole chromosome or a whole genome of a single cell, or a plurality of a single cell.

In context of the herein disclosed invention the term "single cell" shall refer to one individual cell from which by for example strand-specific sequencing, a single cell library is generated. A single cell library in context of the invention describes the plurality of sequence reads obtained by sequencing the genome of said single cell. Furthermore, the invention in some aspects and embodiments refers to a plurality of single cells, or multiplicity of single cells, which in this case refers to the generation of a plurality of separate and independent sequence libraries for each single cell contained in the plurality of single cells. In one preferred embodiment of the invention, up to 96 single cells of a cell line are sequenced individually. Such embodiments are preferred as such assays can be performed in multiwall plates such as 96 well plates or 384 well plates.

The term "reference sequence of the at least one target chromosomal region" refers to a database version of a fully sequenced reference of the target. Usually, such reference will be a full chromosome sequence. In some instances the reference sequence is also denoted as "reference scaffold" or "reference genomic scaffold" or "reference assembly" or similar expression. For human sequences for example the *The Genome Reference Consortium* frequently publishes and updates the reference sequence of the human genome, as well as other genomes such as mouse, zebrafish and chicken genomes (https://www.ncbi.nlm.nih.gov/grc).

The term "reference state" in context of the present invention shall refer to state or distribution of sequencing data that is used as a reference for a comparison with a sample dataset, for example in order to identify aberrations. Such a reference state may be a real set of sequencing data used as a reference, or may be state of the data that is expected for a certain underlying sampled chromosomal region. Usually a reference state in context of the invention shall pertain to the distribution of sequences within a chromosome, or set of chromosomes (genome), that is expected for a non-aberrant single cell or population of cells. As an example, a reference state of a usual diploid human genome would be a distribution of human chromosomes in somatic cells that is common to a majority of humans. However, in certain aspects and embodiments, the reference state may also contain unusual chromosomal architectures or aneuploidies - the reference state according to the invention is determined based on the samples analysed and questions to be answered with the methods of the invention. As a mere illustrative example, the sample analysed with the method of the invention may be derived from a trisomy 21 individually who is screened for other SVs. Most importantly the term "reference state" in context of the invention shall not be confused with "reference sequence", the latter being defined above and referring to an assembly of sequences that is used for aligning sequence reads.

The term "aligning" or "alignment", of a sequence in context of the herein disclosed invention shall denote the mapping of a strand-specific sequence to a reference scaffold, such as a herein described reference genome or reference chromosome matching the respective strand-specific sequence. Aligning sequence reads, or portions thereof, to the corresponding reference scaffold is well known in the art. Such methods may include Bowtie (Genome Biol, 2009;10(3):R25) or Burrows Wheeler Alignment (BWA) (Bioinformatics, 2009 Jul 15;25(14):1754-60. doi: 10.1093/bioinformatics/btp324). Aligning all sequence reads, or portions thereof, to a reference chromosome scaffold results in positional ordering of the sequence information along both strands of the reference, for example of the at least one target chromosomal region.

As used herein, the term "phasing" refers to the process of determining whether two or more nucleic acid sequences (typically comprising regions of sequence variation) are located on the same nucleic acid template, such as a chromosome or a chromosomal fragment. Phasing may refer to resolving two or more single-nucleotide variants or polymorphisms (SNPs) within a single sequencing read. Preferably, phasing may refer to resolving sequencing data over a large genomic region, or resolving a whole genome sequence.

The term "phased" as used in the context of sequences for two or more polymorphic sites means the sequence present at those polymorphic sites are known whether to be derived from a single chromosome.

The term "phased nucleic acid sequence" as used in the context of a single chromosome refers to nucleic acid sequence of a single chromosome where the nucleic acid sequence is obtained from sequencing of a single chromosome. The term "phased nucleic acid sequence" as used in the context of a single chromosomal fragment refers to nucleic acid sequence of a single chromosomal fragment where the nucleic acid sequence is obtained from sequencing of a single chromosomal fragment.

The term "haplotype" is a contraction of the phrase "haploid genotype", and is presently accepted to mean a set of nucleotide sequence polymorphisms or alleles present on a single maternal or paternal chromosome, usually inherited as a unit. Alternatively, haplotype may refer to a set of single-nucleotide polymorphisms (SNPs) that are linked or present together on a single chromosome. The term haplotype may be used to refer to as few as two alleles or SNPs that are linked or present together on a single chromosome.

The term "haplotype identity" is the correspondence of an observed haplotype in a sequence of interest to a known haplotype of a reference sequence, such as a chromosome. For example, a haplotype identity may correspond to the identity of a sequence to either the maternal or paternal haplotype of a diploid organism. In context of the present invention for each target chromosomal region a haplotype identity "Hi" or "H2" can be assigned corresponding to the observed haplotype distribution of all sequences observed in the library or experiment. In some preferred embodiments the H1 is the haplotype sequenced on one strand, and H2 is the haplotype sequenced on the complementary strand.

"Polymerase chain reaction," or "PCR," means a reaction for the in vitro amplification of specific DNA sequences by the simultaneous primer extension of complementary strands of DNA. In other words, PCR is a reaction for making multiple copies or replicates of a target nucleic acid flanked by primer binding sites, such reaction comprising one or more repetitions of the following steps: (i) denaturing the target nucleic acid, (ii) annealing primers to the primer binding sites, and (iii) extending the primers by a nucleic acid polymerase in the presence of nucleoside triphosphates. Usually, the reaction is cycled through different temperatures optimized for each step in a thermal cycler instrument. Particular temperatures, durations at each step, and rates of change between steps depend on many factors well-known to those of ordinary skill in the art.

The term "complementary" refers to the ability of polynucleotides to form base pairs with one another. Base pairs are typically formed by hydrogen bonds between nucleotide units in antiparallel polynucleotide strands. Complementary polynucleotide strands can base pair in the Watson-Crick manner (e.g., A to T, A to U, C to G), or in any other manner that allows for the formation of duplexes. The term "complementary" is also used to denote the respective complementary DNA strand. For example referring to the complementary strand of the Watson strand refers to the Crick strand, and *vice versa.*

The term "polynucleotide" or "nucleic acid" refers to polymers of nucleotides of any length, and includes but is not limited to single stranded or double stranded molecule of DNA, RNA, or DNA/RNA hybrids including polynucleotide chains of regularly and irregularly alternating deoxyribosyl moieties and ribosyl moieties (i.e., wherein alternate nucleotide units have an -OH, then and -H, then an -OH, then an -H, and so on at the 2' position of a sugar moiety), and modifications of these kinds of polynucleotides wherein the substitution or attachment of various entities or moieties to the nucleotide units at any position, as well as naturally-occurring or non-naturally occurring backbones, are included. A polynucleotide may be further modified after polymerization, such as by conjugation with a labeling component. A "fragment" or "segment" of a nucleic acid is a small piece of that nucleic acid. Preferably the polynucleotides used or assayed in context the invention are DNA molecules, such as chromosomes or genomes of eukaryotes.

"Homozygous" state means a genetic condition existing when identical alleles reside at corresponding loci on homologous chromosomes. In contrast, "heterozygous" state means a genetic condition existing when different alleles reside at corresponding loci on homologous chromosomes.

A "gene" refers to a polynucleotide containing at least one open reading frame that is capable of encoding a particular protein after being transcribed and translated.

A "subject," "individual" or "patient" is used interchangeably herein, which refers to a vertebrate, e.g., a mammal, e.g., a human.

The term "amplifying" as used herein refers to generating one or more copies of a target nucleic acid, using the target nucleic acid as a template.

As used herein, the term "genome(s)" means the hereditary information of an individual typically encoded in nucleic acids, either DNA, or RNA, and including both genes and non-coding sequences. The genome may refer to the nucleic acids making up one set of chromosomes of an organism (haploid genome) or both sets of chromosomes of an organism (diploid genome) depending on the context in which it is used.

A "target chromosome pair" as used herein refers to a pair of chromosomes of the same type, where a member of the pair is maternally inherited (inherited from the mother) and the other member of the pair is paternally inherited (inherited from the father). For example, a target chromosome pair refers to a pair of chromosome 1, chromosome 2, chromosome 3, and including up to chromosome 21, chromosome 22, and chromosome X. One or more target chromosome pairs may be simultaneously analyzed by the methods disclosed herein to determine the sequence of the maternally and paternally inherited chromosome of the target chromosome pair.

A "single copy" or "single copies" of a target chromosome pairs as used herein refers to a single physical DNA molecule, either the chromosome per se, or packaged (with the assistance of chromosomal proteins such as histones) in the form of a chromosome. In a normal diploid human cell, there are 46 single chromosomes, 23 single chromosomes from the mother and 23 single chromosomes from the father. Single copies of a target chromosome are also referred to as single copies of a chromosome type. Single copies of one or multiple chromosome types are usually separated into individual containers in the method described herein.

A "chromosome type" as used herein refers to a specific chromosome present in a cell. In a normal diploid human cell of a female, there are 22 types of autosomal chromosomes and one type of sex chromosome (chromosome X). In a normal diploid human cell of a male, there are 22 types of autosomal chromosomes and two types of sex chromosomes (chromosomes X and Y).

The term "polymorphic site" or "polymorphism" as used herein refers to a localized region within a chromosome at which the nucleotide sequence varies from a reference sequence in at least one individual in a population. Sequence variations can be substitutions, insertions or deletions of one or more bases. Polymorphisms that alter the structure of a chromosome or a larger nucleic acid molecule are SV as described herein elsewhere.

As used herein, the term "single nucleotide polymorphism(s) or SNP(s)" means a polymorphic site at which the sequence variation is caused by substitution of a single base at a specific position. SNPs refer to nucleotide variations at a defined genomic position among a population. A SNP within a coding region, in which both forms lead to the same protein sequence, is termed synonymous; if different proteins are produced they are non-synonymous. SNPs may have consequences for gene splicing, transcription factor binding, or the sequence of non-coding RNA, for example, and/or may indicate the haplotype of the organism.

As used herein, the term "hybridization" means one or more processes for co-localizing complementary, single-stranded nucleic acids, and/or co-localizing complementary non-traditional molecules with single- or double-stranded nucleic acids through strand separation (e.g., by denaturation) and re-annealing, for example. In illustrative embodiments, complementary nucleic acid molecules, optionally oligonucleotides, may hybridize to single- or double-stranded DNA. Methods for hybridization are known in the art, and include, but are not limited to, conditions for low and high stringency hybridization (Sambrook and Russell. (2001) Molecular Cloning: A Laboratory Manual 3rd edition. Cold Spring Harbor Laboratory Press; Sambrook, Fritsch, Maniatis. Molecular Cloning: A Laboratory Manual 3rd edition). Stringency of the hybridization may be controlled (e.g. by the washing conditions) to require up to 100% complementarity between the probe and the target sequence (high stringency), or to allow some mismatches between the probe and the target sequence (low stringency). Factors to determine the appropriate hybridization and wash conditions based on the target and the probe are known in the art. In illustrative embodiments, following the first wash using $0.2 \times SSC/0.1\%$ SDS for 10 minutes at 68° C., two additional washes with $0.2 \times SSC/0.1\%$ SDS for 15 minutes each at 68° C. are performed for high stringency washes, two additional washes at $0.2 \times SSC/0.1\%$ SDS for 15 minutes each at 42° C. for moderate stringency washes, and two additional washes $0.2 \times SSC/0.1\%$ SDS for 15 minutes each at room temperature for low stringency washes.

The term "allele" as used herein refers to a particular form of a genetic locus, or a genomic region, or an entire chromosome, distinguished from other forms by its particular nucleotide sequence.

The term "locus" as used herein refers to a location on a chromosome or DNA molecule corresponding to a gene or a physical or phenotypic feature.

The term "sample" as used herein relates to a material or mixture of materials, typically, although not necessarily, in liquid form, containing one or more analytes of interest, which is in the present context of the invention a sample containing cellular material or at least genomic material of one or more cells. The term "chromosomal sample" as used herein relates to a material or mixture of materials, containing chromosomes from a subject. Similar the term "genomic sample" relates to a material or mixture of materials, containing genomic material from a subject or cell.

The term "assigning" with regard to information in context of the present invention shall mean that any kind of information is connected to a certain sequence entity such as a predetermined or preselected window of the reference scaffold, or a sequence read. Preferably, numbers of observed or mapped reads or portions of reads are assigned as information in accordance with the herein disclosed three channels (i) to (iii).

A "sequence window" means a section of the scaffold sequence into which one or more sequence reads, or portions thereof, can be mapped during the alignment. The size of the sequence window is selected depending on the coverage of the sequencing data, or arbitrarily chosen depending on the application of the methods of the invention. In context of the present invention a sequence window may have a size of 1 to 50 kb, or preferably 1 to 10kb or most preferably about 1, 2, 3, 4, 5, 6, 7, 8, 9, 10, 11, 12, 13, 14, 15, 16, 17, 18, 19, or 20 kb. Windows of the invention may also be larger such as 50kb, 100kb, 200kb or 500kb. An example window in accordance with the herein presented examples is about 50kb.

The term "three layered information" means in context of the invention the integration of three separate channels of information that can be derived from strand specific sequencing in combination with haplotype phasing of the sequence read information.

For the purposes of the present invention, the term "coverage," refers to the average number of reads representing a given nucleotide in the reconstructed sequence. It can be calculated from the length of the original genome (G), the number of reads (N), and the average read length (L) as $N \times L/G$. For example, a hypothetical genome with 2,000 base pairs reconstructed from 8 reads with an average length of 500 nucleotides will have about two times ($2 \times$) of redundancy. This parameter also enables one to estimate other quantities, such as the percentage of the genome covered by reads (sometimes also called coverage). One of the advantages of the present invention is a stable identification of SV within a target sequence of a single cell which sequenced with a coverage of only 0.01x compared to 30x which is usually a coverage obtained by sequencing the genomic material of over 1000 cells. In context of the present invention it may be preferred that sequence reads have an overall coverage of 0.001x to 100x, preferably about 0.01x to 0.05x, of the target chromosomal region.

The term "germ line" refers to cells in an organism which can trace their eventual cell lineage to either the male or female reproductive cells of the organism. Other cells that are referred to as "somatic cells" are cells which do not directly give rise to gamete or germ line cells. Both germ line cells and somatic cells may be used in some embodiments of the invention depending on the application.

The terms "chromosome instability" (CIN) and "genomic instability" and similar expressions as used herein, pertain to the number or degree of chromosome structural and numerical abnormalities, i.e. deletion or duplication of either whole chromosomes or parts of chromosomes, for example leading to aneuploidy (incorrect number of chromosomes). A high CIN is often associated with or detected in proliferative disorders such as cancer.

In context of the present invention the term "read depth", or "depth" shall refer to the number of reads mapping into the predetermined or preselected sequence window.

The terms "diagnostic signature" or "diagnostic footprints" or similar expression shall in context of the invention refer to an expected difference or signal an SV or other aberration causes in the sequencing data analysed according to the invention compared to the reference state. Examples for SVs in diploid genomes are provided in table 1 herein. However, understanding the genetic patterns of inheritance one skilled in the art will be able to determine any other diagnostic signature or diagnostic footprint depending on the underlying situation.

The term "ground state" when used in context of the invention denotes the distribution of parental template strands within a single cell or a population of single cells. Hence, in preferred embodiments of the invention a ground state shall denote whether a single cell comprises any number of W or C template strands. In a diploid scenario, as a non-limiting example, the ground state could be WW, CC, WC or CW (see also table 1).

In some embodiments, the strand-specific sequence data is provided in order to commence the method of the present invention. In other embodiments, also preferred, the method might include preparatory steps to prepare or filter the sequence data or even obtain the sequence data by strand sequencing of a sample comprising genetic material of the target chromosomal region.

In one embodiment the strand specific sequence data may already include sequence reads, or portions thereof, that are mapped to a reference scaffold. In other embodiments the sequence reads, or portions thereof, are mapped or aligned to the corresponding reference scaffold using standard aligning tools known in the art.

Preferably, as a first step of the scTRIP specific method, reads across each individual cell or experiment are assigned to windows ("binned") of a given width. In some embodiments the width of the window is selected depending on the coverage and the specific conditions of the data or application. Preferred lengths of windows are described herein elsewhere. In some embodiments, which are preferred, the mapped reads are assigned to windows based on their start position; however, other reference positions might also be used. Further, in some embodiments, a strand state is assigned to each of said windows, which indicates the template strand distribution or relative abundance of W and C reads, for the chromosomal region. In disomic datasets, the strand state is indicated as WW (Watson-Watson), CC (Crick-Crick) or WC (Watson-Crick). In preferred embodiments the strand state assignment may be performed using a hidden Markov model (HMM).

Optionally, the invention may include various steps of quality control and data normalization according to the specific methods used in example 1 herein.

In preferred embodiments of the present invention, the herein disclosed methods integrate all three channels of information, such as depth, orientation and haplo-phase. Therefore, preferably in step (d) all three channels of sequence information (i) to (iii) are assigned to the at least one predetermined sequence window.

In some embodiments the strand-specific sequence data comprises sequence reads which are derived from one of at least two separate strands of the at least one target chromosomal region, preferably the strand-specific sequence data comprises further sequence reads which are derived from the other of the at least two separate strands, for example wherein one strand is from the paternal and the other strand is from the maternal chromosome (but could further comprise sequence reads derived from additional strands, as in the case of triploidy, etc.). Hence, in some embodiments of the method of the invention in step (b) each sequence read, or portion thereof, is aligned with the direction forward or reverse which retains strand-specific sequence information.

In some embodiments of the invention, the method may comprise of identifying strand state and/or detecting sister chromatid exchanges (SCE) in the sequence data. During the step of strand state detection it is in some embodiments preferred to simultaneously detect SCEs. Using Strand-seq, each chromosomal homologue within a single cell is sequenced either on the W or the C strand (leading to the observed WW, WC, or CC strand patterns in the case of diploidy). Strand state detection and SV discovery is improved by detecting SCE events (with typically ~6 SCEs seen per diploid cellular genome), which can flip the strand state of a homologue along chromosomes.

In some embodiments the methods of the invention may comprise a step of segmenting the at least one target chromosomal region, wherein the segmenting is performed on basis of the channels of sequence information (i) to (iii), each individually or together. In principle, segmenting seeks to identify breakpoints in the information distribution along the target chromosomal region, and thereby identify boundaries of candidate SVs. Since the invention uses also strand-specific sequence data, also the breakpoints of candidate SVs that are copy-number neutral can be detected during segmenting. In embodiments where a population of single cell sequence data is analysed, it is preferable to segment across all cells simultaneously. In some embodiments such segment is also referred to as a sub-region.

The present invention includes a step of haplotype phasing of the sequences. In some preferred embodiments haplotype phasing classifies WC regions into either WC or CW states, where the first position refers to H1 and the second to H2. Such a step is preferable as this distinction is then used during SV identification to predict SVs in a haplotype-aware manner, which is an advantage of the present invention. In order to perform this distinction, in some embodiments whole-chromosome haplotypes of at least a couple of dozen SNVs are used; these can be obtained from an external data source or will alternatively be identified in the strand-specific sequence data directly as a step of the method of the invention. Given a list of heterozygous SNVs, as a non-limiting example, the workflow of the present invention may include the StrandPhaseR algorithm (Porubsky, D. et al. Dense and accurate whole-chromosome haplotyping of individual genomes. Nat. Commun. 8, 1293 (2017)) to generate chromosome-scale haplotypes (see Methods section in the examples for details). In some embodiments of the invention step (c) involves that a chromosomal haplotype identity (H1/H2) along the at least one target chromosomal region is assigned to any of the given reads by assigning Single Nucleotide Polymorphisms (SNP), preferably wherein such SNPs do not have a disease association. Such assigning is referred to herein in some instances as "haplotype-tagging" the sequence read. In some embodiments of the invention the haplotype identity of all reads derived from a single strand (W or C) are assigned a haplotype identity (H1/H2) based on some of the reads from the same strand which contain or overlap with one or more SNPs. This embodiment allows to haplo-phase reads which do not contain or overlap with any SNPs.

It is particularly preferred that the haplotype phasing is performed in a "strand-aware manner". In context of the invention such embodiment shall entail that the assigned haplotype identity of any given sequence read is connected to the information which orientation the same sequence read has. Therefore in a preferred embodiment of the invention the information of channel (ii) and (iii) for each sequence read, or portion thereof, are connected.

In some instances, which may be preferred, the sequencing data comprises a multitude of non-overlapping and/or overlapping sequence reads. However, in particular in case of single cell analysis, which usually entails low sequencing coverage, read duplicates which are often artefacts, for example by PCR, are removed. Thus in preferred embodiments the strand-specific sequence data does not comprise overlapping sequence reads.

As already described herein, the methods of the invention are useful for the detection of various SV. Therefore, preferably the method of the first aspect may include a step (e) of identifying a structural variation (SV) by performing step (d) for a multiplicity (at least two) of windows within the sequence data of the positional ordered and aligned sequence reads, and identifying within the multiplicity of windows a sub-region comprising one or more windows having an unusual/altered/changed distribution of the information of any one, or all of, or any combination of, channels (i) to (iii). The unusual/altered/changed distribution in this invention is preferably any of the herein disclosed diagnostic footprints which are indicative of one or more SVs. Such diagnostic footprints in accordance with the invention are described in the following:

The diagnostic footprints for SV detection in the integrated data of the present invention take three data layers into account - read depth, read orientation and phase. In some embodiments in addition to a single cell, a population of single cells may be analysed in order to increase detection and/or discrimination between two different SV classes achieving similar SV likelihoods, for example haplotype-tags, or "haplotagging" (phased reads containing a heterozygous SNPs) may preferably also be considered for classification. In embodiments with absence of haplotype tags, the methods of the invention consider total segment coverage (here represented as ploidy level; for example $N$=2 represent disomy or the same copy number as the reference) and the fraction of Watson reads (abbreviated as 'W. frac', and computed as W/(W+C)). The SV discovery signatures developed for this invention depend on the underlying strand state of a target chromosomal region, and whether an SV is homozygous or heterozygous - *i.e.* they are different in WC, CW, WW or CC chromosomal regions, and for homozygous versus heterozygous duplications, for example. Table 1 shows an overview of SV diagnostic footprints in the context of heterozygous and homozygous SVs, and for different patterns of mitotic strand segregation, and such footprints are preferred embodiments of the invention:

**Table 1:** Diagnostic Footprints according to the invention

| SV diagnostic footprints in a WC ground state | | | | | | |
|---|---|---|---|---|---|---|
| | | | Haplotype tags | | | |
| SV state | Depth | W:C | W | C | W cov | C cov |
| Reference state | 2N | 50% | H1 | H2 | 1N | 1N |
| Deletion of H1 | 1N | 0% | - | H2 | 0N | 1N |
| Deletion (homozygous) | 0N | - | - | - | 0N | 0N |
| Duplication of H1 | 3N | 66% | 2xH1 | H2 | 2N | 1N |
| Duplication (homozygous) | 4N | 50% | 2xH1 | 2xH2 | 2N | 2N |

(continued)

| SV diagnostic footprints in a WC ground state | | | | | | |
|---|---|---|---|---|---|---|
| | | | Haplotype tags | | | |
| SV state | Depth | W:C | W | C | W cov | C cov |
| Inversion of H1 | 2N | 0% | - | H1+H2 | 0N | 2N |
| Inversion (homozygous)[1] | 2N | 50% | H2 | H1 | 1N | 1N |
| Inverted duplication of H1[2] | 3N | 33% | H1 | H1+H2 | 1N | 2N |

| SV diagnostic footprints in a CW ground state | | | | | | |
|---|---|---|---|---|---|---|
| | | | Haplotype tags | | | |
| SV state | Depth | W:C | W | C | W cov | C cov |
| Reference state | 2N | 50% | H2 | H1 | 1N | 1N |
| Deletion of H1 | 1N | 0% | H2 | - | 1N | 0N |
| Deletion (homozygous) | 0N | - | - | - | 0N | 0N |
| Duplication of H1 | 3N | 66% | H2 | 2xH1 | 1N | 2N |
| Duplication (homozygous) | 4N | 50% | 2xH2 | 2xH1 | 2N | 2N |
| Inversion of H1 | 2N | 0% | H1+H2 | - | 2N | 0N |
| Inversion (homozygous)[1] | 2N | 50% | H1 | H2 | 1N | 1N |
| Inverted duplication of H1[2] | 3N | 33% | H1+H2 | H1 | 2N | 1N |

| SV diagnostic footprints in a WW ground state | | | | | | |
|---|---|---|---|---|---|---|
| | | | Haplotype tags | | | |
| SV state | Depth | W:C | W | C | W cov | C cov |
| Reference state | 2N | 100% | H1+H2 | - | 2N | 0N |
| Deletion of H1[3] | 1N | 100% | H2 | - | 1N | 0N |
| Deletion (homozygous) | 0N | - | - | - | 0N | 0N |
| Duplication of H1[3] | 3N | 100% | 2xH1+H2 | - | 3N | 0N |
| Duplication (homozygous) | 4N | 100% | 2xH1+2xH2 | - | 4N | 0N |
| Inversion of H1[3] | 2N | 50% | H2 | H1 | 1N | 0N |
| Inversion (homozygous) | 2N | 0% | - | H1+H2 | 0N | 2N |
| Inverted duplication of H1[3] | 3N | 67% | H1+H2 | H1 | 2N | 1N |

| SV diagnostic footprints in a CC ground state | | | | | | |
|---|---|---|---|---|---|---|
| | | | Haplotype tags | | | |
| SV state | Depth | W:C | W | C | W cov | C cov |
| Reference state | 2N | 0% | - | H1+H2 | 0N | 2N |
| Deletion of H1[3] | 1N | 0% | - | H2 | 0N | 1N |
| Deletion (homozygous) | 0N | - | - | - | 0N | 0N |
| Duplication of H1[3] | 3N | 0% | - | 2xH1+H2 | 0N | 3N |
| Duplication (homozygous) | 4N | 0% | - | 2xH1+2xH2 | 0N | 4N |
| Inversion of H1[3] | 2N | 50% | H2 | H1 | 1N | 1N |
| Inversion (homozygous) | 2N | 100% | H1+H2 | - | 2N | 0N |
| Inverted duplication of H1[3] | 3N | 33% | H1 | H1+H2 | 1N | 2N |

[1] Cannot be distinguished from a reference state in WC chromosomes* (yet can be resolved for CC and WW chromosomes, and hence when assessing subclonal SVs in a cell population)

[2] Cannot be distinguished from a heterozygous duplication in WC chromosomes* (yet can be resolved for CC and WW chromosomes, and hence when assessing subclonal SVs in a cell population)

[3] Cannot be phased in WW or CC chromosomes* (yet can be resolved for WC chromosomes, and hence when assessing subclonal SVs in a cell population)

As already explained for segmenting, said sub-region or segment may be defined by at least one but preferably two breakpoints, and wherein such breakpoints indicate a change of any one, or any combination, or all, of the information of channels (i) to (iii) compared to the reference state and/or compared to an overall distribution of said channel information within in the sequence data.

In some embodiments said reference state of said chromosomal region is a state of the information of the channels which is expected for a non-aberrant distribution and/or predetermined state of the information of said chromosomal region.

In some embodiments the reference state in a target diploid chromosomal region is in the event the diploid target chromosomal region comprises a first template strand derived from the first parental target chromosomal region and a second template strand derived from the second parental target chromosomal region; said reference state is:

If the first parental target chromosomal region is Watson (W), and the second parental target chromosomal region is Crick (C) - the **WC reference state:**

Channel (i): number of total reads correspond to the presence of about 2x the target chromosomal region (2N);

Channel (ii): number of reads for each W and C strand correspond to the presence of about 1x the target chromosomal region (1N);

Channel (iii): number of W-reads which are H1 identity correspond to 1x, and number of C-reads which are H2 identity correspond to 1x; or

If the first parental target chromosomal region is C, and the second parental target chromosomal region is W - the **CW reference state:**

Channel (i): number of total reads correspond to the presence of about 2x the target chromosomal region (2N);

Channel (ii): number of reads for each W and C strand correspond to the presence of about 1x the target chromosomal region (1N);

Channel (iii): number of W-reads which are H2 identity correspond to 1x, and number of C-reads which are H1 identity correspond to 1x; or

If the first and the second parental target chromosomal region is W - **the WW reference state:**

Channel (i): number of total reads correspond to the presence of about 2x the target chromosomal region (2N);

Channel (ii): number of reads for the W strand correspond to the presence of about 2x the target chromosomal region (2N), and wherein only residual (oN) reads are present;

Channel (iii): number of W-reads which are H1 identity correspond to 1x, and number of W-reads which are H2 identity correspond to 1x, and wherein only residual reads are present corresponding to oN; or

If the first and the second parental target chromosomal region is C - the CC reference state:

Channel (i): number of total reads correspond to the presence of about 2x the target chromosomal region (2N);

Channel (ii): number of reads for the C strand correspond to the presence of about 2x the target chromosomal region (2N), and wherein only residual W reads are present corresponding to oN;

Channel (iii): number of C-reads which are H1 identity correspond to 1x, and number of C-reads which are H2 identity correspond to 1x, and wherein only residual W reads are present corresponding to oN;

wherein the SV is detected if there is a variation from the reference state, and optionally, wherein the SV is classified in accordance to a variation indicated in table 1.

In particular preferred is that any of the SV mentioned in table 1 are detected based on the indicated diagnostic footprint such SV would display depending on the respective ground state of the cell.

In some embodiments the SV is an altered ploidy state, and wherein the sequence data comprise a multiplicity of target chromosomal regions of different chromosomes, and wherein the altered ploidy state is identified by a difference in overall distribution of any one, all of, or any combination of, the information of channels (i) to (iii), between a candidate polyploidy chromosomal region of one chromosome compared to one or more other chromosomal regions of other chromosomes. Preferably, the method of the invention involves determining the distribution of W and C strands in a population of single cells and deriving therefrom, the ploidy state for each target chromosomal region, preferably target chromosome.

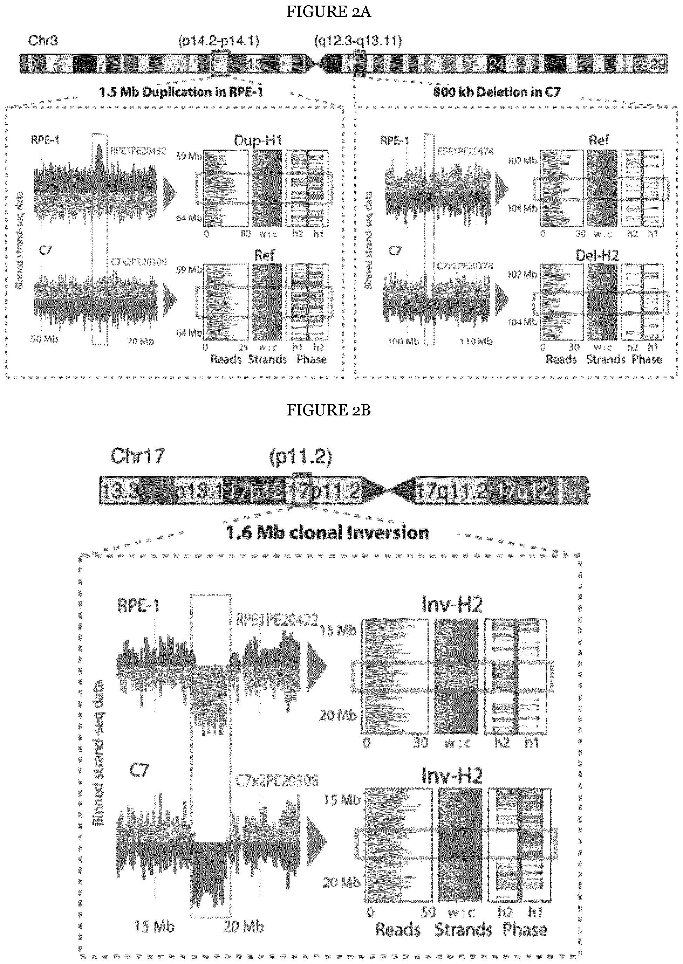

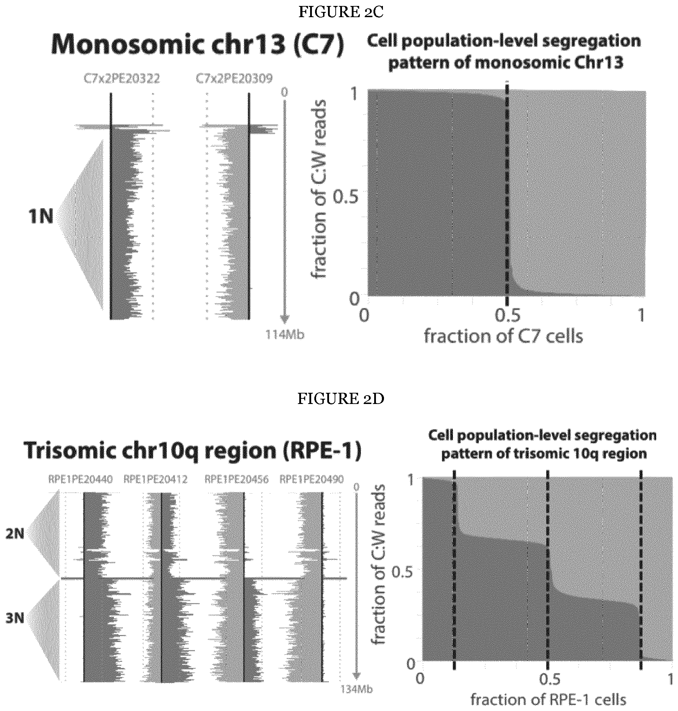

A detailed description of the identification of an aneuploidy is provided in the example section. The detection of ploidy states of a target chromosomal region of a single cell is based on the fact that in a diploid cell sequenced by Strand-seq, demonstrate random and independent mitotic segregation of replicated chromosomes to the resulting daughter cells. This implies that approximately 50% of all autosomes will show a characteristic pattern where one homolog is sequenced on the plus strand (here W, for Watson) and the other homolog is sequenced on the minus strand (C, for Crick) - hereafter termed WC-pattern. The remaining autosomes are sequenced either only on the C strand (approximately 25%; CC-pattern), or only on the W strand (approximately 25%; WW-pattern), respectively (Fig. 2). A binomial distribution (see example section) can be used to compute expected frequencies of autosomal strand patterns for different cellular ploidy states. In a triploid cell, for example, the CCC-pattern (all reads of an autosome map to the C-strand) and the WWW-pattern (all reads map to the W-strand) will be seen for 12.5% of all autosomes, respectively. The CWW-pattern and the CCW-pattern, respectively, will each be seen for 37.5% of all autosomes. Tetraploidy and haploidy, by comparison, will result in their own discernible strand patterns (Table 2). These distinct strand state patterns (i.e. relative abundances of W and C reads) and/or expected frequencies of strand inheritance patterns for a given chromosomal region can be used to identify aneuploidies in the sample. Different from existing methods, these diagnostic footprints do not require additional data (such as the detection of additional somatic variants in a given cell) for making ploidy assignments, and as such are much more powerful and applicable for detecting potentially pathogenic ploidy alterations in cells.

Diagnostic footprints characteristic for several cellular ploidy states are shown in table 2. A binomial distribution can be used to compute expected frequencies of autosomal strand patterns for different ploidy states. W, Watson strand of the genome. C, Crick strand.

**Table 2: Diagnostic strand patterns (footprints) for aneuploidies**

| Ploidy state | Strand patterns observed | | | | |
|---|---|---|---|---|---|
| **Haploid** | C | W | | | |
| Strand-ratios: 1:0 | 50% | 50% | - | - | - |
| **Diploid** | CC | CW | WW | | |
| Strand-ratios: 1:1, 2:0 | 25% | 50% | 25% | - | - |
| **Triploid** | CCC | CCW | WWC | WWW | - |
| Strand-ratios: 2:1, 3:0 | 12,50% | 37,50% | 37,50% | 12,50% | |
| **Tetraploid** | CCCC | CCCW | CCWW | CWWW | WWWW |
| Strand-ratios: 4:0, 3:1, 2:2 | 6,25% | 25% | 37,50% | 25% | 6,25% |

The method of detecting cellular ploidies is preferably a method wherein at least strand-specific sequence data and read depth are used. More preferably also the haplotype phase is integrated.

In other embodiments, the detection of ploidies involves that a strand-specific sequence data comprises data derived from a population of individual cells to allow detection of the distribution of the W and/or C strands. The more single cell data included the more complex aneuploidies can be detected by the method of the present invention.

In some embodiments, the method of the invention is performed with strand-specific sequence data of the at least one target chromosomal region of at least two or more single cells, preferably 10 or more, more preferably 50 or more, most preferably 90 or more or 350 or more; and preferably, wherein the multiplicity of single cells is derived from the same or identical origin, such as the same individual and/or the same tissue or sample type. Such population or multiplicity of single cells are preferably of the same origin and expected to share said polyploidy and/or translocation. A polyploidy or translocation is preferably detected if the distribution of the strand-orientation within the population is altered from the expected pattern. In some embodiments a polyploidy is detected if the distribution of sequenced forward or reverse strands for each chromosome differs from the overall distribution expected for diploid chromosomal (autosomal) segregation, such as 50% WC, 25% WW and 25% CC.

A cell, or a single cell, in context of the invention may be any biological cell, or cell-like structure, comprising a polynucleotide genome or parts thereof. A cell therefore may be a virus, a prokaryotic cell, or a eukaryotic cell, such as an animal or plant cell, preferably wherein the animal cell is a mammalian cell such as a mouse, rat or human cell. Any cell type or any cell of any tissue origin may be used for the present invention. Preferably the at least one single cell is obtained from a cellular sample of a patient, and wherein said single cell is either a cell associated with a disease, or is a healthy cell of said patient, preferably wherein the method is performed for a multiplicity of single cells associated with the disease and/or healthy cells.

The methods of the invention are particular useful for diagnosing a disorder, or the probability of a subject to develop a disorder, and finally, in order to stage a disorder or monitor it, or even to estimate disease severity. There are many genetic disorders which are associated with SVs of any kind. Hence, some preferred embodiments of the invention also encompass further step (f) diagnosing a condition based on the identity of, location of, or number of detected SV within the target chromosomal region. A detail of diagnostic applications is provided herein below. In some embodiments the detected SVs of said target chromosomal region may be compared with a known reference state of said chromosomal region, such as a known state of the chromosomal region of a healthy cell. In addition, in order to detect possible pathological impacts of a SV the invention may include detecting SV-affected genes or genetic elements within the target chromosomal region. Since the invention identifies the chromosomal location of each detected SV, it may be a preferred embodiment to further identify genetic elements, preferably genes, that are affected by the SV, for example if their open reading frame is disrupted by the breakpoint of the SV, or by copy number alteration, or by impairment of any regulatory element in the gene region.

Any method according to the herein disclosed invention is in some preferred embodiments an *in-vitro* method, and/or is an *in-silico* method.

In some further embodiments, as already described herein elsewhere, the method is performed with a multiplicity of single cell libraries. In such embodiments the method may further comprises a step of calculating a probability of occurrence of a SV at a given position, for example by using a Bayesian network of any one, any combination of or all channels (i) to (iii), of the analysed single cell population.

## Karyotyping using scTRIP

Karyotyping a genome is a valuable method in both clinical practice and research. Either to diagnose genetic abnormalities in a patient, or a disease associated tissue, or embryonic cells in reproductive medicine. In research karyotyping allows the study of such SVs, evolutionary events and inheritance patterns of phenotypes. Traditional karyotyping is usually performed on lymphocytes and amniocytes using labor intensive methods such as Giemsa staining (G-banding). Because chromosomes are visualized on an optical microscope, the ability to resolve detailed mutations (involving only a small part of a chromosome) is limited. While more detailed karyotyping techniques, such as FISH (fluorescent ire situ hybridization) are available, they rely on specific probes and it is not economically or technically feasible to perform FISH on the entire chromosome set (i.e., the complete genome).

Hence, the object of the invention is solved in another aspect by a method of karyotyping a single cell, or a population of multiple single cells, or a subject from which such cells are obtained, the method comprising,

(a) Providing strand specific sequence data of the at least one target chromosomal region, preferably the complete genome, of at least one single cell, or each of the population of single cells,

(b) Performing a method of scTRIP as described herein elsewhere,

(c) Detecting one or more SVs within the target chromosomal region of said single cell, or the population of single cells, and

(d) Obtaining an in-silico karyotype based on all detected SVs from the output of the scTRIP method; for example the karyotype may be visualized by location, probability and/or type of SV on a schematic representation of the analysed genome. Such representation may correspond to the analysed genome in a state where chromosomes are in their metaphase or prometaphase. An example of such an in silico karyotype is provided in the figures.

Preferably the method includes performing the method of scTRIP with a population of cells in order to obtain a comprehensive karyotype for example including possible translocations and aneuploidies as well as the possibility to obtain an allelic frequency of all SVs that are found within the population of cells.

Hence additionally provided is a method of karyotyping a genome of at least one single cell of interest, comprising: a) obtaining a plurality of (preferably non-overlapping) strand specific sequences from random locations of the genome of the at least one single cell; b) mapping said test strand specific sequences to a genomic reference scaffold to obtain a test distribution of mapped strand specific sequences; c) assigning to a predetermined sequence window within the reference scaffold (i) number of mapped sequence reads, (ii) number of mapped forward strand reads and number of reverse strand reads, preferably a ratio thereof, and (iii) assigning a haplotype identity (H1/H2) to the strand-specific reads, to obtain a three layered test distribution of mapped sequences; d) identifying a statistically significant alteration between an expected distribution, wherein such an alteration indicates a karyotypic abnormality in the genome of the at least one single cell; or e) comparing the three layered test distribution to a reference distribution obtained from a reference cell (such as a healthy cell), wherein if a significant difference is present said difference indicates a karyotypic difference between the at least one single cell and the reference cell

In one aspect the present invention also pertains to the output data of the method of karyotyping.

**Diagnostics**

Many diseases and disorders are associated with structural variations of either the germ line genome or somatically in a cell or tissue associated with the disorder - the most prominent example being cancer which is often associated with genomic or chromosome instability. Often diseases such as cancer are characterised by a high degree of chromosome instability. The term "chromosome instability" (CIN) has been previously defined and applies equally to this aspect of the invention. In a preferred embodiment, the invention pertains to a method of diagnosing a disease associated with unusual or increased CIN (such as cancer). The degree of chromosomal instability can be traditionally quantified in the prior art by determining the number of centromeres for one particular chromosome or several chromosomes. However, the present invention as described herein provides a much faster, cheaper and more comprehensive view on structural variations in any given sample, and thus allows for an improved quantification of CIN. Hence, in preferred aspects and embodiments, the invention also may be used to study genetic stability in various contexts.

The invention therefore pertains in another aspect to a method of diagnosing a disease in subject, the method comprising, providing strand-specific sequence data of one or more cells of the subject, performing a method of scTRIP as described herein, detecting within the one or more cells any SV, and comparing the detected SV with a reference state, wherein an altered number, type or location of one or more SV in the sample of the subject indicated the presence of a condition, such as a disease, for example cancer. In some instances the invention may include a quantification of CIN based on the type and number of SV detected in a sample.

Disorders that can be diagnosed by the methods of the present invention are manifold and include any germ line encoded genetic disorders or disorders associated with somatic genetic events.

Non limiting examples of human genetic disorders associated with an SV are including their genomic locations: 5q11-q13 (Angelman's syndrome), 5p15.2-p15.3 (Cri-du- chat syndrome), 22q11.2 (DiGeorge syndrome), 17p13.3 (Miller-Dieker syndrome), 15q11-q13 (Prader-Willi syndrome), 22q11.2 (Shprintzen syndrome), 17p11.2 (Smith -Magenis syndrome), 7q11.23 (Williams-Beuren syndrome), 4p16.3 (Wolf-Hirschhorn syndrome), 1q21.1 (microdeletion 1q21.1), 1q21.1 (microduplication 1q21.1), 1q41q42 (Microdeletion 1q41q42), 2p15p16.1 (microdeletion 2p15p16.1), 3q29 (microdeletion 3q29), 7q11.23 (microduplication 7q11.23), 9q22.3 (microdeletion 9q22.3), 12q14 (microdeletion 12q14), 14q11.2 (Microdeletion 14q11.2), 15q13.3 (microdeletion 15q13.3), 15q24 (microdeletion 15q24), 16p11.2 (microdeletion / duplication 16p11.2), 16p11.2p12.2 (microdeletion 16p11.2p12.2), 16p13. 1 (microdeletion 16p13.1), 16p13.1 (microduplication 16p13.1), 17p11.2 (Potocki-Lupski syndrome), 17p11.2 (microduplication 17p11.2), 17q21.31 (microdeletion 17q21.31), 19q13.11 (microdeletion 19q13.11), 22q11.2 (Distal microdeletion 22q11.2), Xq28 (microduplication Xq28), 1 p32.1-p31.1 (microdeletion and duplication 1p32-p31), 7q32.2-q34 (microdeletion 7q33) and 6q22.33-q23.3 (microdeletions 6q22.33).

Many cancer diseases are associated with chromosomal abnormalities. Cancer in general might therefore be diagnosed if a patient sample shows an unusual or increased CIN compared to a reference. Cancers in context of the invention that analysed, predicted, diagnosed or monitored are selected from the following non-limiting list of cancers:

Acoustic neuroma; adenocarcinoma; adrenal gland cancer; anal cancer; angiosarcoma (e.g., lymphangiosarcoma, lymphangioendotheliosarcoma, hemangiosarcoma); appendix cancer; benign monoclonal gammopathy; biliary cancer (e.g., cholangiocarcinoma); bladder cancer; breast cancer (e.g., adenocarcinoma of the breast, papillary carcinoma of the breast, mammary cancer, medullary carcinoma of the breast); brain cancer (e.g., meningioma, glioblastomas, glioma (e.g., astrocytoma, oligodendroglioma), medulloblastoma); bronchus cancer; carcinoid tumor; cervical cancer (e.g., cervical adenocarcinoma); choriocarcinoma; chordoma; craniopharyngioma; colorectal cancer (e.g., colon cancer, rectal cancer, colorectal adenocarcinoma); connective tissue cancer; epithelial carcinoma; ependymoma; endotheliosarcoma (e.g., Kaposi's sarcoma, multiple idiopathic hemorrhagic sarcoma); endometrial cancer (e.g., uterine cancer, uterine sarcoma); esophageal cancer (e.g., adenocarcinoma of the esophagus, Barrett's adenocarinoma); Ewing's sarcoma; eye cancer (e.g., intraocular melanoma, retinoblastoma); familiar hypereosinophilia; gall bladder cancer; gastric cancer (e.g., stomach adenocarcinoma); gastrointestinal stromal tumor (GIST); germ cell cancer; head and neck cancer (e.g., head and neck squamous cell carcinoma, oral cancer (e.g., oral squamous cell carcinoma), throat cancer (e.g., laryngeal cancer, pharyngeal cancer, nasopharyngeal cancer, oropharyngeal cancer)); hematopoietic cancers (e.g., leukemia such as acute lymphocytic leukemia (ALL) (e.g., B-cell ALL, T-cell ALL), acute myelocytic leukemia (AML) (e.g., B-cell AML, T-cell AML), chronic myelocytic leukemia (CML) (e.g., B-cell CML, T-cell CML), and chronic lymphocytic leukemia (CLL) (e.g., B-cell CLL, T-cell CLL)); lymphoma such as Hodgkin lymphoma (HL) (e.g., B-cell HL, T-cell HL) and non-Hodgkin lymphoma (NHL) (e.g., B-cell NHL such as diffuse large cell lymphoma (DLCL) (e.g., diffuse large B-cell lymphoma), follicular lymphoma, chronic lymphocytic leukemia/small lymphocytic lymphoma (CLL/SLL), mantle cell lymphoma (MCL), marginal zone B-cell lymphomas (e.g., mucosa-associated lymphoid tissue (MALT) lymphomas, nodal marginal zone B-cell lymphoma, splenic marginal zone B- cell lymphoma), primary mediastinal B-cell lymphoma, Burkitt lymphoma, lymphoplasmacytic lymphoma (i.e., Waldenstrom's macroglobulinemia), hairy cell leukemia (HCL), immunoblastic large cell lymphoma, precursor B -lymphoblastic lymphoma and primary central nervous system (CNS) lymphoma; and T-cell NHL such as precursor T- lymphoblastic lymphoma/leukemia, peripheral T-cell lymphoma (PTCL) (e.g., cutaneous T- cell lymphoma (CTCL) (e.g., mycosis fungiodes, Sezary syndrome), angioimmunoblastic T- cell lymphoma, extranodal natural killer T-cell lymphoma, enteropathy type T-cell lymphoma, subcutaneous panniculitis-like T-cell lymphoma, and anaplastic large cell lymphoma); a mixture of one or more leukemia/lymphoma as described above; and multiple myeloma (MM)), heavy chain disease (e.g., alpha chain disease, gamma chain disease, mu chain disease); hemangioblastoma; hypopharynx cancer; inflammatory myofibroblastic tumors; immunocytic amyloidosis; kidney cancer (e.g., nephroblastoma a.k.a. Wilms' tumor, renal cell carcinoma); liver cancer (e.g., hepatocellular cancer (HCC), malignant hepatoma); lung cancer (e.g., bronchogenic carcinoma, small cell lung cancer (SCLC), non-small cell lung cancer (NSCLC), adenocarcinoma of the lung); leiomyosarcoma (LMS); mastocytosis (e.g., systemic mastocytosis); muscle cancer; myelodysplastic syndrome (MDS); mesothelioma; myeloproliferative disorder (MPD) (e.g., polycythemia vera (PV), essential thrombocytosis (ET), agnogenic myeloid metaplasia (AMM) a.k.a. myelofibrosis (MF), chronic idiopathic myelofibrosis, chronic myelocytic leukemia (CML), chronic neutrophilic leukemia (CNL), hypereosinophilic syndrome (HES)); neuroblastoma; neurofibroma (e.g., neurofibromatosis (NF) type 1 or type 2, schwannomatosis); neuroendocrine cancer (e.g., gastroenteropancreatic neuroendoctrine tumor (GEP-NET), carcinoid tumor); osteosarcoma (e.g., bone cancer); ovarian cancer (e.g., cystadenocarcinoma, ovarian embryonal carcinoma, ovarian adenocarcinoma); papillary adenocarcinoma; pancreatic cancer (e.g., pancreatic andenocarcinoma, intraductal papillary mucinous neoplasm (IPMN), Islet cell tumors); penile cancer (e.g., Paget's disease of the penis and scrotum); pinealoma; primitive neuroectodermal tumor (PNT); plasma cell neoplasia; paraneoplastic syndromes; intraepithelial neoplasms; prostate cancer (e.g., prostate adenocarcinoma); rectal cancer; rhabdomyosarcoma; salivary gland cancer; skin cancer (e.g., squamous cell carcinoma (SCC), keratoacanthoma (KA), melanoma, basal cell carcinoma (BCC)); small bowel cancer (e.g., appendix cancer); soft tissue sarcoma (e.g., malignant fibrous histiocytoma (MFH), liposarcoma, malignant peripheral nerve sheath tumor (MPNST), chondrosarcoma, fibrosarcoma, myxosarcoma); sebaceous gland carcinoma; small intestine cancer; sweat gland carcinoma; synovioma; testicular cancer (e.g., seminoma, testicular embryonal carcinoma); thyroid cancer (e.g., papillary carcinoma of the thyroid, papillary thyroid carcinoma (PTC), medullary thyroid cancer); urethral cancer; vaginal cancer; and vulvar cancer (e.g., Paget's disease of the vulva).

The method of the invention for diagnosing a disorder is in preferred embodiments a purely *in vitro* or even in silico performed method.

In other embodiments, the diagnostics of the invention may include any one of or all of the following steps: obtaining a sample of a subject to be diagnosed. Such samples may be any biological sample comprising genomic material, preferably cellular samples of the subject. Such samples may be obtained from any source to analyse the general genomic status of the subject, or may be specifically obtained from a tissue or cell type suspected to be involved in a pathology. Hence, such biological samples, in addition to the general definition of samples provided herein, may include any biological tissue, organ, organ system or fluid. Such samples include, but are not limited to, sputum, blood, blood cells (e.g., white cells), amniotic fluid, plasma, semen, bone marrow, and tissue or core, fine or punch needle biopsy samples, urine, peritoneal fluid, and pleural fluid, or cells therefrom. Biological samples may also include sections of tissues such as frozen sections taken for histological purposes. A biological sample may also be referred to as a "patient sample."

A further step included in the diagnostics may be the isolation of the DNA to be analysed with the method of the invention. Such methods of obtaining DNA, purifying and preparing it for sequencing approaches are well known to the skilled artisan. Then further, the diagnostic method of the invention may include strand-specific sequencing to obtain the strand-specific sequence data.

## Cell Quality Control

In another aspect the invention provides a method for assessing the chromosomal stability of a single cell, or within a population of single cells, the method comprising performing a method according to any one of the preceding claims, and wherein an increased total number, or increased number of any one type or multiple types of, SV in the said single cell or population of single cells, indicates chromosomal instability.

As already mentioned herein CIN is a general indicator of many diseases and in particular cancer. Hence, testing CIN with the scTRIP of the invention provides an application to easily access whether a cell population is of low quality as the cell shows an increased CIN. The method is for use in quality control of a genetically engineered cell or population of cells, wherein an increased instability indicates a loss of quality.

In the era of gene editing and autologous T cell therapies, which involve the genetic engineering of autologous or heterologous, or foreign cells for therapeutic purposes, the need for quality controlling engineered cell before they are administered to a human patient increases. Genetic engineering of cells always bears a risk of introducing into the genomes of the engineered cells alterations which might affect genetic stability. In the worst case scenario, increased CIN could upon administration into a patient lead to the development of a cancerous disorder, which must be avoided at all costs. Since the present invention provides a quick and cheap method to assess SVs over a population of cells, it may be used as a quality control procedure of such engineered cells in advance of reinfusion. In one embodiment, the method entails detecting of SVs in a sample of the engineered cells, or cell line, and comparing it to a reference cell or reference state. An observed increase in CIN then would result in a decreased quality of the engineered cells. Also occurrence of certain types of problematic SVs might result in discarding the engineered cells.

Preferably in this aspect the single cell or population of single cells analysed are genetically engineered cells such as by gene editing, viral integration. Preferred engineered cells are immune cells, such as Chimeric Antigen Receptor (CAR) - T cells, T cell receptor (TCR) engineered cells, or antibody engineered cells. However, any cell or cell line might be subject to quality control testing with the methods of the invention. Such applications include stem cell research, such as controlling induced pluripotent stem cells (iPSCs). Hence such stem cells, preferably iPSC, are preferred single cells or population of cells, analysed in accordance with the various aspects and embodiments of the invention.

In some embodiments the single cell or population of single cells, are for use in a cellular therapy of a patient, such as autologous immune cell therapy.

In another aspect, the invention also pertains to a method of screening a candidate compound for its effects on chromosomal stability. The method preferably involves contacting at least one single cell, or a population of cells, with the candidate compound, and thereafter, performing any method of scTRIP described herein before in order to obtain SVs in the treated cells. Another step in the method may include a comparison of the detected SVs in the treated cells with a reference, or with the cells before treatment, or with in parallel non-treated cells.

The method for screening may be applied for example to test side effect of therapeutic compounds on genomic stability. Such compounds can be any compound that might be suspected to have an impact on genomic stability and preferably is selected from polypeptide, peptide, glycoprotein, a peptidomimetic, an antibody or antibody-like molecule; a nucleic acid such as a DNA or RNA, for example an antisense DNA or RNA, a ribozyme, an RNA or DNA aptamer, siRNA, shRNA and the like, including variants or derivatives thereof such as a peptide nucleic acid (PNA); a targeted gene editing construct, such as a CRISPR/Cas9 construct, a carbohydrate such as a polysaccharide or oligosaccharide and the like, including variants or derivatives thereof; a lipid such as a fatty acid and the like, including variants or derivatives thereof; or a small organic molecules including but not limited to small molecule ligands, small cell-permeable molecules, and peptidomimetic compounds. Hence, the term candidate compound shall also comprise any method of treating or altering a cell in order to test such methods ability on genomic stability. Preferred, however, is the testing of anti-cancer agents, such as chemo-therapeutics.

Further the invention in some embodiments and aspects pertains to the following particularly preferred itemized embodiments:

Item 1: A method for analyzing sequencing data of at least one target chromosomal region by single cell tri-channel processing (scTRIP), comprising providing strand specific sequence data of at least one target chromosomal region of at least one single cell, wherein the strand-specific sequencing data comprise a multitude of strand specific sequence reads obtained by sequencing of the target chromosomal region of at least one single cell, aligning the sequence reads, or if the sequence reads are equally fragmented, each fragmented portion of such sequence read, to a reference assembly, and then assign in any given selected window the at least two of three layers of sequence information: (i) number of total sequence reads, or portions thereof (also known as "read depth"); (ii) number of forward (or Watson) sequence reads, or portions thereof, and number of reverse (or Crick) sequence reads, or portions thereof; (iii) number of sequence reads, or portion thereof, assigned with a specific haplotype identity (such as H1 and/or H2).

Item 2: The method according to item 1, comprising the specific steps of:

(a) providing strand-specific sequence data of the at least one target chromosomal region of at least one single cell, wherein the strand-specific sequencing data comprise a multitude of strand specific sequence reads obtained by sequencing of the target chromosomal region of at least one single cell;

(b) aligning each sequence read, or portion thereof, to a reference sequence of the at least one target chromosomal region to bring the sequence reads, or portions thereof, into a positional order along the reference sequence of the at least one target chromosomal region;

(c) assigning to each aligned sequence read, or portion thereof, from (b) a chromosomal haplotype identity (H1/H2) along the at least one target chromosomal region; and

(d) assigning to at least one predetermined sequence window of the positional ordered and aligned sequence reads, or portions thereof, any two of the following channels of sequence information:

(i) number of total sequence reads, or portions thereof, aligned in the at least one predetermined sequence window;

(ii) number of forward sequence reads, or portions thereof, and number of reverse sequence reads, or portions thereof, aligned in the at least one predetermined sequence window;

(iii) number of sequence reads, or portions thereof, assigned to a first (H1) haplotype identity; and/or number of sequence reads, or portions thereof, assigned to a second (H2) haplotype identity, aligned in the at least one predetermined sequence window.

**Item 3:** The method according to item 1 or 2, wherein all three channels of sequence information (i) to (iii) are assigned.

**Item 4:** The method according to any one of items 1 to 3, comprising a step of segmenting the at least one target chromosomal region, wherein the segmenting is performed on basis of the channels of sequence information (i) to (iii), each individually or together.

**Item 5:** The method according to any one of items 1 to 3, wherein the provided sequence reads, such as in item 2 in step (a), are provided independent of read length of the sequence reads.

**Item 6:** The method according to any one of items 1 to 5, wherein the strand-specific sequence data comprises sequence reads which map to one of at least two separate strands of the at least one target chromosomal region, preferably comprising further sequence reads which map to the other of the at least two separate strands, for example wherein one strand is from the paternal and the other strand is from the maternal chromosome (but could further comprise sequence reads mapping to a single strand in the case of monosomy, or additional strands in the case of triploidy, etc.).

**Item 7:** The method according to any one of items 1 to 6, wherein the strand-specific sequence data is obtained by strand-seq (Falconer et al. 2012 Nature Methods. 9 (11): 1107-1112.).

**Item 8:** The method according to any one of the preceding items, wherein the sequencing data comprises a multitude of non-overlapping and/or overlapping sequence reads.

**Item 9:** The method according to any one of the preceding items, wherein, such as in item 2 in step (b), each sequence read, or portion thereof, is aligned with the direction forward or reverse to maintain strand-specific sequence information.

**Item 10:** The method according to any one of the preceding items, further comprising the step:
(e) Identifying a structural variation (SV) by performing step (d) for a multiplicity (at least two) of windows within the sequence data of the positional ordered and aligned sequence reads, and identifying within the multiplicity of windows a sub-region comprising one or more windows having an unusual/altered/changed distribution of the information of any one, or all of, or any combination of, channels (i) to (iii).

**Item 11:** The method according to item 10, wherein said sub-region is defined by at least one, preferably two, breakpoints, and wherein such breakpoints indicate a change of any one, or any combination, or all, of the information of channels (i) to (iii) compared to the reference state and/or compared to an overall distribution of said channel information within in the sequence data.

**Item 12:** The method according to item 9 or 10, wherein said reference state of said chromosomal region is a state of the information of the channels which is expected for a non-aberrant distribution and/or predetermined state of the information of said chromosomal region.

**Item 13:** The method according to item 12, wherein said reference state in a target diploid chromosomal region is in the event the diploid target chromosomal region comprises a first template strand derived from the first parental target chromosomal region and a second template strand derived from the second parental target chromosomal region; said reference state is:

If the first parental target chromosomal region is Watson (W), and the second parental target chromosomal region is Crick (C) - the WC reference state:

Channel (i): number of total reads correspond to the presence of about 2x the target chromosomal region (2N);

Channel (ii): number of reads for each W and C strand correspond to the presence of about 1x the target chromosomal region (1N);

Channel (iii): number of W-reads which are H1 identity correspond to 1x, and number of C-reads which are H2 identity correspond to 1x; or

If the first parental target chromosomal region is C, and the second parental target chromosomal region is W - the CW reference state:

Channel (i): number of total reads correspond to the presence of about 2x the target chromosomal region (2N);

Channel (ii): number of reads for each W and C strand correspond to the presence of about 1x the target chromosomal region (1N);

Channel (iii): number of W-reads which are H2 identity correspond to 1x, and number of C-reads which are H1 identity correspond to 1x; or

If the first and the second parental target chromosomal region is W - the WW reference state:

Channel (i): number of total reads correspond to the presence of about 2x the target chromosomal region (2N);

Channel (ii): number of reads for the W strand correspond to the presence of about 2x the target chromosomal region (2N), and wherein only residual (oN) reads are present;

Channel (iii): number of W-reads which are H1 identity correspond to 1x, and number of W-reads which are H2 identity correspond to 1x, and wherein only residual reads are present corresponding to oN; or

If the first and the second parental target chromosomal region is C - the CC reference state:

Channel (i): number of total reads correspond to the presence of about 2x the target chromosomal region (2N);

Channel (ii): number of reads for the C strand correspond to the presence of about 2x the target chromosomal region (2N), and wherein only residual W reads are present corresponding to oN;

Channel (iii): number of C-reads which are H1 identity correspond to 1x, and number of C-reads which are H2 identity correspond to 1x, and wherein only residual W reads are present corresponding to oN;

wherein the SV is detected if there is a variation from the reference state, and optionally, wherein the SV is classified in accordance to a variation indicated in table 1.

**Item 14:** The method according to any one of items 10 to 12, wherein the SV is a translocation, and wherein the sequence data comprise a multiplicity of target chromosomal regions of different chromosomes, and wherein the translocation is identified by a difference in overall distribution of any one, all of, or any combination of, the information of channels (i) to (iii), between a candidate chromosomal region of one chromosome compared to one or more other chromosomal regions of other chromosomes.

**Item 15:** The method according to any one of items 10 to 12, wherein the SV is an altered ploidy state, and wherein the sequence data comprise a multiplicity of target chromosomal regions of different chromosomes, and wherein the altered ploidy state is identified by a difference in overall distribution of any one, all of, or any combination of, the information of channels (i) to (iii), between a candidate polyploidy chromosomal region of one chromosome compared to one or more other chromosomal regions of other chromosomes.

**Item 16:** The method according to any one of the preceding items, wherein a sequence read has a length of 20 to 500 nucleotides, and wherein portion of a sequence read is used in the event the sequence reads exceed a length threshold (of 500, preferably 1000, or more nucleotides), and such long sequence reads are in silico fragmented into smaller portions of the sequence reads having a length of preferably 20 to 500 (~150) nucleotides, preferably wherein said sequence reads, or portions thereof, within the dataset have an overall comparable sequence length.

**Item 17:** The method according to any one of the preceding items wherein the sequence reads have an overall coverage of 0.001x to 100x, preferably about 0.01x to 0.05x, of the target chromosomal region.

**Item 18:** The method according to any one of the preceding items, wherein in step (c) a chromosomal haplotype identity (H1/H2) along the at least one target chromosomal region is assigned, preferably while retaining strand orientation information (i.e. in a strand-aware manner), and preferably such haplotype is assigned by assigning Single Nucleotide Polymorphisms (SNP) to the sequence reads, or portions thereof, preferably wherein such SNP does not have a disease association.

**Item 19:** The method according to any one of the preceding items, wherein the haplotype identity is assigned to a sequence read, or a portion thereof, comprising a SNP, and identifying the allele of the SNP by comparison to a SNP database, or alternatively by comparing the allele to a multiplicity of further sequenced single cells of the same origin (for example using StrandPhaseR - Porubsky et al. 2017); and optionally, wherein haplotype identity is assigned to a sequence read, or a portion thereof, not comprising a SNP, by inferring said haplotype identity in by strand identity and comparison to other sequence reads, or portions thereof, having the same strand identity and which comprise such SNP.

**Item 20:** The method according to any one of the preceding items, wherein the method is performed with strand-specific sequence data of the at least one target chromosomal region of at least two or more single cells, preferably 10 or more, more preferably 50 or more, most preferably 90 or more or 350 or more; and preferably, wherein the multiplicity of single cells is derived from the same or identical origin, such as the same individual and/or the same tissue or sample type.

**Item 21:** The method according to any one of the preceding items, wherein the target chromosomal region is one or more chromosomes, preferably one or more chromosomes of a diploid organism.

**Item 22:** The method according to any one of the preceding items, wherein the strand-specific sequence data of the at least one target chromosomal region of at least one single cell, comprises data covering the complete genome of said single cell.

**Item 23:** The method according to any one of the preceding items, wherein the cell is a prokaryotic cell, a eukaryotic cell, such as an animal or plant cell, preferably wherein the animal cell is a mammalian cell such as a mouse, rat or human cell.

**Item 24:** The method according to any one of the preceding items, wherein the strand-specific sequence data of the at least one target chromosomal region of at least one single cell is obtained from a cellular sample of a patient, and wherein said single cell is either a cell associated with a disease, or is a healthy cell of said patient, preferably wherein the method is performed for a multiplicity of single cells associated with the disease and/or healthy cells.

**Item 25:** The method according to any one of the preceding items, for detecting polyploidy states and/or balanced or unbalanced translocations within a preferably diploid genome of a cell, wherein the method includes strand-specific sequence data covering the affected chromosomal regions (such as chromosomes) of the single cell, and wherein the method of any one of the preceding items is performed with a multiplicity of single cells of the same origin and/or expected to share said polyploidy and/or translocation; and wherein a polyploidy or translocation is detected if the distribution of the strand-orientation within the population of single cells is altered from the expected pattern.

**Item 26:** The method according to item 26, wherein a polyploidy is detected if the distribution of sequenced forward or reverse strands for each chromosome differs from the overall distribution expected for diploid chromosomal (autosomal) segregation, such as 50% WC, 25% WW and 25% CC.

**Item 27:** The method according to item 26, wherein a translocation is detected if the distribution of forward or reverse reads for any given sub-region within any given target chromosomal region (such as a chromosome) independently segregate with another sub-region of the given chromosome as evidenced by their distribution within the multiplicity of single cells.

**Item 28:** The method according to any one of the preceding items, wherein the method comprises a further step (f) diagnosing a condition based on the identity of, location of, or number of detected SV within the target chromosomal region.

**Item 29:** The method according to item 28, wherein the detected SVs of said target chromosomal region is compared with a known reference state of said chromosomal region, such as a known state of the chromosomal region of a healthy cell.

**Item 30:** The method according to any one of the preceding items, wherein the method further includes detecting SV-affected genes or genetic elements within the target chromosomal region.

**Item 31:** The method according to any one of the preceding items, which is an in-vitro method, or is an in-silico method.

**Item 32:** The method according to any one of the preceding items, wherein the method further comprises a step of calculating a probability of occurrence of a SV at a given position, for example by using a Bayesian network of all channels (i) to (iii).

**Item 33:** A method of detecting a structural variation (SV) in a target chromosomal region, the method comprising, preforming a method according to item 9, and items 10 to 32 when referring to item 9.

**Item 34:** A method of karyotyping a single cell, or a population of multiple single cells, the method comprising,

(a) Providing strand specific sequence data of the at least one target chromosomal region, preferably the complete genome, of at least one single cell, or each of the population of single cells,

(b) Performing a method of items 1 to 31,

(c) Detecting SV within the target chromosomal region of said single cell, or the population of single cells, and

(d) Obtaining an in-silico karyotype based on all detected SVs.

**Item 35:** A method of diagnosing a disease in subject, the method comprising, providing strand specific sequence data of one or more cells of the subject, performing a method according to item 33, detecting within the one or more cells any SV, and comparing the detected SV with a reference state, wherein an altered number, type or location of one or more SV in the sample of the subject indicated the presence of a condition, such as a disease, for example cancer.

**Item 36:** A method for assessing the chromosomal instability (CIN) of a single cell, or within a population of single cells, the method comprising performing a method according to any one of the preceding items, and wherein an increased total number, or increased number of any one type or multiple types, of SV in the said single cell or population of single cells, indicates chromosomal instability.

**Item 37**: The method according to item 36, for use in quality control of a cell or population of cells, wherein an increased instability indicates a loss of quality, preferably subsequent to an (genetic) alteration of said cell or population of cells.

**Item 38**: The method according to item 36 or 37, wherein the single cell or population of single cells is genetically engineered, preferably such as by reprogramming, gene editing or viral integration.

**Item 39**: The method according to any one of items 36 to 38, wherein the single cell or population of single cells, are for use in a cellular therapy of a patient, such as autologous immune cell therapy.

**Item 40**: A computer readable medium comprising computer readable instructions stored thereon that when run on a computer perform a method according to any one of items 1 to 33.

**Item 41**: A method of karyotyping a genome of at least one single cell of interest, comprising: a) obtaining a plurality of (preferably non-overlapping) strand specific sequences from random locations of the genome of the at least one single cell; b) mapping said test strand specific sequences to a genomic reference scaffold to obtain a test distribution of mapped strand specific sequences; c) assigning to a predetermined sequence window within the reference scaffold (i) number of mapped sequence reads, (ii) number of mapped forward strand reads and number of reverse strand reads, preferably a ratio thereof, and (iii) a haplotype identity (H1/H2), to obtained a three layered test distribution of mapped sequences; d) identifying a statistically significant alteration between an expected distribution, wherein such an alteration indicates a karyotypic abnormality in the genome of the at least one single cell; or e) comparing the three layered test distribution to a reference distribution obtained from a reference cell (such as a healthy cell), wherein if a significant difference is present said difference indicates a karyotypic difference between the at least one single cell and the reference cell

In a final aspect, the invention pertains also to a computer readable medium comprising computer readable instructions stored thereon that when run on a computer perform a method according to the herein disclosed invention, preferably scTRIP.

The above-described embodiments can be implemented in any of numerous ways.

For example, the embodiments may be implemented using hardware, software or a combination thereof. When implemented in software, the software code can be executed on any suitable processor or collection of processors, whether provided in a single computer or distributed among multiple computers. It should be appreciated that any component or collection of components that perform the functions described above can be generically considered as one or more controllers that control the above-discussed functions. The one or more controllers can be implemented in numerous ways, such as with dedicated hardware, or with general purpose hardware (e.g., one or more processors) that is programmed using microcode or software to perform the functions recited above.

In this respect, it should be appreciated that one implementation comprises at least one computer-readable storage medium (i.e., at least one tangible, non-transitory computer-readable medium), such as a computer memory (e.g., hard drive, flash memory, processor working memory, etc.), a floppy disk, an optical disk, a magnetic tape, or other tangible, non-transitory computer-readable medium, encoded with a computer program (i.e., a plurality of instructions), which, when executed on one or more processors, performs above-discussed functions. The computer-readable storage medium can be transportable such that the program stored thereon can be loaded onto any computer resource to implement techniques discussed herein. In addition, it should be appreciated that the reference to a computer program which, when executed, performs above-discussed functions, is not limited to an application program running on a host computer. Rather, the term "computer program" is used herein in a generic sense to reference any type of computer code (e.g., software or microcode) that can be employed to program one or more processors to implement above-techniques.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by $\pm 20\%$, $\pm 15\%$, $\pm 10\%$, and for example $\pm 5\%$. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

BRIEF DESCRIPTION OF THE FIGURES

The figures show:

**Figure 1** shows (**a**) Overview of the Strand-seq sequencing protocol. Strand-seq involves incorporating BrdU into dividing cells, followed by removal of the BrdU containing strands through nicking, and short read sequencing of the remaining strand[21]. Strand-seq libraries preserve strand-orientation and chromosomal homolog (haplotype) identity. Dashed line: strand (BrdU) label. W, Watson strand (orange); C, Crick (green); H, haplotype. (**b**) Scheme depicting how template strand co-segregation patterns during mitosis reveal SVs in single cells. *Del*, deletion; *Inv*, inversion; *Tr*, translocation. Segments of derivative chromosomes share the same template strand during DNA replication. H1/H2, haplotype 1 and 2 of a chromosome; h1/h2, haplotype 1 and 2 of another chromosome. (**c**) The scTRIP computational approach exploits three data layers: read depth, strand ratio, and chromosome-length haplotype phase. Red lollipops: reads assigned to H1 based on overlapping SNPs; blue lollipops: reads assigned to H2. Haplotype phase is assessed in a strand-aware fashion, with phased W reads shown as lollipops on left of ideogram and phased C reads shown to right. In contrast to prior SV detection approaches, scTRIP does not depend on discordant or split reads, the scalable detection of which has been considered infeasible in single cells. Panels **d-f** depict diagnostic footprints for chromosomes where both haplotypes are labeled on different strands ('WC/CW chromosomes'). Our framework also detects and scores equivalent footprints on CC and WW chromosomes (see **Table 1**). (**d**) Del, detected as losses in read depth affecting a single haplotype, combined with unaltered read orientation. Dup, detected as a haplotype-specific gain in depth with unaltered read orientation. (**e**) Balanced Inv, identified as haplotype-phased read orientation 'flips' with unaltered depth. InvDup, characterized by inverted reads detected for one haplotype coinciding with a read depth gain of the same haplotype. (**f**) Balanced translocation, detected as correlated template strand switches affecting the same paired genomic regions in cells harboring the SV. (**g**) Bayesian framework for SV discovery. The probability distributions depicted represent an InvDup on H1 (segments on both strands are seen for haplotype 1 (H1), whereas H2 is represented on the W strand only); (**h**) Bayesian graphical model for haplotype-aware SV classification. Model shown used to enable haplotype-aware SV discovery in single cells. This graphical model adopts the common plate notation: Circles represent random variables, squares show the model parameters, gray (white) objects show observed (latent) variables, arrows indicate dependencies, and large rectangles indicate that the enclosed variables exists multiple times. The model describes $J$ single cells, $K$ segments, and $H=2$ haplotypes. Random variables: segment length $L$, ground state $T$, haplotype SV status $V$ (to be inferred), copy numbers of W/C reads $N^{W/C}$, read counts in W/C direction $X^{W/C}$, and read counts in W/C direction tagged by haplotype $X^{W/C}_{tag}$. Note that the read counts are not observed by their haplotypes (white circles inside the H box), but they are *observed* with no haplotype information (gray circles outside the H box). The fraction of reads that overlap with a heterozygous SNP are observed by haplotype (tagged gray read count variables inside the H box). Model parameters: the fraction of background reads $\alpha$, negative Binomial parameter $\rho$ and r, and the heterozygosity rate $h$.

**Figure 2** shows scTRIP reveals deletions, duplications, inversions and chromosome aneuploidies in epithelial cells. (**a**) Binned read counts separated by DNA strand and haplotype reveal the presence of SVs in single cells (W, Watson strand (orange); C, Crick (green)). Left panel: haplotype-resolved duplication (Dup) on 3p, which is present in RPE-1 but absent in C7. Right panel: haplotype-resolved deletion (Del) on 3q present in C7 and absent in RPE-1. The box 'Depth' depicts read counts; 'Strands' depicts the W:C fraction; 'Phase' shows the location of haplotype-phased SNPs, with lollipop orientation reflecting the strand state of the read containing the SNP (W on the left, C on the right, of the ideogram). (**b**) Chromosome 17p haplotype-resolved inversion (Inv) shared across both C7 and RPE-1. (**c**) Diagnostic footprint of a monosomic chromosome. Template strand state patterns depicted are from C7, which has a karyotypically defined[30] monosomy 13. The left panel shows chromosome 13 strand-patterns from two single cells, with a visible 1:0 pattern characteristic for monosomy (1N). The right panel summarizes the fraction of observed W and C reads across 154 sequenced cells. (**d**) Diagnostic footprint of a trisomic region. Template strand state patterns depicted are from RPE-1 cells exhibiting a karyotypically defined 10q trisomic region[27]. The left panel shows chromosome 10 strand-patterns from four single cells. The right panel summarizes the fraction of observed W and C reads for the trisomic (3N) 10q region across 80 sequenced cells, revealing 2:1 and 3:0 strand ratios characteristic for trisomy (**Table 2**).

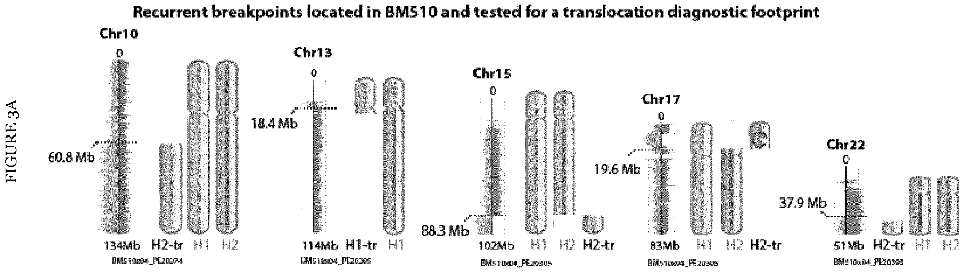

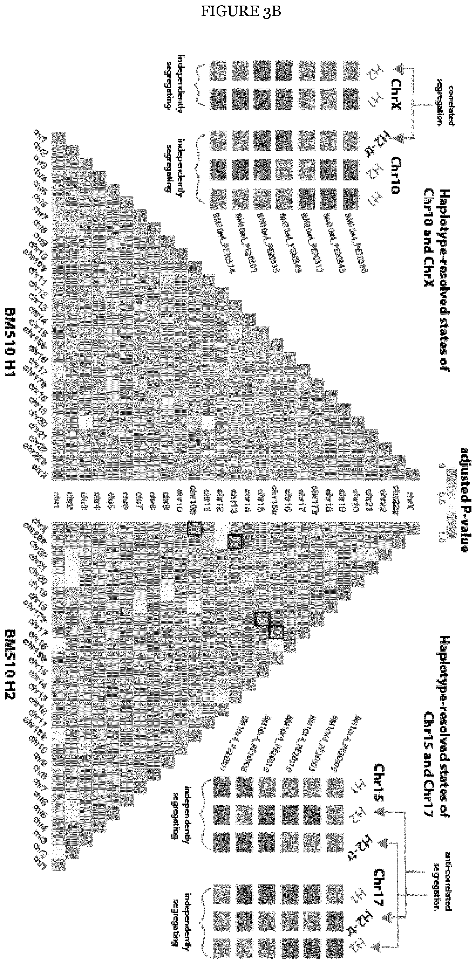

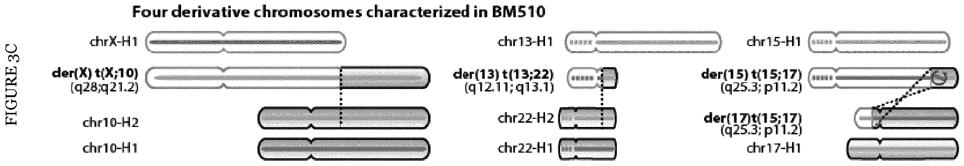

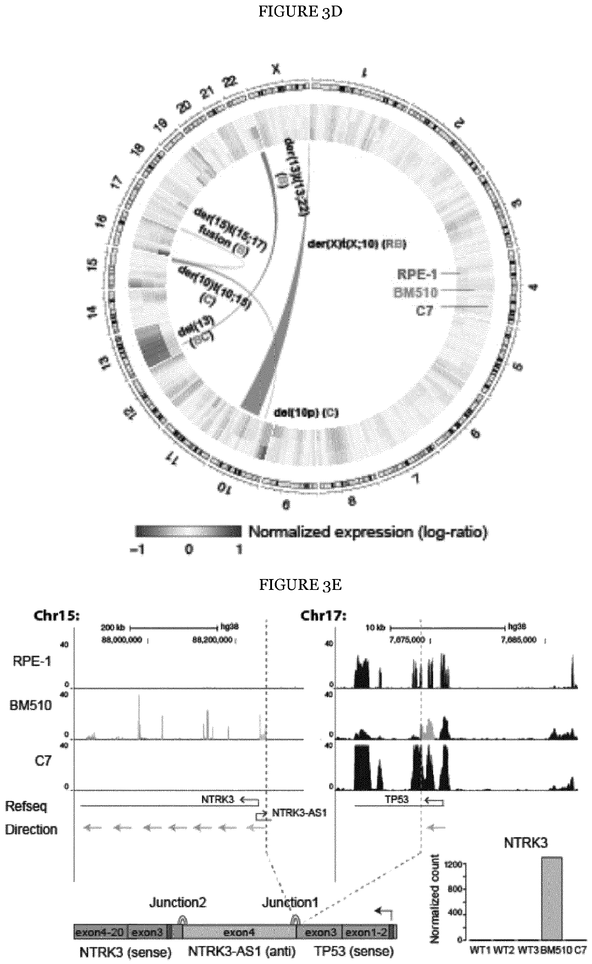

**Figure 3** shows Translocation discovery in single cells. **(a)** In BM510, segments from chromosomes 10, 13, 15, 17 and 22 failed to co-segregate with the respective chromosomes they originated from, suggesting putative involvement in translocations (use of 'tr', as in "H2-tr' or 'chr10tr', denotes the candidate translocation status of these segments). **(b)** Pyramid in the center: Unbiased analysis of translocations in BM510. Pairwise heatmap depicting segmental template-strand correlation values for each haplotype, highlighting the segment co-segregation diagnostic footprint (**Fig. 1F**) of translocations (correlation values are here expressed as Benjamini-Hochberg adjusted P-values). Orange boxes with black outline depict significant correlations ($P<0.01$; Fisher's exact test) in four cases - corresponding to four derivative chromosomes we discovered in BM510. Schemes to the left and right: Colored boxes exemplify haplotype-resolved template strand state of segments for the non-reciprocal der(X) t(X;10) translocation and the t(15;17) reciprocal translocations. (In each case only a few cells are depicted for visualisation purposes.) Box colours: W (orange); C (green). Grey arrows highlight pairwise correlations between segments, where the paired segments always exhibit the same strand state (*e.g.* chrX and chr10tr), or always exhibit inverse strand states (e.g. chr15tr and chr17; reflecting inverted orientations of these translocation partners). Inversion within the translocated portion of 17p is denoted with a circular arrow. **(c)** Center: cartoon representation of four inferred derivative chromosomes. Dashed lines correspond to unassembled regions at acrocentric chromosomes 13 and 15. **(d)** Circos plot depicting translocations and averaged gene expression values across genomic windows[77], computed from RNA-seq data generated for BM510 (here denoted 'B'), RPE-1 ('R') and C7 ('C'). **Fig. S11** resolves expression by haplotype. **(e)** Validation of gene fusion in BM510. RNA-seq based read depth for *NTRK$_3$* (green), *NTRK$_3$-AS1* (yellow) and *TP53* (blue) is depicted for C7, RPE-1 and BM510. Purple dashed lines: detected fusion junctions. Lower left corner: inferred fusion transcript. Purple boxes show start codon locations. Lower right corner: *NTRK3* dysregulation in BM510. R1-3, RNA-seq replicates of RPE-1. Ex., exon.

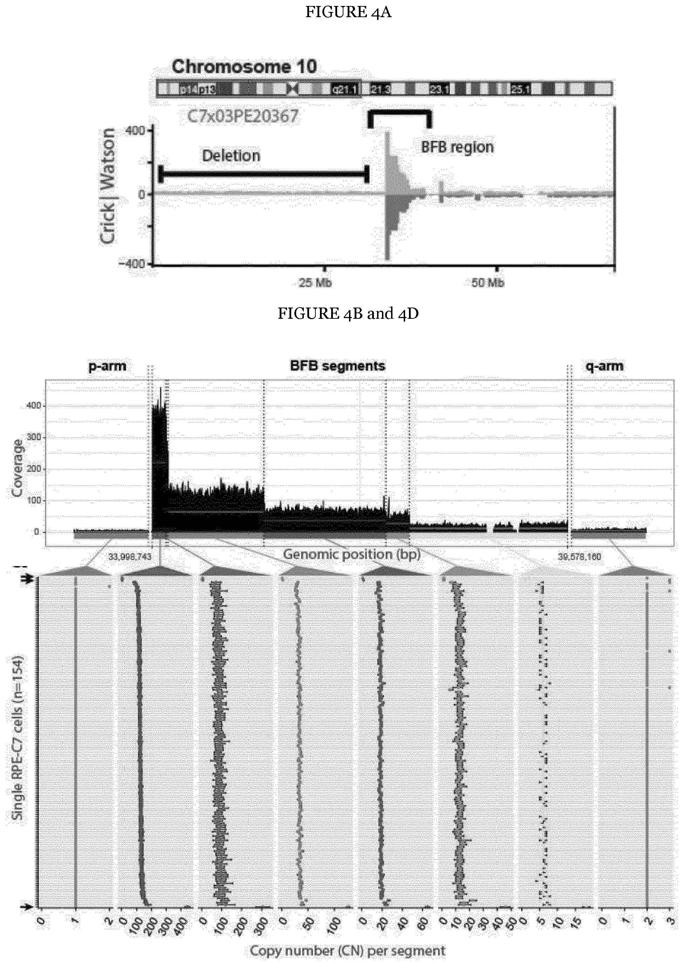

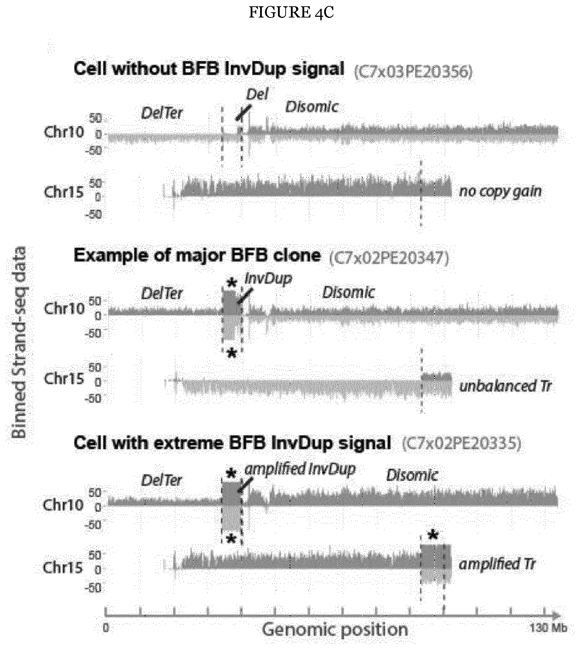

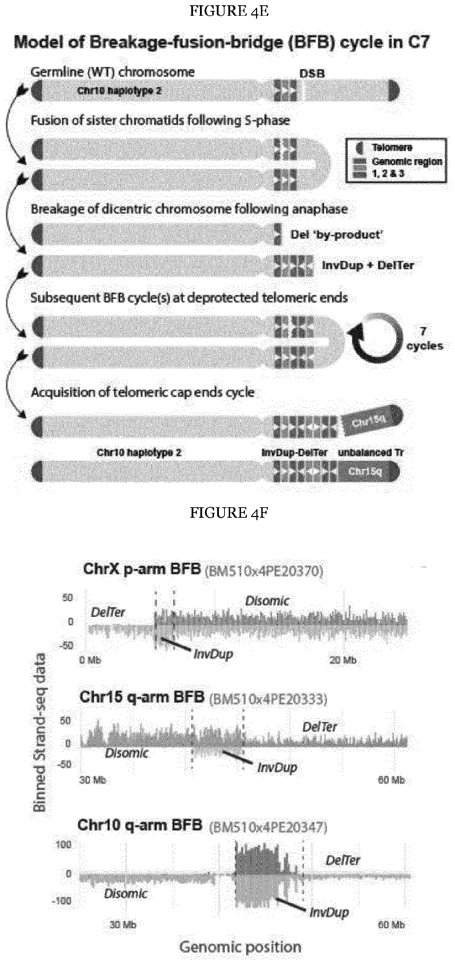

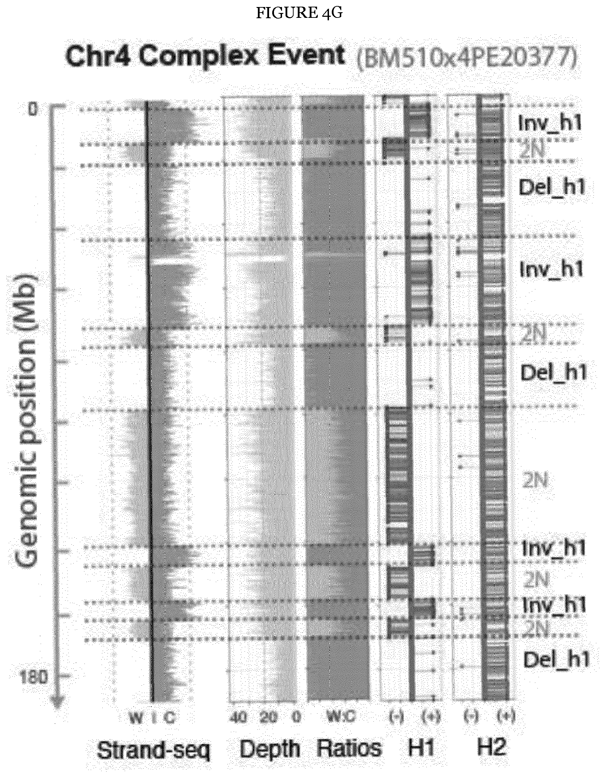

**Figure 4** shows Single cell characterization of complex rearrangement processes. **(a)** Strand-specific read depth of C7 cell with region of InvDup mediated amplification on 10p, with adjacent terminal deletion (DelTer) of the same haplotype, resulting from BFB cycles. **(b)** Aggregated read data from 154 C7 cells. Colours indicate six copy number segments (red, blue, green, purple, orange and yellow) identified within the amplicon. Grey: regions flanking the amplicon. **(c)** Depiction of three C7 cells, with estimated maximum copy-number (CN) of 1 (upper panel), CN of $\sim$110 (middle panel), and CN of $\sim$440 (lower panel) at the 10p amplicon region indicated in red. A gained segment on 15q, which scTRIP inferred to have undergone unbalanced translocation with the amplicon region, is shown beneath (this SV is absent from cells lacking the amplicon; upper panel). Read counts for W (green) and C (orange) are capped at 50 (*, saturated read counts). Tr, translocation. **(d)** Genetic diversity at 10p. CN (x-axis) is shown across 154 sequenced C7 cells (y-axis), providing cell-by-cell estimates of CN for each segment in (b). At least 3 different groups are readily discernible: high CN, intermediate CN, and loss of the 10p region (compare with panel (c)). Error bars reflect 95% confidence intervals. Arrows denote cells with CN=i and CN of $\sim$440 at the 10p amplicon. **(e)** Model of sSVs leading to the observed structures seen for the 'major clone'. Amplification via BFB cycles typically proceeds in $2^n$ copy-number steps, suggesting $\sim$7 successive BFB cycles occurred. According to our model, translocation of 15q terminal sequence stabilized 10p. DSB, double strand break. **(f)** The scar of BFBs, corresponding to InvDups flanked by DelTer on the same haplotype, identified in single BM510 cells. **(g)** Clustered rearrangements involving Dels and Invs in a single BM510 cell. Shown is the binned read data (left) seperated into the three data channels typical to scTRIP. All clustered SVs affect a single haplotype (Hi, red).

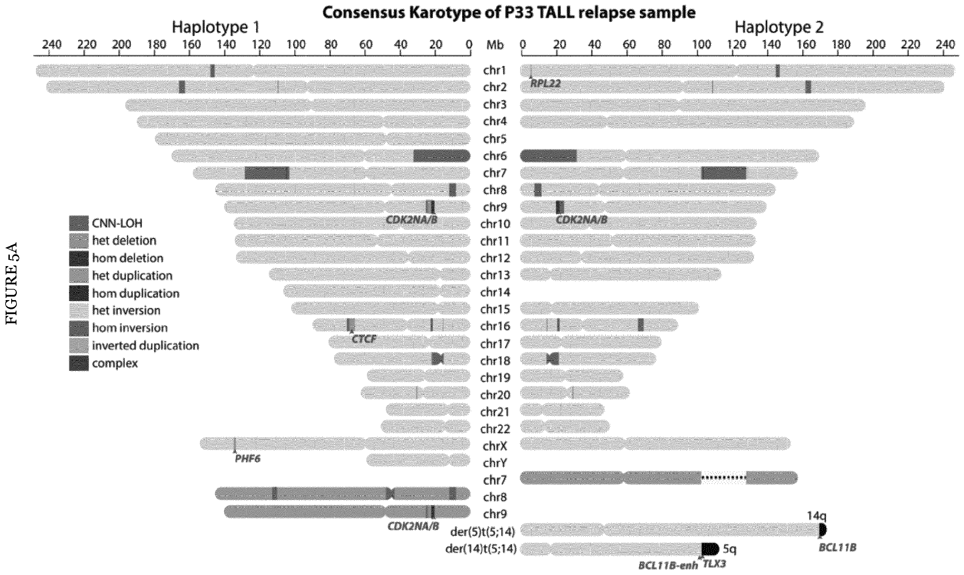

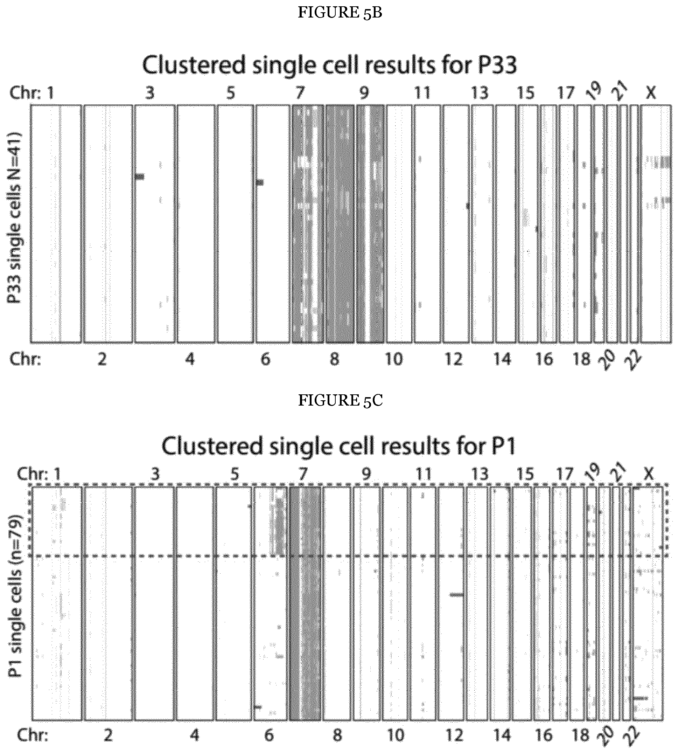

**Figure 5** shows Single cell sequencing based karyotypes of PDX-derived T-ALL relapses. **(a)** Haplotype-resolved consensus P33 karyotype constructed from 41 sequenced cells, using single cell sequencing based SV calls generated by scTRIP. Heterozygous SVs are depicted only on the haplotype they have been mapped to. Homozygous SVs (by definition) appear on both haplotypes. CNN-LOH, copy-neutral loss in heterozygosity (shown on both haplotypes)[78]. Chromosomes colored in pink reflect duplicated homologs. This T-ALL patient carries two chromosome X haplotypes (see also **Fig. S16**) as well as a Y chromosome, indicating transmission of an X and a Y chromosome from the father, whereas the mother contributed her X chromosome to the karyotype (Klinefelter or XXY syndrome). Affected leukemia-related genes are highlighted in red. '*BCL11B-enh*' denotes a previously described enhancer region in 3' of the *BCL11B* gene. **(b)** "Heatmap" of SVs arranged using Ward's method for hierarchical clustering of SVs genotype likelihoods in P33, showing the presence of a single dominant clone and evidence of few additional somatic DNA alterations resulting in karyotypic diversity in this T-ALL relapse. **(c)** "Heatmap" of SV events called in an additional T-TALL sample, P1. Red dotted box outlines a clear subclonal population in the sample, represented by 25 cells.

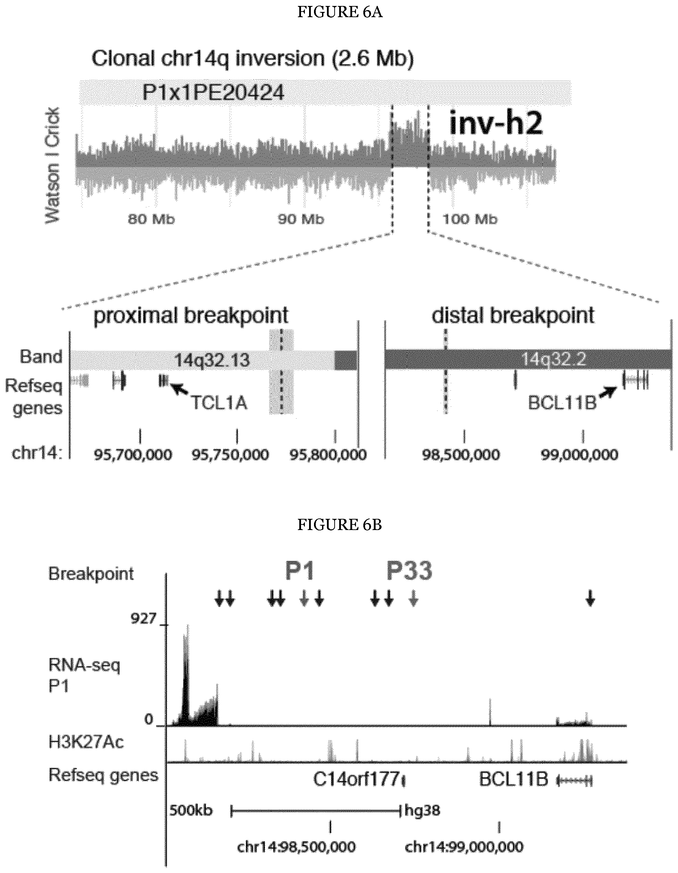

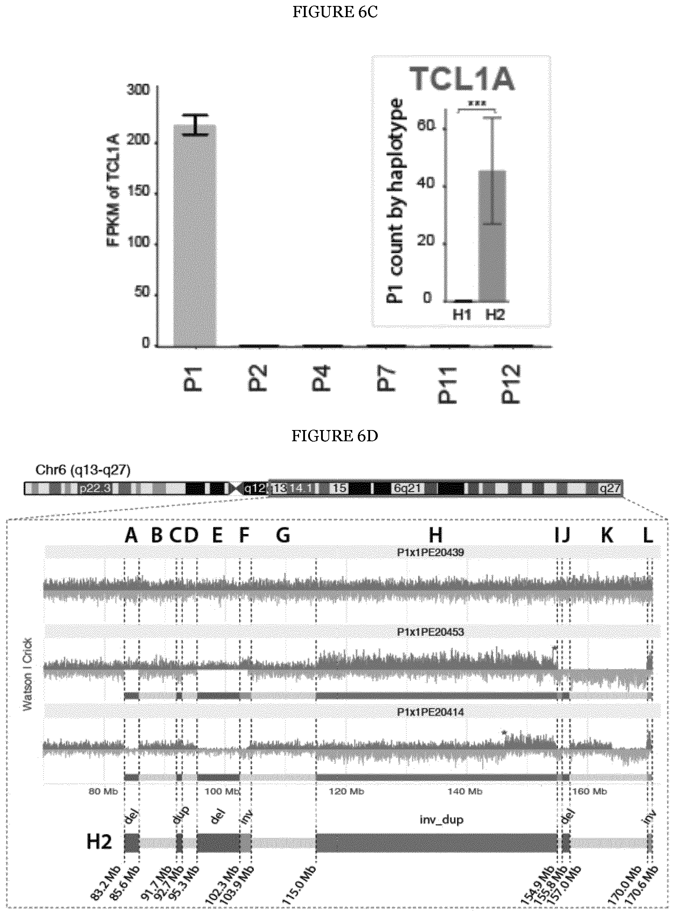

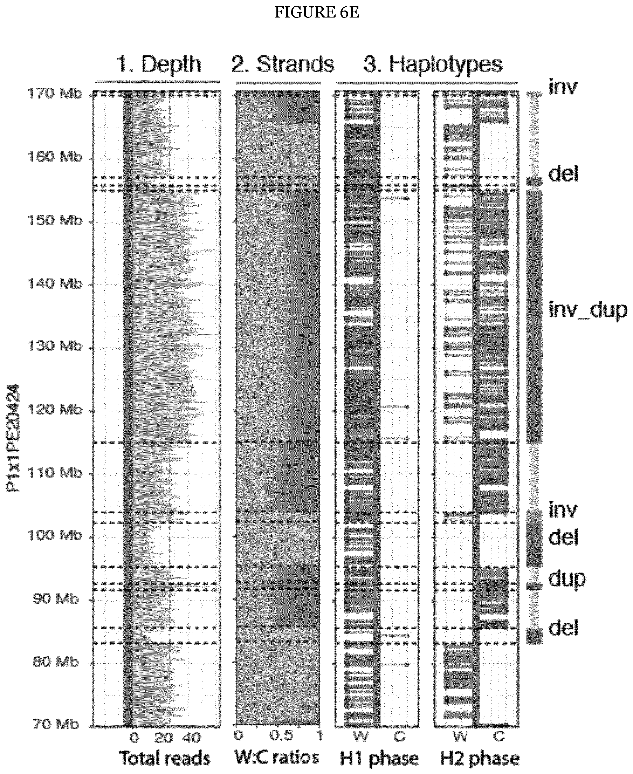

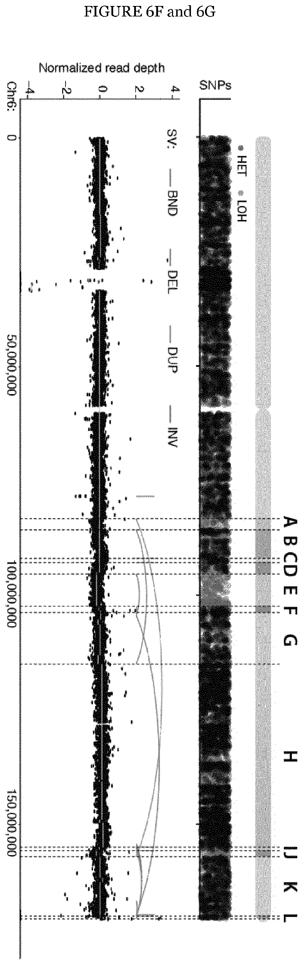

**Figure 6** shows Single cell sequencing of PDX-derived T-ALL relapse P1 reveals previously unrecognized SVs. **(a)** Haplotype-resolved balanced 14q32 Inv inferred in P1 using scTRIP. The leftmost breakpoint (thick light blue line) resides close to *TCL1A,* whereas the rightmost breakpoint (thin light blue line) is in 3' of *BCL11B*. **(b)** The rightmost Inv breakpoint falls into a "gene desert" region in 3' *BCL11B* containing several enhancers. Black arrows show breakpoints of translocations resulting in T-ALL oncogene dysregulation from a recent study[45]. Colored arrows: SV breakpoints in T-ALL donors P1 and P33. (**c**) Dysregulation of *TCL1A* in conjunction with 14q32 Inv. Larger barplot shows *TCL1A* dysregulation in P1 compared to five arbitrarily chosen T-ALLs. Inset barplot shows allele-specific RNA-seq analysis demonstrating *TCL1A* dysregulation occurs only on the inverted (H2) haplotype. (**d**) Reconstruction of subclonal clustered DNA rearrangements at 6q via scTRIP. (**e**) Haplotype-resolved analysis of SVs clustered at 6q, all of which fall onto haplotype H2. (**f**) Detection of interspersed losses and retention of LOH in conjunction with the clustered SVs, indicative for a DNA rearrangements burst[41]. (LOH, signified by an abundance of red dots, was called as reported in the **Methods.** Regions with normal density of reference heterozygous SNPs (red), but with decreased density of additionally detected heterozygous SNPs (black), are indicative for LOH.) (**g**) Verification of subclonal clustered rearrangement burst at 6q, by bulk long-insert size paired-end sequencing[75] to 165x physical coverage. Breakpoints inferred by scTRIP are shown as dotted lines, and scTRIP-inferred segments are denoted using the letters A to L. Colored breakpoint-connecting lines depict the paired-end mapping based rearrangement graph (i.e., deletion-type, tandem duplication-type, and inversion-type paired-ends). Using bulk whole-exome and mate-pair sequencing, read-depth shifts at these breakpoints were subtle and thus, this subclonal complex rearrangement escaped prior *de novo* SV detection efforts in bulk sequencing data.

EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

**Methods and Materials**

*Cell Lines and Culture.* hTERT RPE-1 cells were purchased from ATCC (CRL-4000) and checked for mycoplasma contamination. BM510 cells were generated using the CAST protocol and derived from the RPE-1 parental line (as previously-described in Mardin et al. 2015). C7 cells were acquired from Riches et al 2001. Cell lines were maintained in DMEM-F12 medium supplemented with 10% fetal bovine serum and antibiotics (Life Technologies). Ethics Statement. The protocols used in this study received approval from the relevant institutional review boards and ethics committees. The T-ALL patient samples were approved by the University of Kiel ethics board, and obtained from clinical trials ALL-BFM 2000 (P33; age: 14 years at diagnosis) or AIEOP-BFM ALL 2009 (P1; age: 12 years at diagnosis). Written informed consent had been obtained from these patients, and experiments conformed to the principles set out in the WMA Declaration of Helsinki and the Department of Health and Human Services Belmont Report. The in *vivo* animal experiments were approved by the veterinary office of the Canton of Zurich, in compliance with ethical regulations for animal research.

*Single cell DNA sequencing of RPE and T-ALL cells.* RPE cells and PDX-derived T-ALL cells were cultured using previously established protocols[28,66]. The inventors incorporated BrdU (40$\mu$M; Sigma, B5002) into growing cells for 18-48 hours, single nuclei were then sorted into 96-well plates using the BD FACSMelody cell sorter, and strand-specific DNA sequencing libraries were generated using the previously described Strand-seq protocol[21,67]. The BrdU concentration used was recently shown to have no measurable effect on sister chromatid exchanges[24], a sensitive measure of DNA integrity and genomic instability[24]. To generate libraries at scale, the Strand-seq protocol was implemented on a Biomek FX$^P$ liquid handling robotic system, which requires two days to produce 96 barcoded single cell libraries. Libraries were sequenced on a NextSeq5000 (MID-mode, 75bp paired-end protocol), demultiplexed and aligned to GRCh38 reference assembly (BWA 0.7.15). High quality libraries (obtained from cells undergoing one complete round of DNA replication with BrdU incorporation) were selected as described in[21,67]. Briefly, libraries showing very low, uneven coverage, or an excess of background reads' yielding noisy single cell data were filtered prior to analysis. In a typical experiment, ~80% of cells yield high quality libraries reflecting BrdU incorporation in exactly a single cell cycle. Cells with incomplete BrdU incorporation or cells undergoing more than one DNA synthesis phase under BrdU exposure are identified during cell sorting and thus get only rarely sequenced during Strand-seq experiments[21,67], typically contributing to less than 10% of sequenced cells. Such 'unusable libraries' hence do not palpably contribute to experimental costs.

*Chromosome-length haplotype phasing of heterozygous SNPs.* The inventor's SV discovery framework as described herein phases template strands using StrandPhaseR[22]. The underlying rationale is that for 'WC chromosomes' (chromosomes where one parental homolog is inherited as W template strand and the other homolog is inherited as C template strand), heterozygous SNPs can be immediately phased into chromosome-length haplotypes (a feature unique to strand-specific DNA sequencing). To maximize the number of informative SNPs for full haplotype construction the inventors aggregated reads from all single cell sequencing libraries and an internal 100 cell control and performed SNP discovery by re-genotyping the 1000 Genomes Project (1000GP) SNP sites[68] using Freebayes[69]. All heterozygous SNPs with QUAL>=10 where used for haplotype reconstruction and single cell haplotagging (described below).

Discovery of deletions, duplications, inversions and inverted duplications in single cells. The inventors developed the core workflow of the method of the inventors to enable single cell discovery of Dup, Del, Inv, and InvDup SVs. Input data to the workflow are a set of single-cell BAM files from a donor sample, aligned to a reference genome. The core workflow performs binned read counting, normalization of coverage, segmentation, strand state and sister chromatid exchange (SCE) detection, and haplotype-aware SV classification. A brief description of each step is provided below, and for additional details see Supplementary Information.

*Binned read counting.* Reads for each individual cell, chromosome and strand were binned into 100kb windows. PCR duplicates, improper pairs and reads with a low mapping quality (<10) were removed to count only unique, high-quality fragments.

*Normalization of coverage.* Normalization was performed to adjust for systematic read depth fluctuations. To derive suitable scaling factors, the inventors performed an analysis of Strand-seq data from 1,058 single cells generated across nine 1000GP lymphoblastoid cell lines made available through the HGSVC project (http://ftp.1000genomes.ebi.ac.uk/vol1/ftp/data_collections/hgsv_sv_discovery/working/201 51203_strand_seq/), and pursued normalization with a linear model used to infer a scaling factor for each genomic bin.

*Joint segmentation of single cells in a population.* Segmentation was performed by jointly processing strand-resolved binned read depth data across all single cells of a sample, used as multivariate input signal with a squared-error assumption[70]. Given a number of allowed change points $k$, a dynamic programming algorithm was employed to identify the discrete positions of $k$ change points with a minimal sum of squared error. Analyzing all cells jointly in this way rendered even relatively small SVs (~200kb) detectable once these are present with sufficient evidence in the single cell dataset (*e.g.* seen in enough cells). The number of breakpoints was chosen separately for each chromosome as the minimal k, such that using $k+1$ breakpoints would only yield a marginal improvement, operationalized as the difference of squared error terms being below a pre-selected threshold.

*Strand-state and SCE detection in individual cells.* The interpretation of strand-specific binned read counts relies on the knowledge of the underlying state of template strands for a given chromosome (WW, CC, or WC). These "ground states" stay constant over the length of each chromosome in each single cell, unless they are altered through SCEs[21,71]. To detect SCEs, the inventors performed the same segmentation procedure described above in each cell *separately* (as opposed to *jointly* across all cells, as for the segmentation). The inventors then inferred putative SCEs by identifying changes in strand state in individual cells that are otherwise incompatible with breakpoints uncovered by the joint segmentation (Supplementary Information). Leveraging these putative SCEs, the inventors then assigned a ground state to each segment (Supplementary Information). To facilitate haplotype-resolved SV calling, the inventors employed StrandPhaseR[72] to distinguish segments with ground state WC, where Haplotype 1 is represented by Watson (W) reads and Haplotype 2 by Crick (C) reads, from ground state CW, where it is *vice versa*.

*Haplotype-aware SV classification.* The inventors developed a Bayesian framework to compute posterior probabilities for each SV diagnostic footprint, and derive haplotype-resolved SV genotype likelihoods. To this end, the inventors modeled strand-specific read counts using a negative binomial (NB) distribution, which captures the overdispersion typical for massively-parallel sequencing data [54]. The NB distribution has two parameters, $p$ and $r$; the parameter $p$ controls the relationship of mean and variance and was estimated jointly across all cells, while $r$ is proportional to the mean and hence varies from cell to cell to reflect the different total read counts per single-cell library. After estimating $p$ and $r$, the inventors computed haplotype-aware SV genotype likelihoods for each segment in each single cell: For a given ground state (see above), each SV diagnostic footprint translates into the expected number of copies sequenced in W and C orientation contributing to the genomic segment (Table S1), which gives rise to a likelihood with respect to the NB model. The fact that the inventor's model distinguishes WC from CW ground states (see *Strand-state and SCE detection* above) renders the inventor's model implicitly whole-chromosome haplotype-aware - a key feature not met by any prior approach for somatic variant calling in single cells. In addition to this, the inventors also incorporated the count of W or C reads assignable to a single haplotype via overlapping SNPs in the likelihood calculation, and refer to this procedure as "haplotagging" (since it involves reads "tagged" by a particular haplotype). The inventors modeled the respective counts of tagged reads using a multinomial distribution. The output is a matrix of predicted SVs with probability scores for each single cell.

*SV calling in a cell population.* The inventor's workflow estimates VAF levels for each SV and uses them to define prior probabilities for each SV (Empirical Bayes). In this way, the framework benefits from observing SVs in more than one cell, which leads to an increased prior and hence to more confident SV discoveries. The inventor's framework adjusts for the tradeoff between sensitively calling subclonal SVs, and accurately identifying SVs seen consistently among cells. The inventors parameterized this tradeoff into a 'strict' and 'lenient' SV caller, whereby the 'strict' caller optimizes precision for SVs seen with VAF ≥5%, and the 'lenient' caller targets all SVs including those present in a single cell only. Unless stated otherwise, SV calls presented in this study were generated using the 'strict' parameterization, to achieve a callset that minimises false positive SVs. The inventors explored the limits of these parameterizations using simulations, by randomly implanting Dels, Dups and Invs into single cells *in silico.* The inventors analyzed 200 single cells per simulation, applying coverage levels typical for Strand-seq [21] (400,000 read fragments per cell). The inventors observed excellent recall and precisions for SVs ≥1Mb in size when present with >40% VAF (Fig. S5). And while the inventors detected a decrease in recall and precision for events present with lower VAF, the inventors were able to recover smaller SVs and those with lower VAF down to individual cells.

Single cell dissection of translocations. The inventors discovered translocations in single cells by searching for segments exhibiting strand-states that are inconsistent with the chromosomes these segments originate from, while being consistent (correlated, or anti-correlated) in strand-state with another segment of the genome (*i.e.*, their translocation partner) (Supplementary Information). To infer translocations, the inventors determined the strand states of each chromosome in a homolog-resolved manner. In cases where strand states appeared to change across a haplotype (because this haplotype exhibited SVs or SCEs), the inventors used the majority strand state (*i.e.* 'ground state', see above) to pursue translocation inference. The inventors examined template strand co-segregation by generating contingency tables tallying the number of cells with equivalent strand states versus those not having equivalent strand states (see Fig. 3B). The inventors employed Fisher's exact test to infer the probability of the count distribution in the contingency table, followed by p-value adjustment[73].

Characterization of breakage-fusion bridge (BFB) cycles in single cells. To infer and characterize BFB cycles in single cells, the inventors first employed the inventor's framework with lenient parameterization to infer InvDups flanked by a DelTer event on the same homolog/haplotype. The inventors tested whether InvDup-DelTer footprints resulting from BFB cycles may arise in single cells by chance, by searching for structures where an InvDup on one haplotype would be flanked by a DelTer on the other haplotype (for instance, an InvDup (Hi)-DelTer (H2) event, where H1 and H2 denote different haplotypes). No such structures were detected, and InvDup-DelTer footprints thus always occurred on the same haplotype, consistent with BFB cycle formation. To ensure high sensitivity of the inventor's single cell based quantifications shown in Fig. S14, the inventors additionally performed manual inspection of the single cell data for evidence of at least one of the following rearrangement classes: (*i*) an InvDup, (*ii*) a DelTer resulting in copy-number=1 on an otherwise disomic chromosome. These cells were inspected for InvDup-DelTer patterns indicative for BFBs, based on the diagnostic footprints defined in Fig. 1.

Single cell based CNN-LOH discovery. For CNN-LOH detection, the inventor's framework first assembles consensus haplotypes for each sample, by analyzing all single cell Strand-seq libraries available for a sample using StrandPhaseR[22]. Each single cell is then compared to these consensus haplotypes in a disomic context, to identify discrepancies matching the CNN-LOH footprint. To detect clonally present CNN-LOH events, the inventors used the 1000GP[68] reference SNP panel to re-genotype aggregated single cell libraries in each sample. These re-genotyped (observed) SNPs were then compared to the 1000GP reference sets to identify genomic regions showing marked depletion in heterozygous SNPs indicative for CNN-LOH. To this end, the inventors downsampled the 1000GP reference variants to the SNP numbers observed in the single cell data, and subsequently merged both data sets (observed and reference variants), sorting all SNPs by genomic position. The inventors performed a sliding window search through these sorted SNPs, moving one SNP at a time, and compared the number of observed and reference SNPs in each window by computing the ratio $R$=observed SNPs/reference SNPs. In heterozygous disomic regions, $R$ values of ~1 will be expected, whereas deviations are indicative of CNN-LOH. Window sizes (determined by the number of SNPs in a window) were defined as the median SNP count per 500kb window. The inventors employed circular binary segmentation (CBS)[74] to detect changes in $R$, and assigned each segment a state based on the mean value of $R$. Segments ≥2Mb in size exhibiting mean values $R \leq 0.15$ were reported as CNN-LOH.

Bulk genomic DNA sequencing. Genomic DNA was extracted using the DNA Blood Mini Kit (Qiagen, Hilden, Germany). 300 ng of high molecular weight genomic DNA was fragmented to 100 -700bp (300bp average size) with a Covaris S2 instrument (LGC Genomics) and cleaned up with Agencourt AMPure XP (Beckman Coulter, Brea, USA). DNA library preparation was performed using the NEBNext Ultra II DNA Library Prep Kit (New England Biolabs, Ipswich, USA). The inventors employed 15ng of adapter ligated DNA and performed amplification with 10 cycles of PCR. DNA was size selected on a 0.75% agarose gel, by picking the length range between 400 and 500 bp. Library quantification and quality control was performed using a Qubit 2.0 Fluorometer (Thermo Fisher Scientific, Waltham, USA) and a 2100 Bioanalyzer platform (Agilent Technologies, Santa Clara, USA). WGS was pursued using an Illumina HiSeq4000 (Illumina, San Diego, USA) platform, using 150 bp paired-end reads. Mate-pair sequencing with large insert size (~5kb) was pursued as described previously[75]. SV detection in bulk DNA sequence data was pursued using Delly2[31]. RPE-1 WGS data was sequenced to 32× coverage.

Bulk RNA-seq. Total RNA was extracted from RPE cells using the RNeasy MinElute Cleanup kit (Qiagen, Hilden, Germany). RNA quality control was performed using the 2100 Bioanalyzer platform (Agilent Technologies, Santa Clara, USA). Library preparation was pursued with a Beckman Biomek FX automated liquid handling system (Beckman Coulter, Brea, USA), with 200 ng starting material using TruSeq Stranded mRNA HT chemistry (Illumina, San Diego, USA). Samples were prepared with custom 6 base pair barcodes to enable pooling. Library quantification and quality control were performed using a Fragment Analyzer (Advanced Analytics Technologies, Ames, USA). RNA-Seq was pursued on an Illumina HiSeq 2500 platform (Illumina, San Diego, USA), using 50 base pair single reads. For RNA sequencing in T-ALL, total RNA was extracted using TRIzol (Invitrogen Life Technologies). The RNA was than treated with TURBO DNase (Thermo Fisher Scientific, Darmstadt, Germany) and purified using RNA Clean&Concentrator-5 (Zymo Research, Freiburg, Germany). The inventors required a minimal RIN (RNA Integrity Number) of 7 as measured using a Bioanalyzer (Agilent, Santa Clara, CA) with the Agilent RNA 6000 Nano Kit. Cytoplasmic ribosomal RNA was depleted by Ribo-Zero rRNA Removal Kit (Illumina, San Diego, CA) and the libraries were prepared from 1 μg of RNA using TruSeq RNA Library Prep (Illumina, San Diego, CA). These samples were sequenced on a Illumina HiSeq 2000 lane as 75 bp single ends. Fusion junctions were detected using the STAR aligner[76].

Quantitative real time PCR (qPCR). RNA from PDX-derived T-ALL samples was extracted using a RNeasy Mini kit according to manufacturer's instructions (cat 74106, Qiagen, Hombrechtikon, Switzerland), and cDNA was generated using High Capacity cDNA Reverse Transcription Kit (Applied BioSystems, Foster City, USA). qPCR was performed using a TaqMan Gene Expression Master Mix (Applied BioSystems) in triplicate using an ABI7900HT Analyzer with SDS2.2 software. Threshold cycle values were determined using the 2-ΔΔCT method, normalized to human-GAPDH (HS02786624_g1, Applied BioSystems).

The examples show:

**Example 1: scTRIP enables systematic discovery of a wide variety of SV classes in single cells**

The underlying rationale of scTRIP is that each class of SV can be identified via a specific 'diagnostic footprint'. These diagnostic footprints capture the co-segregation patterns of rearranged DNA segments made visible by sequencing single strands of each chromosome in a cell, as follows: During S-phase, the DNA double strand unwinds, and the two resulting single strands (Watson ['W'] and Crick ['C']) act as templates for DNA replication. In Strand-seq, newly replicated strands incorporate Bromodeoxyuridine (BrdU)[21], which acts as a traceable label for these non-template strands (see **Fig. 1A** depicting the Strand-seq protocol)[24]. During mitosis, each of the two daughter cells receive one copy of each chromosomal homolog through independent and random chromatid segregation[21]. The labeled nascent strand is then removed, and the segregation pattern of each chromosomal segment is analyzed following strand-specific sequencing (**Fig. 1B**). scTRIP combines this strand-specific segregation information with read depth and haplotype phase information to capture newly-defined diagnostic footprints that characterize each SV class (**Fig. 1C-F**).

The diagnostic footprint of deletions (Del) is defined by read depth losses affecting a single haplotype, coupled with unaltered read orientation (**Fig. 1B,D** and **Table 1**). Duplications (Dup) are characterized by haplotype-specific gains with unaltered orientation (**Fig. 1D, right panel**). In the case of balanced inversions (Inv), read orientation is altered with the re-oriented reads mapping to a single haplotype at constant read depth (**Fig. 1B,E**). Re-oriented reads co-locating with a read depth gain on the re-oriented haplotype signify an inverted duplication (InvDup; **Fig. 1E, right panel**). In the case of inter-chromosomal SVs, physically connected segments will co-segregate during mitosis, allowing the discovery of translocations. This is because segments originating from different chromosomes will now be adjacent to each other and thus will receive the same non-template strand-label during replication (**Fig. 1B**). Segments showing correlating strand states in different cells without a change in read depth characterize balanced translocations (**Fig. 1F**), whereas unbalanced translocations exhibit a similar footprint in conjunction with a read depth gain of the affected haplotype. Finally, altered cellular ploidy states also exhibit their own diagnostic footprints (**Table 2**).

To exploit these diagnostic footprints a joint calling framework enabling the systematic discovery of SVs on a cell-by-cell basis was developed. Described in detail in the following, the framework first aligns, normalizes and places strand-specific read data into genomic bins, and assigns template strand states and chromosome-scale haplotypes for all cells. It then identifies putative SVs by segmentation (**Methods**), and using a Bayesian model estimates genotype likelihoods for each segment and each single cell (**Fig. 1G**). The model integrates read depth, strand and haplotype phase signals to predict the most probable SV class described by the diagnostic footprints. By performing SV discovery in a haplotype-aware manner, our joint calling framework also combines signals across cells (**Methods**) to sensitively detect subclonal SVs in a heterogeneous cell population. Finally, by analyzing adjacent SVs arising on the same haplotype it can unravel complex rearrangements, an abundant class of somatic structural variation in cancer[25,26]. As a first benchmark, the inventors performed simulation experiments and observed excellent recall and precision after randomly placing SVs into cell populations in silico, even down to a single cell.

*A detailed description of the scTRIP framework:*

The core computational framework described here in further detail, has been developed for detecting Dup, Del, Inv, InvDup, and 'other/complex SV' classes in single cells, based on scTRIP's SV diagnostic footprints.

Input data required by the framework are a set of single-cell (Strand-seq) BAM files from the same donor sample. In the present study, these data were aligned to build GRCh38 of the human reference genome (GCA 000001405.15 GRCh.38 genomic.fna). To later enable haplotype phasing and haplotype-resolved SV assignments, the framework performs re-genotyping of SNPs provided by the 1000 Genomes Project (1000GP; phase 3) to detect heterozygous sites from the single-cell input data. When using the framework a VCF file with these 1000GP SNP sites is to be provided as input. Alternatively, the scTRIP pipeline is able to call SNPs directly from the single-cell data or to use externally generated SNP calls for a given sample, e.g. based on bulk WGS. Additionally, a tab-separated file with normalization factors (see below) per bin across the genome is used as input to the framework.

*Binned read counting in single cells.* At first, reads in all individual cells are binned, for each strand. Bins have a fixed width (default: 100kb), starting from position o up the end of the chromosome. Mapped reads were assigned to bins based on their start position and filtered according to the following criteria: non-primary and supplementary alignments are excluded; alignments with the QC failure flag are excluded; PCR duplicates are excluded; reads with mapping quality <= 10 are excluded. In case of paired-end data only the first read of each pair (based on the BAM flag 0x40) was used to avoid double-counting. Cells with too little coverage (median count per bin of 3 or less) were removed by default. The parameters $p$ and $r$ of the NB distribution were determined in the same manner as for SV classification (see respective section below). During parameter estimation, bins were excluded from the parameter estimation process if their mean coverage across all cells was very low (<0.1, where coverage was previously normalized to 1) or if they showed a highly abnormal WC/(WC+CC+WW) fraction *($WC_{frac}$)* across cells. Bins were deemed abnormal if exhibiting either $WC_{frac}$<0.05 or $WC_{frac}$> 0.95, reflecting bins that either never showed WC status, or those that exhibited always WC status, as for example often seen in regions within or near centromeres.

*Coverage normalization in single cells.* The framework pursues normalization of read coverage prior to SV calling. To estimate suitable parameters for normalization, Strand-seq data recently generated by the Human Genome Structural Variation Consortium (HGSVC) was analysed comprising 9 lymphoblastoid cell lines from the 1000 Genomes Project (1000GP) (*i.e.*, samples NA19238, NA19239, NA19240, HG00731, HG00732, HG00733, HG00512, HG00513, and HG00514). The inventors utilised 1058 cells from these HGSVC samples sequenced via Strand-seq, obtained from ftp.1000genomes.ebi.ac.uk/vol1/ftp/data_collections/hgsv_sv_discovery/working/20151203-s trand_seq/, and subjected these cells to the same binning scheme described above. Analysis of several of these 1000GP samples showed that these do not carry any germline copy number variants (CNVs) ≥200kb. To identify a scaling factor for normalization these HGSVC Strand-seq data was aggregated, and first masked regions using any of the following 'exclusion criteria': observed mean coverage <50%, observed mean coverage >200%, or observed standard deviation larger than the mean coverage. Then, using the remaining bins, the observed mean bin coverage in the test samples was modelled assuming a linear relationship to the mean HGSVC bin coverage, which explained 66% of the variance with a slope of ~0.6. This linear relationship was used to derive a scaling factor for each bin, which subsequently was applied to all cells of the present study.

Also a "blacklist" was created of regions exhibiting strong sequencing/mapping abnormalities to avoid false positive somatic variant calling. To construct said blacklist, one starts from the 'masked regions' with unusual coverage in the independent HGSVC samples (see previous paragraph). Then such intervals are progressively merged if they exhibited a distance of 500kb or less (which avoided generation of a highly fragmented blacklist). Lastly, it was ensured that no known polymorphic inversion was accidentally masked by removing all intervals from our blacklist that overlapped with a germline inversion larger than 100kb in size reported by the HGSVC. The resulting blacklist was used in all following analyses, which considered regions outside of the blacklisted intervals for single cell SV calling.

*Joint segmentation of single cells.* With regard to segmentation, the strategy suggested by Huber *et al.* Was applied to perform segmentation on a multivariate input using a squared-error assumption[35]. Therefore, the binned read count data for all single cells of a sample were simultaneously used as input, with the rationale that SVs that recur in multiple cells can reinforce each other. Given a number of allowed change points $k$, a dynamic programming algorithm finds the discrete positions of the $k$ change points with minimal sum of squared error (SSE). The change points at level $k$ are computed using knowledge about a set of $k-1$ optimal change points through dynamic programming. This algorithm uses a cost matrix, to determine the cost (summed squared error) of every possible consecutive segment. While the same direction of change was assumed in all samples in the original implementation of Huber *et al.,* we adapted the algorithm to calculate this cost matrix for each cell and strand separately. The inventors additionally adapted the cost matrix to penalize segments which are below 200kb in size, as a means of avoiding over-segmentation. The segmentation procedure (mosaic segment), performs the segmentation separately for each chromosome and outputs the resulting change points up to a maximum number of allowed change points. Appropriate segmentation parameters were selected by assessing the benefit of increasing the number of change points (k) in terms of the summed squared errors (SSE) of the piecewise constant function compared to the actual count data. Let $SSE_k$ be the residual error associated to partitioning a chromosome into k segments. Then the smallest number $k$ was selected such that $SSE_k - SSE_{k+1}$ is below a user-set parameter (default: 0.1, which is used in this study) to adjust the number of change points $k$ for a chromosome.

*Strand state and SCE detection in single cells.* Detecting SV diagnostic signatures depends on whether the corresponding segment in a single-cell followed a WW, CC, WC, or CW pattern of mitotic segregation (**Table 1**). In context of the present invention refer to the underlying baseline distribution of W and C reads along a chromosome as the "ground state" (see **Methods**). While the ground state usually stays the same along the length of a chromosome, it can be altered by sister chromatid exchanges (SCEs), which underlie mitotic patterns of recombination unrelated to structural variation. Changepoints in Strand-seq data that result from mitotic recombination events/SCEs represent a source of "noise" that the method of the invention is able to correct for. Fortunately, SCEs happen independently in each single cell[2], and unlike SVs, SCEs are not transmitted clonally to daughter cells (*i.e.* are only detectable in the cell they occur in[2]). Hence, changepoints resulting from SCEs are very unlikely to recur at the same position in >1 cell of a sample[1,2]. The present invention uses changepoint recurrence as a key criterion for distinguishing SCEs from SVs. To identify SCEs, the same segmentation strategy was employed as described above, but to each single cell *separately* rather than jointly. To do so, the threshold to select the number of breakpoints k (see above) was set to 0.5. The inventors assigned an *observed state* to each resulting segment by computing the fraction $f_{WC} = W/(W + C)$ and assigning state WW if $f_{WC} > 0.8$, state CC if $f_{WC} < 0.2$ and state WC/CW otherwise. The states of neighbouring segments were compared to each other and if the states were unchanged the intervening changepoint was discarded, while the remaining changepoints were subsequently further considered as putative SCEs. Note that herein "WC/CW" is used to indicated that no distinction is made between these two states in this step, distinguishing the two happens in the subsequent strand phasing step.

An important consideration is that in some cases, changepoints detected in this way may correspond to SVs rather than SCEs. The following strategy was employed to select a high confidence list of SCEs: first select those changepoints far away (>500kb) from any breakpoint identified during the joint segmentation (see previous paragraph); these changepoints are likely to represent true SCEs. With this provisional set of candidate SCEs, each of the three ground states WW, CC, WC/CW was considered to determine a plausible "ground state". The assumption was employed that a given state at the beginning of a chromosome and a set of SCE positions (which change the state) uniquely determine the state for every segment on the chromosome. To assess which of the three ground states (WW, CC, or WC/CW) at a chromosome start to pick, the *discordant length* was computed, defined as the total length of genomic intervals for which the observed state differs from the predicted ground state. Although highly unlikely, in rare occasions, an SCE changepoint may appear to coincide with an SV breakpoint. In order to enable the method of the invention to recover such rare SCEs, all putative SCEs closer than 500kb to a breakpoint in the joint segmentation were analyzed. If adding one of these putative SCEs reduces the *discordant length* by 20Mb or more, the method of the invention assigns these SCE status. Doing so, the method of the invention is able to avoid that missed SCEs result in an incorrectly assigned ground state along larger parts of a chromosome. Note that adding at most one such additional SCE precludes masking most true SVs, which have two breakpoints, whereas SCEs lead typically only to a single "switch" (changepoint) in W and C states along a chromosome. Also, it should be noted that since SCEs never associate with copy-number alteration, the chance that SCEs are confused with SVs is near "zero" for many SV classes - that is for, Del, Dup, InvDup, and complex rearrangements - even if these SV are present only in a single cell. Thus, in reality, SCEs are only very rarely incorrectly assigned SV status (as also evidenced by our experimental validation data).

*Chromosome-length haplotype phasing using single-cell sequencing data.* To facilitate haplotype-aware SV calling, the inventors phased all available chromosomes using StrandPhaseR. While building whole-chromosome haplotypes for a sample, regions were assigned represented by both W and C strands as either WC or CW for each cell. That is, reads were used overlapping heterozygous SNPs to determine whether haplotype H1 was represented by W reads and H2 by C reads (a situation we denote as WC), or vice versa (denoted CW) (see **Methods).** In addition to this refined characterization of the ground state, StrandPhaseR outputs the chromosome-wide haplotypes as a VCF file, which the inventors later utilized in the "haplotagging" step. This phasing step of the framework requires at least a few dozen SNPs per chromosome. To ensure availability of enough SNPs, the inventors re-genotyped germline variants previously identified in the 1000GP, using Freebayes with options "-@ <1000GP-snps.vcf> --only-use-input-alleles <input.bam> --genotype-qualities". All heterozygous SNPs were retained with QUAL>=10. Alternatively, the present framework can use externally provided SNPs. To boost the usable coverage for SNP calling, we performed a cell sorting experiment, independently sorting 100 cells (termed the '100 cell control') in each sample, followed by short-read whole genome sequencing to 1.9x mean coverage.

The number of high throughput sequencing reads mapped to genomic windows (or bins) were previously shown to be in agreement with a negative binomial (NB) distribution[37], which can account for overdispersion. The inventors employed the NB distribution as the basis for the Bayesian framework. The NB distribution has two parameters, $p$ and $r$, which are estimated from the observed read counts as follows. The value $n$ was denoted as the number of single cells analyzed in a sample. The assumption is that the number of reads sampled from each single-cell at a fixed bin size is an NB random variable. In reality, the coverage of single cells will be varying resulting in different NB parameters for each cell. Key for parameter estimation is that not only the coverage of individual single cells, but also the total coverage of all single cells together, are derived from an NB distribution. This implies that all single cells should have the same $p$, therefore there are $n+1$ free parameters to estimate (one $p$ parameter and $n$ dispersion parameters).

In an NB distribution, the ratio of the mean to the variance is equal to $1-p$. Having the same $p$ parameter over all single cells implies that the ratio of mean to variance is constant across all single cells. Consequently, the mean and variance of binned read counts among single cells share a linear relationship in which the line connecting these mean-variance points for single cells passes the origin coordinate with a slope determining the *p* parameter. This relationship allows estimation of the shared *p* parameter: for each single-cell, the inventors compute the empirical mean and variance of the observed read counts in fixed-sized bins across the genome. If one denotes the set of empirical mean-variance pairs by $(m_1, s^2_1)$, $(m_2, s^2_2)$, ..., and $(m_n, s^2_n)$, the *p* parameter is estimated as follows:

$$p = \frac{\sum_{i=1}^{n} m_i}{\sum_{i=1}^{n} s^2_i}$$

After obtaining *p*, it is estimates the dispersion parameter $r_j$ of each single cell *j* by setting the distribution mean to the average read count per bin of that single cell. The inventors employed a trimmed mean for estimating the dispersion parameters (with trim parameter set to 0.05), to remove the effect of abnormally high or zero read counts (e.g. seen in regions of low mappability).

*SV diagnostic footprints.* Each SV diagnostic footprint (**Fig. 1**) can be translated into the expected number of copies sequenced in W and C orientation contributing to the genomic segment under consideration. **Table 1** shows this relationship for each SV class, both for chromosomes where both haplotypes are represented by different template strands (here referred to as 'WC/CW chromosomes') and for such where both haplotypes are represented by the same template strand ('WW chromosomes' and 'CC chromosomes'). Every haplotype-resolved SV implies a particular segment strand pattern in WC, CW, WW, and CC chromosomes, respectively. For example, if the ground state of a single cell in a chromosomal region is WW and the SV status in a segment in that region is 'inverted duplication of the paternal haplotype represented on the W strand', the observed segment strand pattern will be WWC in this given single cell. By comparison, if the ground state is WC (W for the H1 haplotype), and the SV status is deletion of the H1 haplotype, the observed segment strand pattern is C (see **Table 1**). These expectations are formalized in our Bayesian model, which we describe in the following.

The inventors utilized a Bayesian model (**Fig. 1h**) to compute haplotype-resolved SV genotype likelihoods for each segment in each single cell. The inventors model *V*, the SV type to be inferred, as a pair $(\vec{C}, \overleftrightarrow{C})$, where $\vec{C}$ gives the number of copies of that segment in forward direction and $\overleftrightarrow{C}$ gives the number of copies of that segment in reverse direction (i.e. when an inversion is present). That is, the pair (1,0) encodes the reference state of a haplotype (one forward copy and zero inverted copies). As illustrated in **Fig. 1h**, each segment $k \in K$ and haplotype $h \in H = \{h_1, h_2\}$ in single cell $j \in J$ comes with a variable *V* for this SV state, which we refer to as $V_{j,k,h}$. Together with the ground state *T*, each SV state *V* deterministically leads to a corresponding "copy number" observed in Crick direction $N^C$ and in Watson direction $N^W$, as explained in the previous section on SV diagnostic signatures (also see **Table 1**). Conditional on the sum of Crick and Watson copy numbers of both haplotypes, the corresponding coverages $X^C$ and $X^W$ are assumed to follow a negative binomial (NB) distribution

$$X^W_{j,k} \mid (N^W_{j,k,h_1} + N^W_{j,k,h_2}) \sim NB(r^W_{j,k}, p)$$

$$X^C_{j,k} \mid (N^C_{j,k,h_1} + N^C_{j,k,h_2}) \sim NB(r^C_{j,k}, p)$$

for each single cell *j* and segment *k*. Here, *p* is the estimated common *p*-parameter of the NB distribution (see *Estimating Negative Binomial parameters* above), and $r^W_{j,}$ and $r^C_{j,}$ are proportional to the estimated parameter $r_j$ (also see above), the segment size $L_k$ and the Watson and Crick segment copy numbers ( $N^W_{j,k} = N^W_{j,k_{h1}} + N^W_{j,k_{h2}}$ and

$N^C_{j,k} = N^C_{j,k_{h1}} + N^C_{j,k_{h2}}$ ) and hence are computed as follows (for $d \in \{W, C\}$):

$$r^d_{j,k} = \begin{cases} \frac{1}{2}\alpha r_j L_k & \text{if } N^d_{j,k} = 0 \\ \frac{1}{2}(1-\alpha)r_j L_k N^d_{j,k} & \text{otherwise} \end{cases}$$

In this formula, $\alpha$ is a parameter in our model indicating the fraction of "background reads", which represents noise in Strand-seq data (for example due to regions with incomplete BrdU incorporation or removal)[1,2]) These background reads are taken into account by assuming. $\alpha$ = 0.1, which reflects an upper bound for the abundance of such background reads observed in practice. Note that the $\frac{1}{2}$ coefficients in the above formula serve to scale the dispersion parameter to copy number 1($r_j$ is estimated above to reflect a diploid state of copy number 2). In summary, every haplotype-resolved SV class ($V$) in a segment together with the ground state ($T$), define a Watson and Crick copy number ($N$) used to compute the NB likelihood of observed read counts. Through this mechanism, likelihoods for all diagnostic signatures in **Table 1** are obtained.

*Incorporating haplotype-specific sequencing reads ('haplotagging').* One of the key advantages of scTRIP is the ability to utilize haplotype information made available through strand-specific sequencing. In the base model described in the previous paragraph, this haplotype-awareness is brought forth by distinguishing WC from CW ground states (also see *Chromosome-length haplotype phasing using single-cell sequencing data*). The present framework is additionally able to make use of reads not directly assigned to a haplotype (i.e. those in WW and CC regions) owing to their overlap with a haplotype-phased SNP. This feature can further facilitate validation and falsification of putative SVs seen only in few or even individual cells. The inventors utilize the whole-chromosome haplotypes generated using StrandPhaseR [36] to tag reads by haplotype using the 'haplotag' command of WhatsHap [38,39], resulting in one 'haplotagged' BAM file per single cell library. These BAM files are then used to compute the number of Watson/Crick reads that could be tagged by haplotype H1/H2, respectively, for each segment and each single cell. The resulting happlotagged read counts are incorporated in the Bayesian model as random variables $X_{tag}^W$ and $X_{tag}^C$ (see **Fig. 1h**). The inventors employed a multinomial distribution to model the conditional distribution of these tagged read counts given the (haplotype- and strand-specific) copy numbers $N^C$ and $N^W$. More precisely, we defined parameters of the multinomial distributions $p_{j,k,h_1}^C$, $p_{j,k,h_2}^C$, $p_{j,k,h_1}^W$, and $p_{j,k,h_2}^W$, for each segment $k$ and single cell $j$, such that they are proportional to the corresponding copy numbers:

$$p_{j,k,h}^d \propto \max(\alpha, N_{j,k,h}^d)$$

where $d \in \{W, C\}$ as before. Here, $\alpha$ is again a rate of background reads (set to $\alpha$ = 0.1) and the $p_{j,k,h}^d$ are normalized to sum up to one. Given the total number of reads and the (haplotype- and strand-specific) copy numbers $N^C$ and $N^W$, the tagged reads are multinomially distributed:

$$(X_{j,k,h_1,tag}^C, X_{j,k,h_2,tag}^C, X_{j,k,h_1,tag}^W, X_{j,k,h_2,tag}^W) \sim \text{Multinomial}(p_{j,k,h_1}^C, p_{j,k,h_2}^C, p_{j,k,h_1}^W, p_{j,k,h_2}^W)$$

*Employing the Bayesian model for SV calling.* To utilize our Bayesian model for SV calling, the inventors defined prior probabilities and combined them with the model-based likelihoods for each single cell and segment. The inventors started by regularizing the raw likelihoods, adding a small constant (set to $10^{-6}$) to all likelihoods and renormalizing afterwards. This ensures that very small values (or hard zeros) are avoided and corresponds to the error assumption that every SV genotype is possible with this given small probability, no matter what the data suggests. Then, two forms of priors were used. First, biological knowledge was captured on the plausibility of observing certain event types. To do this, the priors were defined to be proportional to a pre-specified constant per SV type and chose these constants as follows: ref=200, del/inv/dup=100, invdup=90, other/complex=1. While this choice is somewhat arbitrary, it encourages the SV calling process to prefer the reference state (ref) over canonical SVs (del/inv/dup/invdup) over more exotic SV classes, for example involving an inversion on one haplotype and a deletion on the other haplotype (other/complex) - unless the model observed sufficient evidence to overwhelm these priors. Thus, the caller was required to gather more evidence for SV classes deemed implausible. The second type of priors applied acts on each segment separately and uses the raw likelihoods computed by the model across all cells to compute a probability distribution over all SV types. That is, for each segment the likelihoods were summed up per SV type across all cells and normalized to one, which corresponds to estimating the frequency of each SV genotype for that segment. The intuition behind this procedure is that one needs to encourage the SV caller to prefer SV types present in many cells over those SV types present only on few cells - unless the evidence inherent to the genotype likelihoods is strong enough to overwhelm these priors. Before applying these priors, the prior of each SV genotype was set to zero if the estimated frequency of that genotype was below a threshold, which was termed GTCLTTOFF (set to 0.05 for the strict call set and set to o for the lenient call set). Effectively, this means that the strict parameterization only considers an SV genotype if the likelihoods across all cells suggest it to be present in the cell population at an expected frequency of at least 5%. The lenient call set, in contrast, disables this cutoff by setting it to zero and hence readily permits SV genotypes present in individual cells only. Lastly, the inventors used the resulting posterior probabilities to compute log odds ratios (of an SV genotype vs. the reference state), and accepted an SV call if the log odds ratio was at least 4. SV calls in segments with >20% blacklisted bins were discarded.

*Call set post-processing: Filtering:* A filtering routine was developed to be used only in conjunction with the strict parameterization, the main goal of which is to arrive at a high confidence SV callset for all SVs with VAF greater than 5%. This filtering routine removes rare inversions seen in only 1 or 2 cells, since rare inversions may occasionally correspond to SCEs. This routine further removes SV calls exhibiting particular biases, most importantly, those biased to occur largely in the context of a certain ground state. In particular, while SVs can be detected in the context of all four ground states (WW, CC, WC and CW; see **Table 1**). Calling deletions or duplications on WW or CC chromosomes is indeed conceptually related to previously developed copy-number profiling methodology; *i.e.*, SVs called on WW or CC chromosomes will not benefit from the ability of scTRIP to call these SVs based on strand-specific read depth gain or loss (**Fig. 1, Table 1**).

*The following hard filters were implemented to be used with the strict parameterization:*

(i) Removal of inversions seen in less than 3 cells.

(ii) Removal of deletions seen in multiple cells, if these show a bias towards occurring mostly in WW and CC chromosomes with less than a third seen in WC or CW regions (deletions with log odds ratio ≥50 will not be removed by this hard filter). As reasoned further above, we implemented this filter since deletions that are repeatedly seen in the WW or CC ground state, but not or only rarely in the WC ground state, are (according to our experience) of lower confidence.

(iii) Removal of duplications seen in multiple cells, if these show a bias towards occurring in WW and CC chromosomes, with less than a third seen in WC or CW chromosomes (duplications with log odds ratio ≥50 will not be removed by this hard filter). As reasoned further above, we implemented this filter since according to our experience duplications that are repeatedly seen in the WW or CC ground state, but not or only rarely in the WC ground state, are of lower confidence.

(iv) Removal of SVs overlapping UCSC annotated segmental duplications in the genome (file: segDups_hg38_UCSCtrack.bed.gz) by more than 50% (we found such SV calls to be of lower confidence).

*Merging:* A merging routine was developed to be used in conjunction with the strict parameterization, which groups adjacent SVs with a similar VAF (where VAF ≥ 0.1) into a single SV call to avoid over-segmentation and produce a final high confidence somatic SV sites list. To this end, the inventors considered VAFs of adjacent SVs to be similar if $VAF_{SV1}/VAF_{SV2} \geq 0.75$ (for cases where $VAF_{SV2}>VAF_{SV1}$) or $VAF_{SV2}/VAF_{SV1} \geq 0.75$ (for cases where $VAF_{SV1}>VAF_{SV2}$), and grouped all immediately neighboring SVs selected by this similarity criterion. SVs merged by this routine will nearly always correspond to a single structural variation event in validation experiments.

*Strict and lenient parameterizations of our single cell SV discovery framework.* As alluded to above, our framework comes with the ability to adjust for the tradeoff between sensitively calling SVs present at low VAF, and accurately identifying SVs consistently seen among cells. The inventors parameterized this tradeoff into a 'strict' and 'lenient' SV caller, whereby the 'strict' caller optimizes precision for SVs seen with VAF≥5%, while the 'lenient' caller targets all SVs including such present only in a single cell. These parameterizations differ in three settings: the GTCUTOFF (see *Using the Bayesian model for SV calling*), whether or not haplotagged reads counts are incorporated (see *Incorporating haplotype-specific sequencing reads*), and whether filtering is enabled (see *Call set post-processing*). The strict caller uses GTCUTOFF=0.05, while the lenient caller uses GTCUTOFF=o. For the strict caller, we disabled the haplotagging feature, while we enabled haplotagging for the lenient call set - with the reasoning that haplotagging is mostly valuable to resolve putative SVs with low VAF. Lastly, we used the filtering described in the previous paragraph for the strict caller, while the inventors proceeded with the unfiltered set for the lenient caller. It is recommended using the strict caller to enable reliable detection of subclonal SVs down to a VAF of 5%. The lenient caller should be used for analyzing SVs across the whole VAF spectrum down to the individual cell.

**Example 2: SV landscapes of RPE cells uncovered by scTRIP**

To investigate single cell SV landscapes using scTRIP the inventors next generated strand-specific DNA sequencing libraries from telomerase-immortalized retinal pigment epithelial (RPE) cells. hTERT RPE cells (RPE-1) are commonly used to study patterns of genomic instability[20,27-29], and additionally C7 RPE cells were used, which show anchorage-independent growth used as an indicator for cellular transformation[30]. Both RPE-1 and C7 cells originate from the same anonymous female donor. The iventors sequenced 80 and 154 single cells for RPE-1 and C7, respectively, to a median depth of 387,000 mapped non-duplicate fragments (**Methods**). This amounts to only 0.01X genomic coverage per cell.

The inventors first searched for Dels, Dups, Invs and InvDups. Following read normalization, 54 SVs in RPE-1 were identified, and 53 in C7 cells. 22 SVs were present only in RPE-1, and 21 were present only in C7, and thus likely correspond to sample-specific SVs (*i.e.* SVs that formed somatically or in the cultured cells rather than corresponding to germline variants; hereafter simply referred to as 'somatic SVs'). Two representative SVs are shown in **Fig. 2A**, including a 1.4 Megabase (Mb) somatic Dup seen in RPE-1, and a 800 kb somatic Del detected in C7. While all but one of the Dels and Dups events were unique to RPE-1 and C7, Inv and InvDup events, including an Inv on 17p shown in **Fig. 2B**, were largely shared between both. These variants mapped to sites of known inversion polymorphisms[23]. The inventors also identified chromosome arm-level CNAs, including deletion of 13q in C7, and duplication of a large 10q region in RPE-1. The 13q-arm showed a 1:0 strand ratio diagnostic for monosomy (**Fig. 2C**), whereas the gained 10q region exhibited 2:1 and 3:0 strand ratios diagnostic for trisomic regions (**Fig. 2D**).

**Example 3: Dissecting complex cancer-related translocations in single cells**

To assess the ability of scTRIP to detect a wider diversity of SV classes, the inventors subjected RPE-1 cells to the CAST protocol[28]: the inventors silenced the mitotic spindle machinery to construct an anchorage-independent line (BM510) likely to exhibit genome instability. The inventors sequenced 145 single BM510 cells detecting overall 67 SVs when searching for Dels, Dups, Invs and InvDups events. Additionally, several DNA segments did not segregate with the respective chromosomes they originated from, indicating inter-chromosomal SV formation (**Fig. 3A**). The inventors performed translocation detection with scTRIP searching for diagnostic co-segregation footprints (**Fig. 3B**), and identified four translocations in BM510 (**Fig. 3B,C**). The inventors additionally subjected RPE-1 and C7 to translocation detection, identifying one translocation each (**Fig. 3D**).

One translocation was shared between RPE-1 and BM510 and involved the aforementioned gained 10q segment that underwent unbalanced translocation with a chromosome X haplotype (**Fig. 3B**). The inventors leveraged the footprints of sister chromatid exchange events[21] to orient and order the segment, which placed the 10q gain to the telomeric tip of Xq, consistent with the published RPE-1 spectral kaiyotype[27] (**Fig. 3C**). In BM510, scTRIP also uncovered balanced reciprocal translocations involving 15q and 17p (**Fig. 3B,C**). Notably, a *de novo* somatic inversion was additionally detected on the same 17p haplotype, which shared one of its breakpoints with the reciprocal translocation (**Fig. 3C**). Since these SVs shared one of their breakpoints, it is likely that both arose jointly, potentially involving a complex rearrangement process. Analysis of the locus revealed that the inversion encompassed the *TP53* gene, and upon translocating fused the 5′ exons of *TP53* to coding regions of the *NTRK3* oncogene[32] (**Fig. 3E**). This suggests that scTRIP can reveal fusion genes using single cell sequence data.

Bulk whole genome sequencing (WGS) and RNA-Seq (**Methods**) analyses revealed excellent accuracy and specificity of the inventor's framework. The inventors validated all translocations (100%), with 4/5 recapitulated by WGS and the remaining der(X) t(X;10) event by the existing karyotype data[27]. No additional translocation was detected in the deep sequencing data, indicating excellent sensitivity of scTRIP. WGS failed to verify the der(X) t(X;10) unbalanced translocation because the chrX breakpoint resides in highly repetitive telomeric DNA (resulting in ambiguous alignments hampering read pair analysis), whereas scTRIP uses mitotic co-segregation patterns not affected by repetitive breakpoints. The inventors also observed increased expression of the duplicated haplotype in the context of the der(X) t(X;10) event, corroborating the inventor's haplotype placements. Finally, the inventors verified the presence of the complex rearrangement at 17p, and uncovered expressed *NTRK* gene fusion transcripts exclusive to BM510 (**Fig. 3D,E**). Thus, scTRIP enables the haplotype-resolved discovery of translocations by single cell sequencing with high accuracy and sensitivity, which included detection of a translocation missed by bulk WGS.

**Example 4: Single cell dissection of a complex DNA rearrangement** process

Cancer genomes frequently harbor clustered SVs arising via complex rearrangements, which facilitate accelerated cancer evolution[33]. One process leading to such SVs are breakage-fusion-bridge cycles (BFBs)[34-39]. BFBs are initiated by the loss of a terminal chromosome segment, which causes newly replicated sister chromatids to fuse. The resulting dicentric chromosome will lead to a chromosomal bridge, the resolution of which via DNA breakage can initiate a new BFB[14]. Thus BFBs successively duplicate DNA segments in inverted orientation (*i.e.* generate InvDups), typically with an adjacent deletion of the terminal chromosome segment of the same haplotype (*i.e.* terminal deletion, here referred to as 'DelTer'). BFBs rising to high VAF can be inferred from bulk WGS by analyzing 'fold-back inversions' (read-pairs aligning close to one another in inverted orientation)[34]. Owing to high coverage requirements, fold-back inversions cannot be systematically tracked in single cells. But the inventors reasoned that scTRIP could provide the opportunity to directly study BFB formation in single cells.

To investigate BFBs the inventors first turned to C7, in which fold-back inversions were previously described[28]. scTRIP located clustered InvDups on the 10p-arm in 152 out of 154 sequenced cells (**Fig. 4**). Closer analysis of 10p showed an amplicon with 'stepwise' InvDup events with an adjacent DelTer on the same haplotype, consistent with BFBs (**Fig. 4A-C** and **Fig. S12**). The remaining two cells lacking InvDups, notably, showed a larger DelTer affecting the same 10p segment (**Fig. 4C**). By aggregating sequencing reads across cells, the inventors identified 8 discernable segments along chromosome 10, which included the 10p amplicon (comprising six copy-number segments) and its adjacent regions (the 10p terminal region, and the centromere-proximal region) (**Fig. 4B**). To further characterize the genetic heterogeneity seen at 10p, the inventors inferred the cell-specific copy-number of all 8 segments (**Fig. 4D**). This revealed at least three distinct groups of cells with respect to 10p copy-number: (i) A large group presenting 'intermediate' copy-number with 100-130 copies detected for the highest copy-number segment (referred to as the 'major clone'). (ii) Two cells that lost the corresponding 10p region through a DelTer, (iii) A single cell exhibiting vastly higher copy-number (~440 copies), which may have undergone additional BFB cycles (**Fig. 4C**).

Additional SVs identified in C7 provided further insights into the rearrangements occurring in the major clone: Namely, the inventors detected an unbalanced translocation stitching a duplicated 15q segment to the 10p amplicon (**Fig. 4C**). The duplicated segment encompassed the 15q telomere (**Fig. 4C**), which may have stabilized the amplicon to terminate the BFB process. In further support of C7 containing at least three groups of cells with respect to 10p structure, the unbalanced translocation was absent from the two cells harbouring the extended DelTer, whereas the translocated region became further amplified in the cell with excessive 10p copy-number (**Fig. 4C**). A model of the temporal sequence of rearrangements leading to the major clone is shown in **Fig. 4E**. These data underscore the ability of scTRIP to characterize BFB cycles for which direct measurement by single cell sequencing was not possible previously.

### Example 5: Abundant BFB formation in anchorage-independent RPE cells

The frequency of BFB-mediated SV formation in somatic cells is unknown. Since scTRIP can systematically detect InvDup and DelTer footprints, the inventors searched all sequenced RPE cells (379 in total) (**Methods**) and identified 15 additional cells exhibiting a BFB formation signature. Out of these, 11 displayed the 'classical' BFB footprint - an InvDup flanked by DelTer on the same homolog with no other SV present on the homolog (**Fig. 4F**). The remaining four instances showed additional rearrangements on the same homolog as the BFB-associated SV. The inventors tested whether InvDup-DelTer footprints coincided by chance by searching for structures where an InvDup on one haplotype was flanked by a DelTer on the other haplotype. Amongst the 379 cells, InvDup-DelTer footprints always occurred on the same haplotype, consistent with the well-known BFB model[38]. 11 out of the 15 InvDup-DelTer events occurred in BM510 affecting 8% (11/145) of the sequenced cells and 4 occurred in C7 affecting 3% (4/154) of the cells. No InvDup-DelTer footprints occurred in RPE-1 cells (0%; 0/80) and thus BFBs occurred exclusively in the transformed, anchorage-independently growing cells. Copy-number estimates of the InvDup regions ranged from 3 to 9, indicating that up to three BFB cycles occurred in these cells (**Fig. 4F**).

Interestingly, all of these 15 InvDup-DelTer footprints were singleton events detected in isolated cells (*i.e.* none were shared across more than one cell) and hence are likely to represent chromosomes with sporadically formed and potentially ongoing BFB cycles. The inventors reasoned that SVs identified in individual cells can serve as a proxy for currently active mutational processes. Using scTRIP, the inventors systematically searched for other abundant SV mutational patterns in the RPE cell line in which the inventors had induced genomic instability (BM510). The inventors located 60 chromosomes with evidence of mitotic errors causing large (megabase-scale) deletion or duplication. Of these, 35/60 (58%) affected an entire homolog arm, 17/60 (28%) involved the tip of a homolog (terminal loss or gain) but not the whole arm, and 7/60 (12%) corresponded to whole homolog aneuploidy (monosomy or trisomy). The unifying characteristic of these abundant SV classes is that they can all result from mitotic segregation errors and reflect ongoing chromosome instability[40].

Further underscoring this, nine cells showed multiple clustered SVs affecting the same haplotype. This included those four cells showing the InvDup-DelTer footprint and at least one additional SV. By employing the infinite sites assumption[37], the inventors inferred the relative ordering of SVs occurring on the same haplotype in these cases, identifying instances where the formation of additional SV preceded BFB formation, as well as such were the formation of additional SV succeeded BFB formation. This analysis also revealed a single cell exhibiting multiple reoriented and lost fragments, all on the same haplotype, resulting in 12 SV breakpoints affecting a single homolog. This rearrangement potentially resulted from a one-off rearrangement burst (chromothripsis)[41,42] (**Fig. 4G**). Therefore, scTRIP enables systematic detection of *de novo* SV formation and discrimination of SV mutational processes, including BFBs and other complex rearrangements, in single cells.

### Example 6: Constructing the karyotype of a PDX-derived T-ALL sample from 41 single cells

To evaluate the potential diagnostic value of scTRIP, the inventors next analyzed patient-derived leukemic cells. Both balanced and complex SVs are abundant in leukemia, but largely escape detection in single cell studies geared towards CNAs[26,41,43]. The inventors characterized PDX-derived[44] samples from two T-cell acute lymphoblastic leukaemia (T-ALL) patients, to investigate the utility of scTRIP to characterize leukemic samples. The inventors first focused on $P_{33}$, a PDX-derived T-ALL relapse from a juvenile patient with Klinefelter Syndrome. The inventors sequenced 41 single cells and used these data to reconstruct the haplotype-resolved karyotype of the major clone at 200kb resolution (**Fig. 5A**). While most chromosomes were disomic, the inventors identified the typical XXY karyotype (Klinefelter Syndrome) and observed trisomies of chromosomes 7, 8, and 9. The inventors further detected 3 regions of CNN-LOH characterized by haplotype-losses in the presence of constant read depth and orientation. Furthermore, the inventors observed 6 focal CNAs, 5 of which affected genes previously reported to be genetically altered in and/or 'driving' T-ALL[43,45-47], including deletions of *PHF6, RPL2* and *CTCF* sized 300kb and larger, as well as homozygous deletions of *CDKN2A* and *CDKN2B* (**Fig. 5A**). The inventors also identified a t(5;14)(q35;q32) balanced translocation (**Fig 5A**) - a recurrent rearrangement in T-ALL known to target $TLX_3$ for oncogenic dysregulation[48]. While few individual cells exhibited karyotypic diversity, the majority of cells supported the karyotype of the major clone (**Fig. 5B**).

The inventors attempted verification of this karyotype with classical (cytogenetic) karyotyping obtained from primary T-ALL during diagnosis - the current clinical standard to genetically characterize T-ALL. Although this verified the duplications of chromosomes X, 7, 8 and 9, classical karyotyping failed to detect all the focal CNAs, and failed to capture the t(5;14)(q35;q32) translocation previously designed as 'cryptic' (*i.e.* 'not detectable by karyotyping')[49]. To verify the additional SVs detected by scTRIP, the inventors next employed CNA profiling by bulk capture sequencing $P_{33}$ at diagnosis, remission and relapse[50], as well as expression measurements. These experiments confirmed all (6/6, 100%) focal CNAs, and verified $TLX_3$ dysregulation supporting the occurrence of a t(5;14)(q35;q32) balanced translocation. The haplotype-resolved karyotype inferred via scTRIP comprised SVs down to 200kb in size, located a 'cryptic' translocation missed by clinical karyotyping, and was built using sequence data from 41 cells, amounting to only ~$0.9\times$ cumulative genomic coverage.

**Example 7: scTRIP uncovers previously unrecognized DNA rearrangements in a PDX-derived T-ALL**

The inventors next turned to a second T-ALL relapse sample obtained from a juvenile female patient (P1). The inventors sequenced 79 single cells of P1 and discovered two subclones, each represented by at least 25 cells (**Fig. 5C**). The inventors first focused on the clonal SVs, which included a novel 2.6Mb balanced inversion at 14q32 (**Fig. 6A**). Interestingly, one of the inversion breakpoints fell into the exact same 14q region affected by the P33 t(5;14)(q35;q32) translocation (**Fig. 6B**). Prior studies have shown that, depending on their precise breakpoint locations, t(5;14) translocations can target $TLX_3$ and *NKX2-5* oncogenes at 5q35 by repositioning enhancer elements at 14q35 into the vicinity of these oncogenes[43,51].

The observation that both T-ALL patients showed balanced SVs affecting the same region motivated further analyses. This revealed that the novel 14q32 inversion the inventors located in P1 juxtaposed the enhancer element-containing region 3' of *BCL11B*[48,51] into the immediate vicinity of the *T-cell leukemia/lymphoma 1A* (*TCL1A*) oncogene (**Fig. 6A**). Prior studies reported different enhancer-juxtaposing rearrangements in T-cell leukemia/lymphoma, as well as in T-ALL, resulting in *TCL1A* over-expression[52,53], and the inventors thus pursued RNA-seq to investigate differential expression in P1. This indeed confirmed *TCL1A* as the most highly overexpressed gene in P1 (>160-fold overexpression compared to five arbitrarily chosen T-ALLs; $P$=1.8E22 Wald test[54], Benjamini-Hochberg correction; **Fig. 6C, left panel**). The inventors reasoned that if *TCL1A* dysregulation had resulted as a consequence of the inversion, then *TCL1A* overexpression would be restricted to the rearranged haplotype. Leveraging scTRIP's haplotype-resolved SV assignments, the inventors performed allele-specific expression analyses, which showed that *TCL1A* overexpression indeed arose only from the haplotype carrying the inversion (**Fig. 6C, right panel**). These data implicate a novel inversion in driving oncogene expression. Further studies are needed to assess recurrence of this inversion in other T-ALL or T-cell malignancies, and to investigate the diversity of oncogene-dysregulating SVs involving the *BCL11B* enhancer. Due to its ability to perform scalable discovery of balanced SVs by shallow sequencing, scTRIP would be well-placed to investigate these questions in the context of larger patient cohorts.

The inventors next analyzed subclonal SVs in P1, and discovered a series of highly clustered subclonal rearrangements affecting a single 6q haplotype (VAF=0.32). These rearrangements comprised two Invs, an InvDup, a Dup, and three Dels, resulting in overall 13 detectable breakpoints spanning nearly 90 Mb of 6q (**Fig. 6D,E**). All cells exhibiting SVs at 6q showed evidence for the full set of 13 breakpoints. Furthermore, the copy-number profile oscillated between only three copy-number states[41], and the inventors observed islands of retention and loss in heterozygosity[41] (**Fig. 6F**) - a rearrangement pattern that is reminiscent of chromothripsis[41,42]. To corroborate these data, the inventors performed long (4.9kb) insert size mate-pair sequencing in bulk to deep (165x) physical coverage. While read depth alterations were barely discernible for this subclonal complex rearrangement, deep mate-pair sequencing confirmed all 13 subclonal SV breakpoints - thus verifying a subclonal rearrangement burst consistent with chromothripsis (**Fig. 6G**). These data underscore the ability of scTRIP to reveal subclonal complex SVs likely to be missed by standard bulk WGS[42].

**Discussion**

scTRIP enables the systematic detection of a wide variety of SVs in single cells using a joint calling framework that integrates read depth, strand, and haplotype phase. It can call subclonal SVs down to VAF<1% and identify SV formation processes acting in single cells, addressing unmet needs of SV detection methods[10,13,26,55,56]. Previous single cell studies investigating distinct SV classes have done so by sequencing only relatively few selected cells deeply after WGA[10,17,57]. And while prior SV detection efforts using Strand-seq were limited to germline inversions[23], the computational advance presented here enables systematic discovery of CNAs, balanced and unbalanced translocations, inversions, inverted duplications and the outcomes of complex SV formation processes including BFBs and chromothripsis - all in single cells. Notably, scTRIP is further able to resolve repeat-embedded SVs (exemplified by an unbalanced translocation exhibiting a breakpoint in telomeric DNA), a class of SV that remains largely inaccessible to standard WGS in bulk. Moreover, SVs detected by scTRIP are haplotype-resolved, which helps to reduce false positive calls and allows integrating allele-specific gene expression data[57,58].

The inventors showcase the ability of scTRIP to measure SV formation processes by identifying BFB cycles in up to 8% of cells from transformed RPE cells, indicating that SV formation via BFB cycles is markedly abundant in these cells. Although initially described ~80 years ago[38], scTRIP now allows the direct and unbiased measurements of BFBs in individual somatic cells. BFB cycles were the most abundant SV formation process identified after chromosomal arm-level and terminal loss/gain events, all of which can result from chromosome bridges[40,59]. BFB cycles occur in a wide variety of cancers[14], can precipitate other mutation processes such as chromothripsis[37], and correlate with disease prognosis[60]. BFB cycles have also been reported outside of somatic cells, that is, in cleavage-stage embryos following *in vitro* fertilization, as revealed by hybridization-based single cell analysis[58]. It is estimated that 20% of all somatic deletions, and >50% of the entirety of somatic SVs in cancer genomes[25,26], arise as complex DNA rearrangements. By enabling direct and robust measurement of these rearrangement processes in single cells, scTRIP will facilitate future investigations on the role of complex SVs in clonal evolution.

The inventor's study also exemplified a potential value for disease classification by surveying balanced and unbalanced SVs, complex SVs, and karyotypic heterogeneity in patient-derived leukemic cells. The inventors constructed the molecular karyotype of a T-ALL sample at 200kb resolution using 41 single cells, amounting to only 0.9x genomic coverage. This revealed submicroscopic CNAs and oncogenic DNA rearrangements invisible to cytogenetic methods currently used in the clinic. Classical cytogenetics is typically pursued for only a limited number of metaphase spreads per patient and normally fails to capture low levels of karyotypic heterogeneity accessible to scTRIP. In one of the T-ALL patients The inventors discovered a subclonal chromothripsis event, highlighting potential utility for disease prognosis, since chromothripsis has been associated with dismal outcome in leukemia[61]. Studies of aberrant clonal expansions in healthy individuals[10] and lineage tracing in cancer patients[62] may also be facilitated by scTRIP in the future. Another potential application area is in rare disease genetics, where scTRIP may help resolve "unclear cases" by widening the spectrum of accessible SVs leading to somatic mosaicism[56]. Furthermore, the inventor's framework can be used to assess genome integrity in conjunction with cell therapy, gene therapy, and therapeutic CRISPR-Cas9 editing, which can result in unanticipated (potentially pathogenic) SVs[63,64]. The ability of scTRIP to generate high-resolution karyotypes could be employed to detect the presence of such unwanted SVs to address safety concerns pertaining to these future therapies.

scTRIP utilizes strand-specific data generated by Strand-seq, which requires labeling chromosomes during replication. Therefore, non-dividing, apoptotic, or fixed cells cannot be sequenced. However, many key cell types are naturally prone to divide or can be cultured, which for example includes fresh or frozen stem and progenitor cells, cancer cells, cells in regenerating or embryonic tissues, iPS cells and cells from diverse model systems including organoids. Moreover, in the future the computational framework underlying scTRIP could be used with strand-specific methods generating reads in the absence of cell division[65].

The inventor's approach enables systematic studies of somatic SV landscapes with much less sequence coverage than WGA-based single cell methods. The inventors demonstrated robust SV discovery using ~2000-fold less reads than required for prior read-pair or split-read based methods[12]. Single cell sequencing to deep coverage, using WGA, can enable mapping somatic SVs <200kb in size, and thus will remain useful for detecting small CNAs or retrotransposons. Compared to scTRIP, however, WGA-based single cell analyses are subject to the limitations of paired-end analyses, including susceptibility to allelic dropouts, difficulties in detecting repeat-embedded SVs, limited scalability and high costs[17]. The combined reagent costs for Strand-seq are ~15$ per cell, and the protocol is readily scalable (see **Methods**) meaning scTRIP enables systematic studies of SV landscapes in hundreds of single cells. Low-depth methods for CNA profiling in single cells, for which scalable methods exist, detect CNAs of 1 to 5 Mb in size[16,18]. These methods show promise for investigating subclonal structure, particularly in cancers with abundant CNAs, but miss key SV classes and fail to identify or discriminate between different SV formation processes.

In conclusion, the joint calling framework of scTRIP enables systematic SV landscape studies in single cells to decipher derivative chromosomes, karyotypic diversity, and to directly investigate SV formation processes. This provides important value over existing methods, and opens up new possibilities in single cell sequencing and genetic heterogeneity studies.

REFERENCES

The references are:

**References**

1. Ciriello, G. et al. Emerging landscape of oncogenic signatures across human cancers. Nat. Genet. 45, 1127-1133 (2013).

2. Mertens, F., Johansson, B., Fioretos, T. & Mitelman, F. The emerging complexity of gene fusions in cancer. Nat. Rev. Cancer 15, 371-381 (2015).

3. Northcott, P. A. et al. The whole-genome landscape of medulloblastoma subtypes. Nature 547, 311-317 (2017).

4. Beroukhim, R., Zhang, X. & Meyerson, M. Copy number alterations unmasked as enhancer hijackers. Nat. Genet. 49, 5-6 (2016).

5. Northcott, P. A. et al. Enhancer hijacking activates GFI1 family oncogenes in medulloblastoma. Nature 511, 428-434 (2014).

6. Kim, C. et al. Chemoresistance Evolution in Triple-Negative Breast Cancer Delineated by Single-Cell Sequencing. Cell 173, 879-893.e13 (2018).

7. Turajlic, S. et al. Tracking Cancer Evolution Reveals Constrained Routes to Metastases: TRACERx Renal. Cell 173, 581-594.e12 (2018).

8. Sottoriva, A. et al. A Big Bang model of human colorectal tumor growth. Nat. Genet. 47, 209-216 (2015).

9. Aparicio, S. & Caldas, C. The implications of clonal genome evolution for cancer medicine. N. Engl. J. Med. 368, 842-851 (2013).

10. Forsberg, L. A., Gisselsson, D. & Dumanski, J. P. Mosaicism in health and disease - clones picking up speed. Nat. Rev. Genet. 18, 128-142 (2017).

11. Stratton, M. R. Exploring the genomes of cancer cells: progress and promise. Science 331, 1553-1558 (2011).

12. Korbel, J. O. et al. Paired-end mapping reveals extensive structural variation in the human genome. Science 318, 420-426 (2007).

13. Layer, R. M., Chiang, C., Quinlan, A. R. & Hall, I. M. LUMPY: a probabilistic framework for structural variant discovery. Genome Biol. 15, R84 (2014).

14. Leibowitz, M. L., Zhang, C.-Z. & Pellman, D. Chromothripsis: A New Mechanism for Rapid Karyotype Evolution. Annu. Rev. Genet. 49, 183-211 (2015).

15. Navin, N. E. Cancer genomics: one cell at a time. Genome Biol. 15, 452 (2014).

16. Zahn, H. et al. Scalable whole-genome single-cell library preparation without preamplification. Nat. Methods 14, 167-173 (2017).

17. Gawad, C., Koh, W. & Quake, S. R. Single-cell genome sequencing: current state of the science. Nat. Rev. Genet. 17, 175-188 (2016).

18. Bakker, B. et al. Single-cell sequencing reveals karyotype heterogeneity in murine and human malignancies. Genome Biol. 17, 115 (2016).

19. Voet, T. et al. Single-cell paired-end genome sequencing reveals structural variation per cell cycle. Nucleic Acids Res. 41, 6119-6138 (2013).

20. Zhang, C. Z. et al. Chromothripsis from DNA damage in micronuclei. Nature 522, 179-184 (2015).

21. Falconer, E. et al. DNA template strand sequencing of single-cells maps genomic rearrangements at high resolution. Nat. Methods 9, 1107-1112 (2012).

22. Porubsky, D. et al. Dense and accurate whole-chromosome haplotyping of individual genomes. Nat. Commun. 8, 1293 (2017).

23. Sanders, A. D. et al. Characterizing polymorphic inversions in human genomes by single-cell sequencing. Genome Res. 26, 1575-1587 (2016).

24. van Wietmarschen, N. & Lansdorp, P. M. Bromodeoxyuridine does not contribute to sister chromatid exchange events in normal or Bloom syndrome cells. Nucleic Acids Res. 44, 6787-6793 (2016).

25. Yang, L. et al. Diverse mechanisms of somatic structural variations in human cancer genomes. Cell 153, 919-929 (2013).

26. Li, Y. et al. Patterns of structural variation in human cancer, bioRxiv. bioRxiv 181339 (2017). doi:10.1101/181339

27. Janssen, A., van der Burg, M., Szuhai, K., Kops, G. J. & Medema, R. H. Chromosome segregation errors as a cause of DNA damage and structural chromosome aberrations. Science 333, 1895-1898 (2011).

28. Mardin, B. R. et al. A cell-based model system links chromothripsis with hyperploidy. Mol. Syst. Biol. 11, 828 (2015).

29. Maciejowski, J., Li, Y., Bosco, N., Campbell, P. J. & de Lange, T. Chromothripsis and Kataegis Induced by Telomere Crisis. Cell 163, 1641-1654 (2015).

30. Riches, A. et al. Neoplastic transformation and cytogenetic changes after Gamma irradiation of human epithelial cells expressing telomerase. Radiat. Res. 155, 222-229 (2001).

31. Rausch, T. et al. DELLY: structural variant discovery by integrated paired-end and split-read analysis. Bioinformatics 28, i333-i339 (2012).

32. Amatu, A., Sartore-Bianchi, A. & Siena, S. NTRK gene fusions as novel targets of cancer therapy across multiple tumour types. ESMO Open 1, e000023 (2016).

33. Zhang, C.-Z., Leibowitz, M. L. & Pellman, D. Chromothripsis and beyond: rapid genome evolution from complex chromosomal rearrangements. Genes Dev. 27, 2513-2530 (2013).

34. Campbell, P. J. et al. The patterns and dynamics of genomic instability in metastatic pancreatic cancer. Nature 467, 1109-1113 (2010).

35. Rode, A., Maass, K. K., Willmund, K. V., Lichter, P. & Ernst, A. Chromothripsis in cancer cells: An update. Int. J. Cancer 138, 2322-2333 (2016).

36. Selvarajah, S. et al. The breakage-fusion-bridge (BFB) cycle as a mechanism for generating genetic heterogeneity in osteosarcoma. Chromosoma 115, 459-467 (2006).

37. Li, Y. et al. Constitutional and somatic rearrangement of chromosome 21 in acute lymphoblastic leukaemia. Nature 508, 98-102 (2014).

38. McClintock, B. The Stability of Broken Ends of Chromosomes in Zea Mays. Genetics 26, 234-282 (1941).

39. Gisselsson, D. et al. Chromosomal breakage-fusion-bridge events cause genetic intratumor heterogeneity. Proc. Natl. Acad. Sci. U. S. A. 97, 5357-5362 (2000).

40. Thompson, S. L., Bakhoum, S. F. & Compton, D. A. Mechanisms of chromosomal instability. Curr. Biol. 20, R285-95 (2010).

41. Stephens, P. J. et al. Massive genomic rearrangement acquired in a single catastrophic event during cancer development. Cell 144, 27-40 (2011).

42. Korbel, J. O. & Campbell, P. J. Criteria for inference of chromothripsis in cancer genomes. Cell 152, 1226-1236 (2013).

43. Girardi, T., Vicente, C., Cools, J. & De Keersmaecker, K. The genetics and molecular biology of T-ALL. Blood 129, 1113-1123 (2017).

44. Richter Pechańska, P. et al. PDX models recapitulate the genetic and epigenetic landscape of pediatric T cell leukemia. EMBO Mol. Med. e9443 (2018).

45. Liu, Y. et al. The genomic landscape of pediatric and young adult T-lineage acute lymphoblastic leukemia. Nat. Genet. 49, 1211-1218 (2017).

46. Wang, Q. et al. Mutations of PHF6 are associated with mutations of NOTCH1, JAK1 and rearrangement of SET-NUP214 in T-cell acute lymphoblastic leukemia. Haematologica 96, 1808-1814 (2011).

47. Rao, S. et al. Inactivation of ribosomal protein L22 promotes transformation by induction of the stemness factor, Lin28B. Blood 120, 3764-3773 (2012).

48. Nagel, S. et al. Activation of TLX3 and NKX2-5 in t(5;14)(q35;q32) T-cell acute lymphoblastic leukemia by remote 3'-BCL11B enhancers and coregulation by PU.1 and HMGA1. Cancer Res. 67, 1461-1471 (2007).

49. Bernard, O. A. et al. A new recurrent and specific cryptic translocation, t(5;14)(q35;q32), is associated with expression of the Hox11L2 gene in T acute lymphoblastic leukemia. Leukemia 15, 1495-1504 (2001).

50. Kunz, J. B. et al. Pediatric T-cell lymphoblastic leukemia evolves into relapse by clonal selection, acquisition of mutations and promoter hypomethylation. Haematologica 100, 1442-1450 (2015).

51. Li, L. et al. A far downstream enhancer for murine Bcl11b controls its T-cell specific expression. Blood 122, 902-911 (2013).

52. Sugimoto, K.-J. et al. T-cell lymphoblastic leukemia/lymphoma with t(7;14)(p15;q32) [TCRγ-TCL1A translocation]: a case report and a review of the literature. Int. J. Clin. Exp. Pathol. 7, 2615-2623 (2014).

53. Virgilio, L. et al. Deregulated expression of TCLi causes T cell leukemia in mice. Proc. Natl. Acad. Sci. U. S. A. 95, 3885-3889 (1998).

54. Love, M. I., Huber, W. & Anders, S. Moderated estimation of fold change and dispersion for RNA-seq data with DESeq2. Genome Biol. 15, 550 (2014).

55. Alkan, C., Coe, B. P. & Eichler, E. E. Genome structural variation discovery and genotyping. Nat. Rev. Genet. 12, 363-376 (2011).

56. Campbell, I. M., Shaw, C. A., Stankiewicz, P. & Lupski, J. R. Somatic mosaicism: implications for disease and transmission genetics. Trends Genet. 31, 382-392 (2015).

57. Dou, Y., Gold, H. D., Luquette, L. J. & Park, P. J. Detecting Somatic Mutations in Normal Cells. Trends Genet. 34, 545-557 (2018).

58. Voet, T. et al. Breakage-fusion-bridge cycles leading to inv dup del occur in human cleavage stage embryos. Hum. Mutat. 32, 783-793 (2011).

59. Bakhoum, S. F. et al. The mitotic origin of chromosomal instability. Curr. Biol. 24, R148-9 (2014).

60. Wang, Y. K. et al. Genomic consequences of aberrant DNA repair mechanisms stratify ovarian cancer histotypes. Nat. Genet. 49, 856-865 (2017).

61. Rücker, F. G. et al. Chromothripsis is linked to TP53 alteration, cell cycle impairment, and dismal outcome in acute myeloid leukemia with complex karyotype. Haematologica 103, e17-e20 (2018).

62. Navin, N. E. & Hicks, J. Tracing the tumor lineage. Mol. Oncol. 4, 267-283 (2010).

63. Lee, H. & Kim, J.-S. Unexpected CRISPR on-target effects. Nat. Biotechnol. 36, 703-704 (2018).

64. Yoshihara, M., Hayashizaki, Y. & Murakawa, Y. Genomic Instability of iPSCs: Challenges Towards Their Clinical Applications. Stem Cell Rev. 13, 7-16 (2017).

65. Mooijman, D., Dey, S. S., Boisset, J. C., Crosetto, N. & van Oudenaarden, A. Single-cell 5hmC sequencing reveals chromosome-wide cell-to-cell variability and enables lineage reconstruction. Nat. Biotechnol. 34, 852-856 (2016).

66. Frismantas, V. et al. Ex vivo drug response profiling detects recurrent sensitivity patterns in drug-resistant acute lymphoblastic leukemia. Blood 129, e26-e37 (2017).

67. Sanders, A. D., Falconer, E., Hills, M., Spierings, D. C. J. & Lansdorp, P. M. Single-cell template strand sequencing by Strand-seq enables the characterization of individual homologs. Nat. Protoc. 12, 1151-1176 (2017).

68. 1000-Genomes-Project-Consortium et al. A global reference for human genetic variation. Nature 526, 68-74 (2015).

69. Garrison, E. & Marth, G. Haplotype-based variant detection from short-read sequencing. arXiv [q-bio.GN] (2012).

70. Huber, W., Toedling, J. & Steinmetz, L. M. Transcript mapping with high-density oligonucleotide tiling arrays. Bioinformatics 22,1963-1970 (2006).

71. Claussin, C. et al. Genome-wide mapping of sister chromatid exchange events in single yeast cells using Strand-seq. Elife 6, (2017).

72. Porubsky, D. et al. Direct chromosome-length haplotyping by single-cell sequencing. Genome Res. 26, 1565-1574 (2016).

73. Benjamini, Y. & Hochberg, Y. Controlling the False Discovery Rate: A Practical and Powerful Approach to Multiple Testing. J. R. Stat. Soc. Series B Stat. Methodol. 57, 289-300 (1995).

74. Klambauer, G. et al. cn.MOPS: mixture of Poissons for discovering copy number variations in next-generation sequencing data with a low false discovery rate. Nucleic Acids Res. 40, e69 (2012).

75. Rausch, T. et al. Genome sequencing of pediatric medulloblastoma links catastrophic DNA rearrangements with TP53 mutations. Cell 148, 59-71 (2012).

76. Dobin, A. et al. STAR: ultrafast universal RNA-seq aligner. Bioinformatics 29, 15-21 (2013).

77. Fan, J. et al. Linking transcriptional and genetic tumor heterogeneity through allele analysis of single-cell RNA-seq data. Genome Res. 28, 1217-1227 (2018).

78. Lapunzina, P. & Monk, D. The consequences of uniparental disomy and copy number neutral loss-of-heterozygosity during human development and cancer. Biol. Cell 103, 303-317 (2011).

79. Vogelstein, B. et al. Cancer genome landscapes. Science 339,1546-1558 (2013).

**Claims**

1. **A method for analyzing sequencing data of at least one target chromosomal region by single cell tri-channel processing (scTRIP),** comprising providing strand specific sequence data of at least one target chromosomal region of at least one single cell, wherein the strand-specific sequencing data comprise a multitude of strand specific sequence reads obtained by sequencing of the target chromosomal region of at least one single cell, aligning the sequence reads, or if the sequence reads are equally fragmented, each fragmented portion of such sequence read, to a reference assembly, and then assign in any given selected window the at least two of three channels of sequence information: (i) number of total sequence reads, or portions thereof; (ii) number of forward (or Watson) sequence reads, or portions thereof, and number of reverse (or Crick) sequence reads, or portions thereof; (iii) numbers of sequence reads, or portion thereof, assigned with a specific haplotype identity (such as H1 and/or H2).

2. The method according to claim 1, wherein all three channels of sequence information (i) to (iii) are assigned.

3. The method according to claim 1 or 2, comprising a step of segmenting the at least one target chromosomal region, wherein the segmenting is performed on basis of the channels of sequence information (i) to (iii), each individually, in any combination, or together.

4. The method according to any one of claims **1** to 3, wherein the strand-specific sequence data comprises sequence reads mapping to at least two separate strands of the at least one target chromosomal region, for example wherein one strand is from the paternal and the other strand is from the maternal chromosome.

5. The method according to any one of claims 1 to 4, wherein the sequencing data comprises a multitude of non-overlapping and/or overlapping sequence reads.

6. The method according to any one of claims 1 to 5, further comprising the step of: Identifying a structural variation (SV) by assigning said sequence information for a multiplicity (at least two) of windows within the sequence data, and identifying within the multiplicity of windows a sub-region comprising one or more windows having an unusual/altered/changed distribution of the information of any one, or all of, or any combination of, channels (i) to (iii), compared to a reference state.

7. The method according to claim 6, wherein said reference state of said chromosomal region is a state of the information of the channels which is expected for a non-aberrant distribution and/or predetermined state of the information of said chromosomal region.

8. The method according to any one of claims 1 to 7, wherein a haplotype identity (H1/H2) is assigned along the at least one target chromosomal region, preferably while retaining strand orientation information (i.e. in a strand aware manner), and preferably such haplotype is assigned by assigning Single Nucleotide Polymorphisms (SNP) to the sequence reads, or portions thereof, preferably wherein such SNP does not have a disease association, and wherein the haplotype identity is assigned to a sequence read, or a portion thereof, comprising a SNP, and identifying the allele of the SNP by comparison to a SNP database, or alternatively by comparing the allele to a multiplicity of further sequenced single cells of the same origin (for example using StrandPhaseR - Porubsky et al. 2017); and, optionally, wherein haplotype identity is assigned to a sequence read, or a portion thereof, not comprising a SNP, by inferring said haplotype identity in by strand identity and comparison to other sequence reads, or portions thereof, having the same strand identity and which comprise such SNP.

9. The method according to any one of claims 1 to 8, wherein the target chromosomal region is one or more chromosomes, preferably one or more chromosomes of a diploid organism.

10. The method according to any one of claims 1 to 9, wherein the strand-specific sequence data of the at least one target chromosomal region of at least one single cell is obtained from a cellular sample of a patient, and wherein said single cell is either a cell associated with a disease, or is a healthy cell of said patient, preferably wherein the method is performed for a multiplicity of single cells associated with the disease and/or healthy cells.

11. The method according to any one of claims 1 to 10, wherein the method comprises a further step of diagnosing a disease or condition based on the identity of, location of, or number of detected SV within the target chromosomal region.

12. **A method of detecting a structural variation (SV) in a target chromosomal region**, the method comprising, preforming a method according to claim 6, and claims 7 to 11 when referring to claim 6.

13. **A method of karyotyping a single cell**, or a population of multiple single cells, the method comprising,

(a) Providing strand specific sequence data of the at least one target chromosomal region, preferably the complete genome, of at least one single cell, or each of the population of single cells,
(b) Performing a method of claims 1 to 11,
(c) Detecting SV within the target chromosomal region of said single cell, or the population of single cells, and
(d) Obtaining an in-silico karyotype based on all detected SVs.

14. **A method of diagnosing a disease or condition in a subject**, the method comprising, providing strand specific sequence data of one or more cells of the subject, performing a method according to claim 11, detecting within the one or more cells any SV, and comparing the detected SV with a reference state, wherein an altered number, type or location of one or SV in the sample of the subject indicated the presence of a condition, such as a disease, for example cancer.

15. **A method for assessing the chromosomal instability (CIN)** of a single cell, or within a population of single cells, the method comprising performing a method according to any one of claims 1 to 13, and wherein an increased total number, or increased number, of any one type or multiple types, of SV in the said single cell or population of single cells, indicates CIN.

16. The method according to claim 15, for use in quality control of a cell or population of cells, wherein an increased instability indicates a loss of quality, preferably wherein the method is performed subsequent to an (genetic) alteration of said cell or population of cells, such as wherein the single cell or population of single cells is genetically engineered, preferably such as by reprogramming, gene editing or viral integration.

17. The method according to claim 15 or 16, wherein the single cell or population of single cells, are for use in a cellular therapy of a patient, such as autologous immune cell therapy.

18. **A computer readable medium** comprising computer readable instructions stored thereon that when run on a computer perform a method according to any one of claims 1 to 17.

## FIGURE 1A

## FIGURE 1B

## FIGURE 1C

Reference state

Depth   Strand   Phase

FIGURE 1D

Deletion

Duplication

FIGURE 1E

Balanced inversion

Inverted duplication

FIGURE 1F

## Diagnostic footprint of inter-chromosomal SV classes

### Balanced translocation

chr9

chr13

IGURE 1G

FIGURE 1H

FIGURE 2A

FIGURE 2B

FIGURE 2C

Monosomic chr13 (C7)  Cell population-level segregation pattern of monosomic Chr13

FIGURE 2D

Trisomic chr10q region (RPE-1)  Cell population-level segregation pattern of trisomic 10q region

## FIGURE 3A

FIGURE 3B

FIGURE 3C

Four derivative chromosomes characterized in BM510

FIGURE 3D

FIGURE 3E

FIGURE 4A

FIGURE 4B and 4D

FIGURE 4C

FIGURE 4E

Model of Breakage-fusion-bridge (BFB) cycle in C7

FIGURE 4F

FIGURE 4G

EP 3 723 096 A1

## FIGURE 5A

FIGURE 5B

## Clustered single cell results for P33

FIGURE 5C

## Clustered single cell results for P1

FIGURE 6A

FIGURE 6B

FIGURE 6C

FIGURE 6D

FIGURE 6E

FIGURE 6F and 6G

Europäisches
Patentamt

European
Patent Office

Office européen
des brevets

# EUROPEAN SEARCH REPORT

**Application Number**

EP 19 16 9090

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ASHLEY D SANDERS ET AL: "Single-cell template strand sequencing by Strand-seq enables the characterization of individual homologs", NATURE PROTOCOLS, vol. 12, no. 6, 11 May 2017 (2017-05-11), pages 1151-1176, XP055631163, GB ISSN: 1754-2189, DOI: 10.1038/nprot.2017.029 * abstract * * page 1151, column 1, paragraph 1 - page 1152, column 1, paragraph 1; figure 1 * * page 1153, column 1, paragraph 2 - page 1154, column 2, paragraph 2 * * page 1155, column 1, paragraph 1 - paragraph 2 * * page 1175, paragraph 3 * * page 1158, column 1, paragraph 1 * | 1-18 | INV. G16B20/20 ADD. G16B30/10 G16B50/10 |
| A | TAMARA BORGONOVO ET AL: "Genetic evaluation of mesenchymal stem cells by G-banded karyotyping in a Cell Technology Center", REVISTA BRASILEIRA DE HEMATOLOGIA E HEMOTERAPIA, vol. 36, no. 3, 3 April 2014 (2014-04-03), pages 202-207, XP055631504, ISSN: 1516-8484, DOI: 10.1016/j.bjhh.2014.03.006 * abstract * * page 202, column 1 - column 2, paragraph 1 * | 15-17 | TECHNICAL FIELDS SEARCHED (IPC) G16B |

The present search report has been drawn up for all claims

| Place of search | Date of completion of the search | Examiner |
|---|---|---|
| Munich | 16 October 2019 | Schmitt, Constanze |

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **FALCONER et al.** *Nature Methods.,* 2012, vol. 9 (11), 1107-1112
- *Genome Biol,* 2009, vol. 10 (3), R25
- *Bioinformatics,* 15 July 2009, vol. 25 (14), 1754-60
- **SAMBROOK ; RUSSELL.** Molecular Cloning: A Laboratory Manual. Cold Spring Harbor Laboratory Press, 2001
- **SAMBROOK ; FRITSCH.** Maniatis. Molecular Cloning: A Laboratory Manual
- **PORUBSKY, D. et al.** Dense and accurate whole-chromosome haplotyping of individual genomes. *Nat. Commun.,* 2017, vol. 8, 1293
- **FALCONER et al.** *Nature Methods,* 2012, vol. 9 (11), 1107-1112
- **CIRIELLO, G. et al.** Emerging landscape of oncogenic signatures across human cancers. *Nat. Genet.,* 2013, vol. 45, 1127-1133
- **MERTENS, F. ; JOHANSSON, B. ; FIORETOS, T. ; MITELMAN, F.** The emerging complexity of gene fusions in cancer. *Nat. Rev. Cancer,* 2015, vol. 15, 371-381
- **NORTHCOTT, P. A. et al.** The whole-genome landscape of medulloblastoma subtypes. *Nature,* 2017, vol. 547, 311-317
- **BEROUKHIM, R. ; ZHANG, X. ; MEYERSON, M.** Copy number alterations unmasked as enhancer hijackers. *Nat. Genet.,* 2016, vol. 49, 5-6
- **NORTHCOTT, P. A. et al.** Enhancer hijacking activates GFI1 family oncogenes in medulloblastoma. *Nature,* 2014, vol. 511, 428-434
- **KIM, C. et al.** Chemoresistance Evolution in Triple-Negative Breast Cancer Delineated by Single-Cell Sequencing. *Cell,* 2018, vol. 173, 879-893
- **TURAJLIC, S. et al.** Tracking Cancer Evolution Reveals Constrained Routes to Metastases: TRACERx Renal. *Cell,* 2018, vol. 173, 581-594
- **SOTTORIVA, A. et al.** A Big Bang model of human colorectal tumor growth. *Nat. Genet.,* 2015, vol. 47, 209-216
- **APARICIO, S. ; CALDAS, C.** The implications of clonal genome evolution for cancer medicine. *N. Engl. J. Med.,* 2013, vol. 368, 842-851
- **FORSBERG, L. A. ; GISSELSSON, D. ; DUMANSKI, J. P.** Mosaicism in health and disease - clones picking up speed. *Nat. Rev. Genet.,* 2017, vol. 18, 128-142

- **STRATTON, M. R.** Exploring the genomes of cancer cells: progress and promise. *Science,* 2011, vol. 331, 1553-1558
- **KORBEL, J. O. et al.** Paired-end mapping reveals extensive structural variation in the human genome. *Science,* 2007, vol. 318, 420-426
- **LAYER, R. M. ; CHIANG, C. ; QUINLAN, A. R. ; HALL, I. M. LUMPY.** a probabilistic framework for structural variant discovery. *Genome Biol.,* 2014, vol. 15, R84
- **LEIBOWITZ, M. L. ; ZHANG, C.-Z. ; PELLMAN, D.** Chromothripsis: A New Mechanism for Rapid Karyotype Evolution. *Annu. Rev. Genet.,* 2015, vol. 49, 183-211
- **NAVIN, N. E.** Cancer genomics: one cell at a time. *Genome Biol.,* 2014, vol. 15, 452
- **ZAHN, H. et al.** Scalable whole-genome single-cell library preparation without preamplification. *Nat. Methods,* 2017, vol. 14, 167-173
- **GAWAD, C. ; KOH, W. ; QUAKE, S. R.** Single-cell genome sequencing: current state of the science. *Nat. Rev. Genet.,* 2016, vol. 17, 175-188
- **BAKKER, B. et al.** Single-cell sequencing reveals karyotype heterogeneity in murine and human malignancies. *Genome Biol.,* 2016, vol. 17, 115
- **VOET, T. et al.** Single-cell paired-end genome sequencing reveals structural variation per cell cycle. *Nucleic Acids Res.,* 2013, vol. 41, 6119-6138
- **ZHANG, C. Z. et al.** Chromothripsis from DNA damage in micronuclei. *Nature,* 2015, vol. 522, 179-184
- **FALCONER, E. et al.** DNA template strand sequencing of single-cells maps genomic rearrangements at high resolution. *Nat. Methods,* 2012, vol. 9, 1107-1112
- **SANDERS, A. D. et al.** Characterizing polymorphic inversions in human genomes by single-cell sequencing. *Genome Res.,* 2016, vol. 26, 1575-1587
- **VAN WIETMARSCHEN, N. ; LANSDORP, P. M.** Bromodeoxyuridine does not contribute to sister chromatid exchange events in normal or Bloom syndrome cells. *Nucleic Acids Res.,* 2016, vol. 44, 6787-6793
- **YANG, L. et al.** Diverse mechanisms of somatic structural variations in human cancer genomes. *Cell,* 2013, vol. 153, 919-929
- **LI, Y. et al.** *Patterns of structural variation in human cancer, bioRxiv. bioRxiv 181339,* 2017

- **JANSSEN, A. ; VAN DER BURG, M. ; SZUHAI, K. ; KOPS, G. J. ; MEDEMA, R. H.** Chromosome segregation errors as a cause of DNA damage and structural chromosome aberrations. *Science,* 2011, vol. 333, 1895-1898
- **MARDIN, B. R. et al.** A cell-based model system links chromothripsis with hyperploidy. *Mol. Syst. Biol.,* 2015, vol. 11, 828
- **MACIEJOWSKI, J. ; LI, Y. ; BOSCO, N. ; CAMPBELL, P. J. ; DE LANGE, T.** Chromothripsis and Kataegis Induced by Telomere Crisis. *Cell,* 2015, vol. 163, 1641-1654
- **RICHES, A. et al.** Neoplastic transformation and cytogenetic changes after Gamma irradiation of human epithelial cells expressing telomerase. *Radiat. Res.,* 2001, vol. 155, 222-229
- **RAUSCH, T. et al.** DELLY: structural variant discovery by integrated paired-end and split-read analysis. *Bioinformatics,* 2012, vol. 28, i333-i339
- **AMATU, A. ; SARTORE-BIANCHI, A. ; SIENA, S.** NTRK gene fusions as novel targets of cancer therapy across multiple tumour types. *ESMO Open,* 2016, vol. 1, e000023
- **ZHANG, C.-Z. ; LEIBOWITZ, M. L. ; PELLMAN, D.** Chromothripsis and beyond: rapid genome evolution from complex chromosomal rearrangements. *Genes Dev.,* 2013, vol. 27, 2513-2530
- **CAMPBELL, P. J. et al.** The patterns and dynamics of genomic instability in metastatic pancreatic cancer. *Nature,* 2010, vol. 467, 1109-1113
- **RODE, A. ; MAASS, K. K. ; WILLMUND, K. V. ; LICHTER, P. ; ERNST, A.** Chromothripsis in cancer cells: An update. *Int. J. Cancer,* 2016, vol. 138, 2322-2333
- **SELVARAJAH, S. et al.** The breakage-fusion-bridge (BFB) cycle as a mechanism for generating genetic heterogeneity in osteosarcoma. *Chromosoma,* 2006, vol. 115, 459-467
- **LI, Y. et al.** Constitutional and somatic rearrangement of chromosome 21 in acute lymphoblastic leukaemia. *Nature,* 2014, vol. 508, 98-102
- **MCCLINTOCK, B.** The Stability of Broken Ends of Chromosomes in Zea Mays. *Genetics,* 1941, vol. 26, 234-282
- **GISSELSSON, D. et al.** Chromosomal breakage-fusion-bridge events cause genetic intratumor heterogeneity. *Proc. Natl. Acad. Sci. U. S. A.,* 2000, vol. 97, 5357-5362
- **THOMPSON, S. L. ; BAKHOUM, S. F. ; COMPTON, D. A.** Mechanisms of chromosomal instability. *Curr. Biol.,* 2010, vol. 20, R285-95
- **STEPHENS, P. J. et al.** Massive genomic rearrangement acquired in a single catastrophic event during cancer development. *Cell,* 2011, vol. 144, 27-40
- **KORBEL, J. O. ; CAMPBELL, P. J.** Criteria for inference of chromothripsis in cancer genomes. *Cell,* 2013, vol. 152, 1226-1236
- **GIRARDI, T. ; VICENTE, C. ; COOLS, J. ; DE KEERSMAECKER, K.** The genetics and molecular biology of T-ALL. *Blood,* 2017, vol. 129, 1113-1123
- **RICHTER□PECHAŃSKA, P. et al.** PDX models recapitulate the genetic and epigenetic landscape of pediatric T□cell leukemia. *EMBO Mol. Med.,* 2018, e9443
- **LIU, Y. et al.** The genomic landscape of pediatric and young adult T-lineage acute lymphoblastic leukemia. *Nat. Genet.,* 2017, vol. 49, 1211-1218
- **WANG, Q. et al.** Mutations of PHF6 are associated with mutations of NOTCH1, JAK1 and rearrangement of SET-NUP214 in T-cell acute lymphoblastic leukemia. *Haematologica,* 2011, vol. 96, 1808-1814
- **RAO, S. et al.** Inactivation of ribosomal protein L22 promotes transformation by induction of the stemness factor, Lin28B. *Blood,* 2012, vol. 120, 3764-3773
- **NAGEL, S. et al.** Activation of TLX3 and NKX2-5 in t(5;14)(q35;q32) T-cell acute lymphoblastic leukemia by remote 3'-BCL11B enhancers and coregulation by PU.1 and HMGA1. *Cancer Res.,* 2007, vol. 67, 1461-1471
- **BERNARD, O. A. et al.** A new recurrent and specific cryptic translocation, t(5;14)(q35;q32), is associated with expression of the Hox11L2 gene in T acute lymphoblastic leukemia. *Leukemia,* 2001, vol. 15, 1495-1504
- **KUNZ, J. B. et al.** Pediatric T-cell lymphoblastic leukemia evolves into relapse by clonal selection, acquisition of mutations and promoter hypomethylation. *Haematologica,* 2015, vol. 100, 1442-1450
- **LI, L. et al.** A far downstream enhancer for murine Bcl11b controls its T-cell specific expression. *Blood,* 2013, vol. 122, 902-911
- **SUGIMOTO, K.-J. et al.** T-cell lymphoblastic leukemia/lymphoma with t(7;14)(p15;q32) [TCRγ-TCL1A translocation]: a case report and a review of the literature. *Int. J. Clin. Exp. Pathol.,* 2014, vol. 7, 2615-2623
- **VIRGILIO, L. et al.** Deregulated expression of TCLi causes T cell leukemia in mice. *Proc. Natl. Acad. Sci. U. S. A.,* 1998, vol. 95, 3885-3889
- **LOVE, M. I. ; HUBER, W. ; ANDERS, S.** Moderated estimation of fold change and dispersion for RNA-seq data with DESeq2. *Genome Biol.,* 2014, vol. 15, 550
- **ALKAN, C. ; COE, B. P. ; EICHLER, E. E.** Genome structural variation discovery and genotyping. *Nat. Rev. Genet.,* 2011, vol. 12, 363-376
- **CAMPBELL, I. M. ; SHAW, C. A. ; STANKIEWICZ, P. ; LUPSKI, J. R.** Somatic mosaicism: implications for disease and transmission genetics. *Trends Genet.,* 2015, vol. 31, 382-392
- **DOU, Y. ; GOLD, H. D. ; LUQUETTE, L. J. ; PARK, P. J.** Detecting Somatic Mutations in Normal Cells. *Trends Genet.,* 2018, vol. 34, 545-557

- **VOET, T. et al.** Breakage-fusion-bridge cycles leading to inv dup del occur in human cleavage stage embryos. *Hum. Mutat.,* 2011, vol. 32, 783-793
- **BAKHOUM, S. F. et al.** The mitotic origin of chromosomal instability. *Curr. Biol.,* 2014, vol. 24, R148-9
- **WANG, Y. K. et al.** Genomic consequences of aberrant DNA repair mechanisms stratify ovarian cancer histotypes. *Nat. Genet.,* 2017, vol. 49, 856-865
- **RÜCKER, F. G. et al.** Chromothripsis is linked to TP53 alteration, cell cycle impairment, and dismal outcome in acute myeloid leukemia with complex karyotype. *Haematologica,* 2018, vol. 103, e17-e20
- **NAVIN, N. E. ; HICKS, J.** Tracing the tumor lineage. *Mol. Oncol.,* 2010, vol. 4, 267-283
- **LEE, H. ; KIM, J.-S.** Unexpected CRISPR on-target effects. *Nat. Biotechnol.,* 2018, vol. 36, 703-704
- **YOSHIHARA, M. ; HAYASHIZAKI, Y. ; MURAKAWA, Y.** Genomic Instability of iPSCs: Challenges Towards Their Clinical Applications. *Stem Cell Rev.,* 2017, vol. 13, 7-16
- **MOOIJMAN, D. ; DEY, S. S. ; BOISSET, J. C. ; CROSETTO, N. ; VAN OUDENAARDEN, A.** Single-cell 5hmC sequencing reveals chromosome-wide cell-to-cell variability and enables lineage reconstruction. *Nat. Biotechnol.,* 2016, vol. 34, 852-856
- **FRISMANTAS, V. et al.** Ex vivo drug response profiling detects recurrent sensitivity patterns in drug-resistant acute lymphoblastic leukemia. *Blood,* 2017, vol. 129, e26-e37
- **SANDERS, A. D. ; FALCONER, E. ; HILLS, M. ; SPIERINGS, D. C. J. ; LANSDORP, P. M.** Single-cell template strand sequencing by Strand-seq enables the characterization of individual homologs. *Nat. Protoc.,* 2017, vol. 12, 1151-1176
- **1000-GENOMES-PROJECT-CONSORTIUM et al.** A global reference for human genetic variation. *Nature,* 2015, vol. 526, 68-74
- **GARRISON, E. ; MARTH, G.** Haplotype-based variant detection from short-read sequencing. *arXiv [q-bio.GN],* 2012
- **HUBER, W. ; TOEDLING, J. ; STEINMETZ, L. M.** Transcript mapping with high-density oligonucleotide tiling arrays. *Bioinformatics,* 2006, vol. 22, 1963-1970
- **CLAUSSIN, C. et al.** Genome-wide mapping of sister chromatid exchange events in single yeast cells using Strand-seq. *Elife,* 2017, vol. 6
- **PORUBSKY, D. et al.** Direct chromosome-length haplotyping by single-cell sequencing. *Genome Res.,* 2016, vol. 26, 1565-1574
- **BENJAMINI, Y. ; HOCHBERG, Y.** Controlling the False Discovery Rate: A Practical and Powerful Approach to Multiple Testing. *J. R. Stat. Soc. Series B Stat. Methodol.,* 1995, vol. 57, 289-300
- **KLAMBAUER, G. et al.** cn.MOPS: mixture of Poissons for discovering copy number variations in next-generation sequencing data with a low false discovery rate. *Nucleic Acids Res.,* 2012, vol. 40, e69
- **RAUSCH, T. et al.** Genome sequencing of pediatric medulloblastoma links catastrophic DNA rearrangements with TP53 mutations. *Cell,* 2012, vol. 148, 59-71
- **DOBIN, A. et al.** STAR: ultrafast universal RNA-seq aligner. *Bioinformatics,* 2013, vol. 29, 15-21
- **FAN, J. et al.** Linking transcriptional and genetic tumor heterogeneity through allele analysis of single-cell RNA-seq data. *Genome Res.,* 2018, vol. 28, 1217-1227
- **LAPUNZINA, P. ; MONK, D.** The consequences of uniparental disomy and copy number neutral loss-of-heterozygosity during human development and cancer. *Biol. Cell,* 2011, vol. 103, 303-317
- **VOGELSTEIN, B. et al.** Cancer genome landscapes. *Science,* 2013, vol. 339, 1546-1558